# EUROPEAN PATENT APPLICATION

(11) **EP 4 063 372 A1**
(43) Date of publication of application: **28.09.2022**
(21) Application number: 20889059.0
(22) Date of filing: 19.11.2020
(51) Int. Cl.: C07F 5/02, C09K 11/06, C07K 1/13, A61K 47/54, A61K 47/59, A61K 47/61, A61K 47/62, A61K 47/68, A61K 49/00, C12N 15/10, C09B 23/04, C09B 23/14, G01N 33/53, G01N 21/64

(54) **COMPOUND AND FLUORESCENTLY LABELED BIOMATERIAL USING SAME**

(30) Priority: 21.11.2019 JP 2019210701; 26.06.2020 JP 2020110909
(71) Applicant: FUJIFILM Corporation, Tokyo 106-8620 (JP)
(72) Inventor: NAKATA, Hiyoku, Ashigarakami-gun, Kanagawa 258-8577 (JP); SAKAKIBARA, Masato, Ashigarakami-gun, Kanagawa 258-8577 (JP); ASAKURA, Akihiro, Ashigarakami-gun, Kanagawa 258-8577 (JP); MAKITA, Keiko, Ashigarakami-gun, Kanagawa 258-8577 (JP)
(74) Representative: HGF
(86) International application number: PCT/JP2020/043224
(87) International publication number: WO 2021/100814

(57) **Abstract**

Provided are the following compound and a fluorescently labeled biological substance having this fluorescent compound. 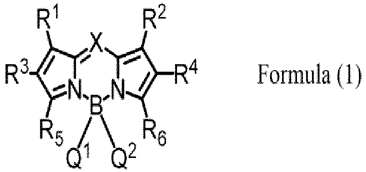 X represents CR⁷ or N.
R¹ to R⁷, Q¹, and Q² represent a hydrogen atom, a halogen atom, a cyano group, or a group represented by Formula (A), and adjacent substituents may form a ring structure.

However, at least one of R³, R⁴, R⁷, Q¹, or Q² contains, as a hydrophilic group, at least one of a carboxy group or a salt thereof, a sulfo group or a salt thereof, a phosphono group or a salt thereof, an onio group, or a polyamino acid residue, where at least one of Q¹ or Q² represents an alkyl group.

## Description

### BACKGROUND OF THE INVENTION

### 1. Field of the Invention

The present invention relates to a compound and a fluorescently labeled biological substance using the compound.

### 2. Description of the Related Art

In pathological examinations, identification of infectious diseases, and the like, fluorescently labeled biological substances obtained by labeling biological substances such as an antibody that binds to a target substance to be detected, with a compound (a dye) are used.

Among them, bioimaging technology for analyzing the dynamics and functions of living body molecules, cells, tissues, and the like in the living body has been utilized for the diagnosis of various diseases. In recent years, in vivo fluorescence imaging, which visualizes and observes a specific portion of a living body with a fluorescent dye, is expected as a new technique for living body observation.

In this in vivo fluorescence imaging, an organic fluorescent dye is generally used. However, the organic fluorescent dye has low light resistance and deteriorates by irradiation with excitation light, and thus the observation of the target living body may not be sufficiently carried out.

A dipyrromethene boron complex (also referred to as BODIPY) is known as a fluorescent dye that has a high quantum yield and exhibits sharp emission characteristics and thus is used in various fields.

For example, WO2016/143699A discloses a dipyrromethene boron complex in which a halogenoalkyl group is bonded to a boron atom, as a compound useful as an amyloid oxygenation catalyst. Further, RSC Advances, 2016, vol.6, 38, p. 32070-32073 discloses, as a compound useful for a photocatalyst that carries out a photooxygenation reaction, a hybrid compound of a dipyrromethene boron complex having enhanced hydrophilicity and nanocellulose, where the dipyrromethene boron complex has a carboxy group as a biological substance reactive group at the meso position and has sulfo groups at the second and sixth positions of the bipyrromethene skeleton.

### SUMMARY OF THE INVENTION

While the dipyrromethene boron complex has the above-described advantages, it is generally inferior in water solubility and has low light resistance. Accordingly, in order to apply the dipyrromethene boron complex to a tool for living body observation, such as in vivo fluorescence imaging, it is necessary to impart both water solubility and light resistance to the dipyrromethene boron complex at a high level. In particular, in consideration of the actual conditions of in vivo fluorescence imaging, it is important that fluorescently labeled biological substances to be obtained can be imparted with excellent light resistance in the biological tissue.

However, the dipyrromethene boron complex described in WO2016/143699A does not have a sufficient level of water solubility and also does not have a sufficient level of light resistance in an aqueous solution. Further, it cannot be said that a fluorescently labeled biological substance, which is obtained from the dipyrromethene boron complex described in RSC Advances, 2016, vol.6, 38, p. 32070-32073, has sufficient light resistance in vivo. The further improvement of the dipyrromethene boron complex is still required for both the above-described water solubility and the excellent light resistance of the fluorescently labeled biological substance to be obtained, in the biological tissue. According to the studies by the inventors of the present invention it was found that such a highly water-soluble dipyrromethene boron complex as described in RSC Advances, 2016, vol.6, 38, p. 32070-32073 is prone to fading, and it is difficult to develop a dipyrromethene boron complex that has both light resistance and solubility, in particular, in terms of the water solubility of the compound and the light resistance of the fluorescently labeled antibody in the biological tissue.

An object of the present invention is to provide a compound that has a dipyrromethene boron complex structure, exhibits excellent hydrophilicity required for the use application to fluorescence labeling of biological substances, and can impart excellent light resistance to fluorescently labeled biological substances in the biological tissue. In addition, another object of the present invention is to provide a fluorescently labeled biological substance having excellent light resistance in the biological tissue, which is obtained by bonding this fluorescent compound to a biological substance.

The inventors of the present invention speculated that the fading of the existing dipyrromethene boron complex having water solubility is mainly caused by photolysis due to reactive oxygen species in the boron atom moiety. Based on this idea, the inventors of the present invention carried out diligent studies and as a result, have found that in a case where at least one alkyl group is bonded to a boron atom in a dipyrromethene boron complex, and a hydrophilic group is further introduced to obtain a specific complex structure, the antioxidative property of the dipyrromethene boron complex compound can be enhanced while maintaining high water solubility. Furthermore, it has been found that in a case where this dipyrromethene boron complex compound is used as a labeling compound (dye) for a biological substance, it is possible to obtain a labeled biological substance (fluorescent dye) having excellent light resistance in the biological tissue. The present invention has been completed through further studies based on these findings.

That is, the above objects of the present invention have been achieved by the following means.
<1> A compound represented by Formula (1), 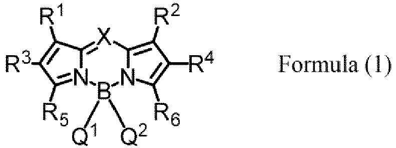 in the formula, X represents CR⁷ or N,
   R¹ to R⁷, Q¹, and Q² represent a hydrogen atom, a halogen atom, a cyano group, or a group represented by Formula (A), and adjacent substituents among R¹ to R⁷, Q¹, and Q² may form a ring structure,
   provided that at least one of R³, R⁴, R⁷, Q¹, or Q² contains at least one group in the following group Pi of hydrophilic groups, and at least one of Q¹ or Q² represents an alkyl group,

      ∗-L³-R¹¹¹ Formula (A)
   in the formula, L³ is a single bond or a linking group obtained by combining one or more kinds of groups among an alkylene group, an alkenylene group, an alkynylene group, an arylene group, a heteroarylene group, and groups respectively represented by Formula (1-1) to Formula (1-9),
   R¹¹¹ represents a hydrogen atom, an alkyl group, an alkenyl group, an alkynyl group, an aryl group, a heteroaryl group, or a monovalent aliphatic heterocyclic group,
   provided that, in a case where the hydrogen atom in R¹¹¹ is a dissociative hydrogen atom, a salt may be formed, and each group in L³ and R¹¹¹ may further have a substituent, and
   ^{∗} represents a bonding portion, 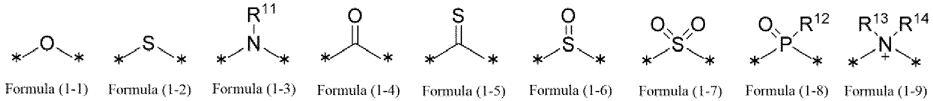
   in the formula, R¹¹ to R¹⁴ represent a hydrogen atom or a substituent, and
   ^{∗} represents a bonding portion.
   <the group Pi of hydrophilic groups>
   a carboxy group or a salt thereof, a sulfo group or a salt thereof, a phosphono group or a salt thereof, an onio group, and a polyamino acid residue.
<2> The compound according to <1>, in which X is CR⁷, and this R⁷ is a hydrogen atom or the group represented by Formula (A).
<3> The compound according to <1> or <2>, in which at least one of R³, R⁴, or R⁷ has at least one group of the group Pi of hydrophilic groups.
<4> The compound according to any one of <1> to <3>, in which at least one of R³ or R⁴ is the group represented by Formula (A), and the group represented by this Formula (A) has at least one group in the following group Pi-1 of hydrophilic groups,
   <the group Pi-1 of hydrophilic groups>
   a carboxy group or a salt thereof, a sulfo group or a salt thereof, and a phosphono group or a salt thereof.
<5> The compound according to <4>, in which L³ in the group represented by Formula (A) is a linking group obtained by combining two or more kinds of groups among an alkenylene group, an alkynylene group, an arylene group, a heteroarylene group, and groups respectively represented by Formula (1-1), Formula (1-3), and Formula (1-4), Formula (1-7), and Formula (1-8), and has at least one group in the group Pi-1 of hydrophilic groups.
<6> The compound according to any one of <1> to <5>, in which at least one of R³ or R⁴ is a carboxy group or a salt thereof, a sulfo group or a salt thereof, or a phosphono group or a salt thereof.
<7> The compound according to any one of <1> to <6>, in which at least one of R³ or R⁴ is a sulfo group or a salt thereof.
<8> The compound according to any one of <1> to <7>, in which Q¹ is a halogen atom, an alkyl group, an alkynyl group, an aryl group, a hydroxy group, or an alkoxy group.
<9> The compound according to any one of <1> to <8>, in which Q² is an alkyl group having 1 to 4 carbon atoms.
<10> The compound according to any one of <1> to <9>, in which Q² is a halogenoalkyl group having 1 to 4 carbon atoms.
<11> The compound according to any one of <1> to <10>, in which R⁷ is a hydrogen atom, an aryl group, or a heteroaryl group.
<12> The compound according to any one of <1> to <11>, in which R⁷ is a hydrogen atom or a group represented by Formula (C), 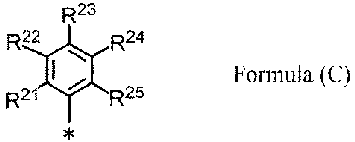
   in the formula, R²¹ represents a substituent, and R²² to R²⁵ represents a hydrogen atom or a substituent, and
   ^{∗} represents a bonding portion.
<13> The compound according to <1>, in which the compound is represented by any of the following compounds (1) to (7), 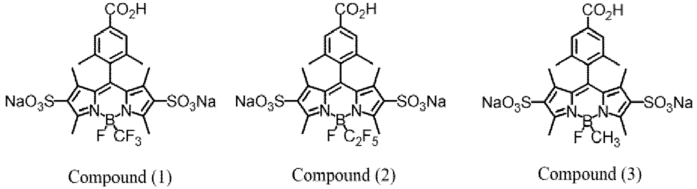 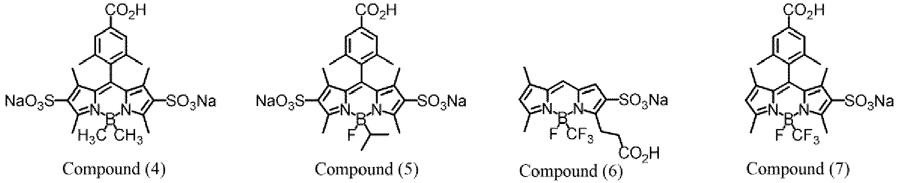
<14> The compound according to <9>, in which Q¹ is a halogen atom, and R⁷ is an aryl group.
<15> The compound according to <14>, in which both R³ and R⁴ are a sulfo group or a salt thereof.
<16> The compound according to <15>, in which Q² is a halogenoalkyl group having 1 to 4 carbon atoms.
<13> The compound according to any one of <1> to <16>, in which at least one of R¹ to R⁷, Q¹, or Q² has a moiety bondable to a biological substance.
<14> A fluorescently labeled biological substance, which is obtained by bonding between the compound according to <17> and a biological substance.
<15> The fluorescently labeled biological substance according to <18>, in which the biological substance is any one of a protein, a peptide, an amino acid, a nucleic acid, a sugar chain, or a lipid.
<16> The fluorescently labeled biological substance according to <18> or <19>,
   in which the bonding between the compound and the biological substance is bonding formed by any one of the followings i) to v),
   i) non-covalent or covalent bond between peptides,
   ii) Van der Waals interaction between a long-chain alkyl group in a compound and a lipid bilayer or lipid in a biological substance,
   iii) an amide bond formed by reacting an N-hydroxysuccinimide ester in a compound with an amino group in a biological substance,
   iv) a thioether bond formed by reacting a maleimide group in a compound with a sulfanyl group in a biological substance, and
   v) a bond associated with a formation of a triazole ring, which is formed by Click reaction between an azido group in a compound and an acetylene group in a biological substance, or between an acetylene group in a compound and an azido group in a biological substance.

The compound according to the embodiment of the present invention is a compound capable of imparting excellent hydrophilicity required for the use application to fluorescence labeling of biological substances and excellent light resistance to fluorescently labeled biological substances be obtained, in the biological tissue. In addition, the fluorescently labeled biological substance according to the embodiment of the present invention has excellent light resistance in the biological tissue and can be suitably used in the biological observation using fluorescence labeling such as in vivo fluorescence imaging.

### DESCRIPTION OF THE PREFERRED EMBODIMENTS

In the present invention, in a case where there are a plurality of substituents or linking groups represented by a specific symbol or formula (hereinafter, referred to as substituents or the like), or in a case where a plurality of substituents or the like are regulated at the same time, the substituents or the like may be the same or different from each other, unless otherwise specified. The same applies to the regulation of the number of substituents or the like. Further, in a case where a plurality of substituents or the like come close to each other (particularly in a case where they are adjacent to each other), they may be linked to each other to form a ring, unless otherwise specified. Further, unless otherwise specified, rings such as an alicyclic ring, an aromatic ring, and a heterocycle may be condensed to form a fused ring.

In the present invention, in a case where E type and Z type of the double bond are present in the molecule, any one of the E type or the Z type, or a mixture thereof may be used unless otherwise specified. In addition, in a case where a compound has a diastereomer and an enantiomer, any one of the diastereomer or the enantiomer may be used, or a mixture thereof may be used unless otherwise specified. For example, in the compound according to the embodiment of the present invention represented by General Formula (1), enantiomers having a boron atom as an asymmetric center may be present. As a result, as long as the compound according to the embodiment of the present invention represented by General Formula (1) includes at least one of the above-described enantiomers having a boron atom as an asymmetric center, it may be a mixture of the enantiomers, and compounds (6) and (7) and labeled antibodies (6) and (7) thereof are a mixture of enantiomers having a boron atom as an asymmetric center. Further, a compound of which the structure is specifically described as the compound represented by General Formula (1) according to the embodiment of the present invention is used in the sense of including an enantiomer in addition to the compound having the described structure.

In the present invention, the representation of a compound (including a complex) or substituent is used to mean not only the compound itself but also a salt thereof, and an ion thereof. For example, in a case where a group selected from the specific groups Pi and Pi-1 of hydrophilic groups described later or the like has a dissociative hydrogen atom, the hydrogen atom may be dissociated to form a corresponding salt structure. Examples of such a group having a dissociative hydrogen atom include a carboxy group, a sulfo group, and a phosphono group.

In a case of a salt structure, the kind of the salt may be one kind, two or more kinds may be mixed, the group having the salt form and the group having the free acid structure may be mixed in the compound, and the compound having the salt structure and the compound having the free acid structure compound may be mixed.

In addition, it is meant to include those in which a part of the structure is changed within the scope that does not impair the effects of the present invention. Furthermore, it is meant that a compound, which is not specified to be substituted or unsubstituted, may have any substituent within the scope that does not impair the effects of the present invention. The same applies to a substituent (for example, a group represented by "alkyl group", "methyl group", "methyl") and a linking group (for example, a group represented by "alkylene group", "methylene group", "methylene"). Among such substituents, a preferred substituent in the present invention is a substituent selected from a substituent group T described later.

In the present invention, the dipyrromethene boron complex structure means a structure in which a dipyrromethene skeleton is coordinated to a boron atom in at least a bidentate coordination. That is, it is formed at least by coordinating two nitrogen atoms of dipyrromethene to a boron atom.

Further, the compound according to the embodiment of the present invention includes a compound in which a specific alkyl group having at least one of Q¹ or Q² is bonded to an adjacent substituent (for example, R⁵ or R⁶) on the dipyrromethene skeleton to form a ring structure, and the dipyrromethene skeleton coordinates at the 3- or 4-position with respect to the boron atom. However, in the present invention, the dipyrromethene skeleton is preferably coordinated in the bidentate coordination with respect to the boron atom. Further, in the compound according to the embodiment of the present invention, adjacent substituents among R¹ to R⁶ may be bonded to each other to form a ring, thereby forming a fused-ring structure. Examples of the combination of the substituents to be bonded to form a ring include R¹ and R³, R³ and R⁵, R² and R⁴, and R⁴ and R⁶. The number of rings to be formed is not particularly limited as long as it can be structurally adopted, and a plurality of rings may be formed.

Further, in the chemical structural formulae described in the present invention, the positive charge on one of the two nitrogen atoms of dipyrromethene and the negative charge on the boron atom are omitted.

In the present invention, in a case where the number of carbon atoms of a certain group is specified, this number of carbon atoms means the number of carbon atoms of the entire group thereof unless otherwise specified in the present invention or the present specification. That is, in a case where this group has a form of further having a substituent, it means the total number of carbon atoms, to which the number of carbon atoms of this substituent is included.

In addition, in the present invention, the numerical range indicated by using "to" means a range including the numerical values before and after "to" as the lower limit value and the upper limit value, respectively.

The compound according to an embodiment of the present invention is represented by Formula (1). The compound represented by this Formula (1) is a dipyrromethene boron complex compound having a dipyrromethene boron complex structure in which the dipyrromethene skeleton is coordinated at least in the bidentate coordination with respect to the boron atom. As a result, it is conceived that the dipole moment and the transition dipole are orthogonal to each other, and thus strong fluorescence is exhibited even in a highly polar solvent.

In addition, the details of the reason why the compound according to the embodiment of the present invention exhibits sufficient hydrophilicity (high water solubility) and antioxidative property required for the use application to fluorescence labeling of biological substances, and can impart excellent light resistance to fluorescently labeled biological substances in the biological tissue, are not clear, but it is conceived to be as follows.

The compound according to the embodiment of the present invention is a compound in which at least one alkyl group is bonded to a boron atom in a dipyrromethene boron complex, and is a compound having a specific structure into which a group selected from a specific group Pi of hydrophilic groups is introduced. The alkyl group is sterically bulkier than the fluorine atom, which is a general boron ligand, and improves the stability (the antioxidative property) of the boron atom by suppressing the approach of the photolysis factor substances such as reactive oxygen, and as a result, it is conceived that in a case where the compound according to the embodiment of the present invention binds to a biological substance, it is possible to improve the light resistance of the fluorescently labeled biological substance in the biological tissue. Further, it is conceived that the energy level of the highest occupied molecular orbital (HOMO) of the compound can be decreased by replacing the above alkyl group with a halogenoalkyl group substituted with a halogen atom, the stability of the boron atom in the compound (the complex) can be improved, and the light resistance in the aqueous solution can also be further improved. Furthermore, it is conceived that in a case where at least one alkyl group is boned to the boron atom and then a specific hydrophilic group is introduced, hydrophilicity can be imparted to the compound while the stability of the boron atom is maintained. As a result of the above, it is conceived that the compound according to the embodiment of the present invention inhibits photolysis of the boron atom moiety due to reactive oxygen, whereby the excellent light resistance can be imparted to the fluorescently labeled biological substance in the biological tissue, and sufficient hydrophilicity can be exhibited as well.

Hereinafter, the compound according to the embodiment of the present invention, which is represented by Formula (1), will be described in detail.

### <Compound represented by Formula (1)>

The compound according to the embodiment of the present invention, which is represented by Formula (1), is as follows and emits fluorescence. Hereinafter, the compound according to the embodiment of the present invention, which is represented by Formula (1), is also referred to as a fluorescent compound.

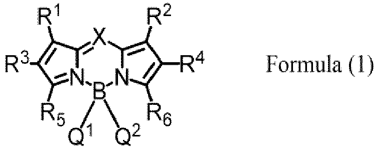

### In the formula, X represents CR⁷ or N.

R¹ to R⁷, Q¹, and Q² represent a hydrogen atom, a halogen atom, a cyano group, or a group represented by Formula (A). Adjacent substituents among R¹ to R⁷, Q¹, and Q² may be linked to each other to form a ring structure.

However, at least one of R³, R⁴, R⁷, Q¹, or Q² contains at least one group of the group Pi of hydrophilic groups described later. Further, at least one of Q¹ or Q² represents an alkyl group.

Hereinafter, the substituent and the like in Formula (1) will be described in detail.

. Group represented by Formula (A)

∗-L³-R¹¹¹ Formula (A)

In the formula, L³ is a single bond or a linking group obtained by combining one or more kinds of groups among an alkylene group, an alkenylene group, an alkynylene group, an arylene group, a heteroarylene group, and groups respectively represented by Formula (1-1) to Formula (1-9).

R¹¹¹ represents a hydrogen atom, an alkyl group, an alkenyl group, an alkynyl group, an aryl group, a heteroaryl group, or a monovalent aliphatic heterocyclic group,

However, in a case where the hydrogen atom in R¹¹¹ is a dissociative hydrogen atom, a salt may be formed.

In addition, each group in L³ and R¹¹¹ may further have a substituent.

Further, it is not allowed that L³ is a single bond and R¹¹¹ is a hydrogen atom at the same time.
^{∗} represents a bonding portion.

For the group represented by Formula (A), R¹¹¹ shall be determined based on the following rules (i) to (iv), and the remainder of the group shall be interpreted as L³. It is noted that a rule (i) shall be given the highest priority, a rule (ii) and subsequently a rule (iii) shall be applied in this order, and then finally a rule (iv) shall be applied.
(i) A case where group represented by Formula (A) has hydrophilic group Pi described later

The terminal portion of the hydrophilic group Pi is interpreted as a group represented by R¹¹¹.

Specifically, it is as follows.

The carboxy group is a group which is represented by a linking group obtained by combing a group represented by L³: Formula (1-4) with a group represented by Formula (1-1), and which is by R¹¹¹: a hydrogen atom.

The sulfo group is a group which is represented by a linking group obtained by combining a group represented by L³: Formula (1-7) with a group represented by Formula (1-1), and which is by R¹¹¹: a hydrogen atom.

The phosphono group is a group which is represented by a linking group obtained by combining a group represented by L³: a group represented by Formula (1-8) where R¹² is -OH, with a group represented by Formula (1-1), and which is by R¹¹¹: a hydrogen atom.

The onio group is a group which is represented by L³: a group represented by Formula (1-9), and which is by R¹¹¹: a hydrogen atom, an alkyl group, an alkenyl group, an alkynyl group, an aryl group, a heteroaryl group, or a monovalent aliphatic heterocyclic group.

The polyamino acid residue is a group which is represented by a group in which the terminal amino group in the polyamino acid residue is represented by a group represented by L³: Formula (1-3) and a group represented by R¹¹¹: a hydrogen atom, or a linking group obtained by combining a group in which the terminal carboxy group in the polyamino acid reside is represented by L³: Formula (1-4) with a group represented by Formula (1-1), and which is represented by R¹¹¹: a hydrogen atom.

It is noted that the groups formed by dissociating hydrogen atoms in the hydrophilic group Pi as the dissociative hydrogen atoms respectively correspond to a salt of the carboxy group, a salt of the sulfo group, or a salt of the phosphono group. This salt is synonymous with a salt described below in the specific hydrophilic group Pi.

However, in a case where the group represented by Formula (A) has two or more hydrophilic groups Pi, the above rule (i) is applied to a hydrophilic group Pi located at the position where the number of bonded atoms for shortest bonding from ^{∗} (the bonding portion) to the hydrophilic group Pi is the maximum number, and the remaining hydrophilic groups Pi are interpreted as substituents further contained in the substituent in L³.

(ii) A case where group represented by Formula (A) is alkyl group

The group is interpreted as a group in which L³ is a single bond and R¹¹¹ is an alkyl group.

(iii) In group represented by Formula (A), terminal structure of group represented by Formula (A) is alkyl group, alkenyl group, alkynyl group, aryl group, heteroaryl group, or monovalent aliphatic heterocyclic group

The group is interpreted as a group in which R¹¹¹ represents an alkyl group, an alkenyl group, an alkynyl group, an aryl group, a heteroaryl group, or a monovalent aliphatic heterocyclic group.

However, in the above (iii), the "terminal structure of group represented by Formula (A)" means a structure located at a position closest to the terminal in the longest bonded chain in the group represented by Formula (A) in a case of being counted from ^{∗} (the bonding portion).

In the above (iii), in a case where the group represented by Formula (A) is a group having a moiety bondable to a biological substance described later, the above-described "terminal structure of group represented by Formula (A)" is read with respect to a group in which a moiety bondable to a biological substance described later in the group represented by Formula (A) is replaced with a hydrogen atom. That is, a moiety bondable to a biological substance described later is interpreted as a substituent which may be further contained in each group in L³ or R¹¹¹.

(iv) For a group represented by Formula (A) that does not correspond to the above (i) to (iii), R¹¹¹ and L³ are determined in order from the terminal structure side of the group represented by Formula (A).

In this case, the alkyl group, the alkenyl group, the alkynyl group, the aryl group, the heteroaryl group, and the monovalent aliphatic heterocyclic group, which can be adopted as R¹¹¹, may be interpreted as the substituent which further has a substituent.

### (L³)

The alkylene group that can be adopted as L³ is synonymous with the group in which one hydrogen atom is further removed from the alkyl group selected from the substituent group T, which is described later, and the same applies to the preferred one thereof.

The alkenylene group that can be adopted as L³ is synonymous with the group in which one hydrogen atom is further removed from the alkenyl group selected from the substituent group T, which is described later, and the same applies to the preferred one thereof.

The alkynylene group that can be adopted as L³ is synonymous with the group in which one hydrogen atom is further removed from the alkynyl group selected from the substituent group T, which is described later, and the same applies to the preferred one thereof.

The arylene group that can be adopted as L³ is synonymous with the group in which one hydrogen atom is further removed from the aryl group selected from the substituent group T, which is described later, and the same applies to the preferred one thereof.

The heteroarylene group that can be adopted as L³ is synonymous with the group in which one hydrogen atom is further removed from the heteroaryl group selected from the substituent group T, which is described later, and the same applies to the preferred one thereof.

The alkylene group, the alkenylene group, the alkynylene group, the arylene group, and the heteroarylene group, which can be adopted as L³, may be an unsubstituted group or a group having a substituent.

The substituent which may be contained in the alkylene group, the alkenylene group, the alkynylene group, the arylene group, and the heteroarylene group, which can be adopted as L³, is not particularly limited, and it is preferably selected from a substituent group T described later. More preferred examples thereof include a halogen atom, an alkoxy group, and a group Pi of hydrophilic groups described later.

Examples of the halogen atom include a fluorine atom, a chlorine atom, a bromine atom, and an iodine atom, where a fluorine atom is preferable.

Further, the number of substituents that can be contained in the alkylene group, the alkenylene group, the alkynylene group, the arylene group, and the heteroarylene group, which can be adopted as L³, is not particularly limited as long as it can be adopted for the structure, and it can be one or more. The upper limit value thereof is not particularly limited, and for example, all hydrogen atoms in the alkylene group, the alkenylene group, the alkynylene group, the arylene group, and the heteroarylene group may be substituted with a substituent.

In the alkylene group, the alkenylene group, the alkynylene group, the arylene group, and the heteroarylene group, which can be adopted as L³, and one or more kinds of groups among the groups respectively represented by Formula (1-1) to Formula (1-9), the kind of group to be combined is not particularly limited as long as the group to be combined has a reasonable chemical structure; however, a linking group obtained by combining 1 to 6 kinds is preferable, and a linking group obtained by combining 1 to 3 kinds is more preferable. That is, L³ does not include a group in which two or more groups each represented by any of Formulae (1-1) to (1-3) are consecutive.

In the linking group obtained by combining an alkylene group, an alkenylene group, an alkynylene group, an arylene group, and an heteroarylene group, which can be adopted as L³, and one or more kinds of groups among the groups respectively represented by Formula (1-1) to Formula (1-9), the number of groups to be combined is not particularly limited; however, preferred examples thereof include 2 to 20 000, where 2 to 2,000 is more preferable, and 2 to 200 is still more preferable.

The groups represented by any of Formula (1-1) to (1-9), which can be adopted as L³, are as follows.

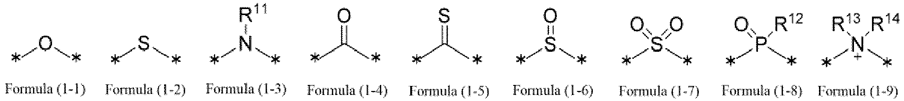

In the formulae, R¹¹ to R¹⁴ each independently represent a hydrogen atom or a substituent.

R¹³ and R¹⁴ may be bonded to form a ring.
^{∗} represents a bonding portion.

The substituent that can be adopted as R¹¹, R¹³, and R¹⁴ is not particularly limited, and it is preferably selected from the substituent group T, which is described later. R¹¹, R¹², and R¹³ are each independently preferably a hydrogen atom, an alkyl group, an aryl group, a heteroaryl group, an acyl group, or a sulfonyl group (a sulfonyl group substituted with an alkyl group, cycloalkyl group, or an aryl group, and more preferably an alkylsulfonyl group), and more preferably a hydrogen atom or an alkyl group.

All the alkyl group, the aryl group, the heteroaryl group, the acyl group, and the sulfonyl group, which can be adopted as R¹¹, R¹³, and R¹⁴, may be unsubstituted group or a group having a substituent. The above group having a substituent is not particularly later; however, preferred examples thereof include an alkyl group, an aryl group, a heteroaryl group, an acyl group, and a sulfonyl group, which have as a substituent a specific hydrophilic group Pi described later.

The substituent that can be adopted as R¹² is not particularly limited and is preferably selected from the substituent group T, which is described later. R¹² is preferably a hydrogen atom, a hydroxy group, an alkoxy group, an aryloxy group, an alkyl group, an aryl group, or a heteroaryl group, more preferably a hydroxy group, an alkoxy group, or an aryloxy group, and still more preferably a hydroxy group.

Preferred examples of the group obtained by combining groups represented by any of Formulae (1-1) to (1-9) include groups represented by any of Formulae (1A-1) to (1A-9).

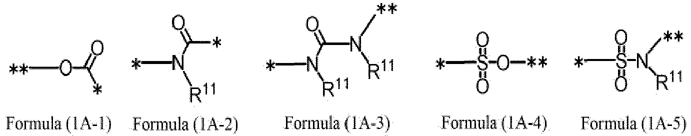

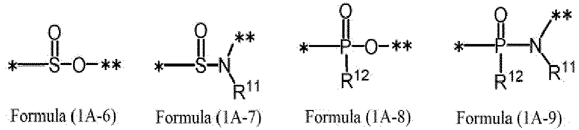

R¹¹ and R¹² respectively are synonymous with the above R¹¹ and R¹².
^{∗} and ^{∗∗} represents a bonding portion. In addition, ^{∗∗} represents a bonding portion to the R¹¹¹ side in a case where the group is a group that can be adopted as L³. Formula (1A-2) may be bonded to R¹¹¹ on any ^{∗} side in L³.

Examples of the linking group obtained by combining the groups represented by any of Formula (1-1) to (1-9), which can be adopted as the above L³, is preferably a group represented by any of Formula (1A-1), (1A-2), (1A-4), or (1A-8), and more preferably a group represented by any of Formula (1A-1) or (1A-4).

Groups represented by Formula (1A-1), (1A-4), and (1A-8) in which R¹² is a hydroxy group, together with the group represented by R¹¹¹ as a hydrogen atom respectively corresponds to a carboxy group, a sulfo group, and phosphono group, as the specific hydrophilic group Pi. In addition, the groups formed by dissociating hydrogen atoms from these groups as the dissociative hydrogen atoms respectively correspond to a salt of the carboxy group, a salt of the sulfo group, or a salt of the phosphono group.

Further, L³ may be a linking group obtained by combining each group represented by any of Formula (1-1) to (1-9) or a linking group obtained by combining these groups, with at least one or more of an alkylene group, an alkenylene group, an alkynylene group, and an arylene group, or it may be a linking group obtained by linking two or more kinds of groups represented by any of Formula (1-1) to (1-9) or two or more linking group obtained by combining these groups, through a group obtained by combining one or two or more of an alkylene group, an alkenylene group, an alkynylene group, an arylene group, and a heteroarylene group.

Specific examples of the linking group obtained by combining two or more kinds of groups that can be adopted as L³ include a group obtained by combining at least 2 kinds (preferably 2 to 4 kinds) of alkylene groups, alkenylene groups, alkynylene groups, arylene groups, and heteroarylene groups; and a linking group obtained by combining at least one kind (preferably 1 to 4 kinds) of an alkylene group, an arylene group, or an heteroarylene group with at least one kind (preferably 1 to 4 kinds) of a group represented by any of Formula (1-1) to (1-9).

Examples of the group having a repeating structure among the linking groups obtained by combining two or more kinds of groups, which can be adopted as L³, include a group represented by alkylene group - [group represented Formula (1A-2) - alkylene group] - group represented any of Formula (1A-1), (1A-4), or (1A-8); a group represented by arylene group - [group represented by Formula (1A-2) - alkylene group]- group represented by any of Formula (1A-1), (1A-4), or (1A-8); a group represented by arylene group - group represented by Formula (1A-2) - [alkylene group - group represented by Formula (1-1)] - alkylene group - group represented by any of Formula (1A-1), (1A-4), or (1A-8); and a group represented by alkylene group - group represented by Formula (1A-2) - [alkylene group - group represented by Formula (1-1)] - alkylene group - group represented by any of Formula (1A-1), (1A-4), or (1A-8). It is noted that [] indicates a repeating structure.

### (R¹¹¹)

In a case where the hydrogen atom that can be adopted as R¹¹¹ is a dissociative hydrogen atom, the dissociative hydrogen atom may be dissociated from the group represented by Formula (A), thereby forming a salt. This salt is synonymous with a salt described below in the specific hydrophilic group Pi.

The description that a hydrogen atom is dissociative means, for example, that the acid dissociation constant (pKa) is 10 or less, preferably 7 or less, and more preferably 5 or less. The acid dissociation constant means a value at 25°C in water.

The alkyl group, the alkenyl group, the alkynyl group, the aryl group, the heteroaryl group, and the monovalent aliphatic heterocyclic group, which can be adopted as R¹¹¹, are respectively synonymous with the corresponding groups in the substituent group T, and the same applies to the preferred ones.

All the alkyl group, the alkenyl group, the alkynyl group, the aryl group, the heteroaryl group, and the monovalent aliphatic heterocyclic group, which can be adopted as R¹¹¹, may be an unsubstituted group or a group having a substituent.

The substituent which may be contained in each of the above groups that can be adopted as R¹¹¹ is not particularly limited, and examples thereof include a group selected from a substituent group T described later, where a halogen atom is preferable. Examples of the halogen atom include a fluorine atom, a chlorine atom, a bromine atom, and an iodine atom, where a fluorine atom is preferable. In addition, it may have as a substituent a moiety bondable to a biological substance described later.

The number of substituents which may be contained in each of the above groups that can be adopted as R¹¹¹ is not particularly limited as long as it can be adopted for the structure, and it may be at least one. The upper limit value thereof is not particularly limited, and for example, all hydrogen atoms in the alkyl group, the alkenyl group, the alkynyl group, the heteroaryl group, and the monovalent aliphatic heterocyclic group may be substituted with a substituent.

Among the alkyl groups that can be adopted as R¹¹¹, preferred examples of the alkyl group having a substituent include a halogenoalkyl group. The halogenoalkyl group that can be adopted as R¹¹¹ is synonymous with the alkyl group in the substituent group T, except that at least one hydrogen atom in the alkyl group in the substituent group T is substituted with a halogen atom, and the same applies to the preferred one thereof.

Examples of the halogen atom include a fluorine atom, a chlorine atom, a bromine atom, and an iodine atom, where a fluorine atom is preferable.

The number of halogen atoms that constitute the halogenoalkyl group is not particularly limited, and the halogenoalkyl group may be, for example, a perhalogenoalkyl group.

Further, in a case where each of the above groups that can be adopted as R¹¹¹ has a substituent other than the hydrophilic group Pi described later, the halogen atom, and the moiety bondable to the biological substance described later, the number of substituents other than the hydrophilic group Pi described later, the halogen atom, and the moiety bondable to the biological substance described later can be, for example, one or more, and it is preferably one or two.

Specifically, examples of the preferred form of the group represented by Formula (A) include the following groups (1) and (2). However, in these examples, the form in which the substituents in L³ and R¹¹¹ have a substituent is not excluded, and it may be unsubstituted or may have a substituent. Regarding the substituent which may be contained in the substituents in L³ and R¹¹¹, the descriptions for the above-described substituent which may be contained in L³ and R¹¹¹ can be applied, respectively.
(1) A case having at least one group selected from group Pi of hydrophilic groups described later

In the group represented by Formula (A), L³ is a linking group obtained by combining one or more kinds of groups among an alkylene group, an alkenylene group, an alkynylene group, an arylene group, a heteroarylene group, and the groups respectively represented by Formula (1-1) to Formula (1-9), and R¹¹¹ is a hydrogen atom, an alkyl group, an alkenyl group, an alkynyl group, an aryl group, a heteroaryl group, or a monovalent aliphatic heterocyclic group.

In this case, L³ is preferably a linking group obtained by combining one or more kinds of groups among an alkylene group, an alkenylene group, an alkynylene group, an arylene group, a heteroarylene group, and the groups respectively represented by Formula (1-1), Formula (1-3), Formula (1-4), Formula (1-7), and Formula (1-8), and more preferably a linking group obtained by combining two or more kinds of groups among an alkylene group, an arylene group, and the groups respectively represented by Formula (1-1), Formula (1-4), and Formula (1-7). In these cases, R¹¹¹ is preferably a hydrogen atom. The group represented by Formula (A) more preferably has at least one group selected from the group Pi-1 of hydrophilic groups described later.

(2) A case where group selected from group Pi of hydrophilic groups described later is not contained

In the group represented by Formula (A), L³ is a single bond, and R¹¹¹ is a hydrogen atom, an alkyl group, an alkenyl group, an alkynyl group, an aryl group, a heteroaryl group, or a monovalent aliphatic heterocyclic group; or L³ is a linking group obtained by combining one or more kinds of groups among an alkylene group, an alkenylene group, an alkynylene group, an arylene group, a heteroarylene group, and groups respectively represented by Formulae (1-1) to (1-9), and R¹¹¹ is a hydrogen atom, an alkyl group, an alkenyl group, an alkynyl group, an aryl group, a heteroaryl group, or a monovalent aliphatic heterocyclic group.

In this case, it is preferable that L³ is a single bond or a group represented by Formula (1-1) and R¹¹¹ is an alkyl group, and it is more preferable that L³ is a single bond and R¹¹¹ is an alkyl group.

### . R¹ to R⁶

R¹ to R⁶ each independently represent a hydrogen atom, a halogen atom, a cyano group, or a group represented by Formula (A), and they are preferably a hydrogen atom or a group represented by Formula (A) and more preferably a hydrogen atom or a group having a preferred form of the above (1) or the above (2).

Examples of the halogen atom that R¹ to R⁶ can adopt include a fluorine atom, a chlorine atom, a bromine atom, and an iodine atom, where a fluorine atom is preferable.

Hereinafter, more preferred groups of R¹ to R⁶ will be described more specifically. However, in each description, each group may further have a substituent (a moiety bondable to a biological substance described later or the like).

R¹ and R² are still more preferably a hydrogen atom or a group having a preferred form of the above (2).

Examples of the group having a preferred form of the above (2) include a group in which L³ is a single bond and R¹¹¹ is an alkyl group, and a group in which L³ is a group represented by Formula (1-1) and R¹¹¹ is an alkyl group.

Examples of R¹ and R² include a hydrogen atom, an alkyl group, and an alkoxy group, where a hydrogen atom or an alkyl group is preferable.

R³ and R⁴ are still more preferably a hydrogen atom or a group having a preferred form of the above (1).

Examples of the group having a preferred form of the above (1) include a group in which L³ a linking group obtained by combining an alkylene group, an alkenylene group, an alkynylene group, an arylene group, a heteroarylene group, and one or more groups of the groups respectively represented by Formula (1-1), Formula (1-3), Formula (1-4), Formula (1-7), and Formula (1-8), and has a group selected from the group Pi-1 of hydrophilic groups described later, and for example, a sulfo group or a salt thereof is included.

Regarding R³ and R⁴, it is preferable that at least one of R³ or R⁴ is a sulfo group or a salt thereof, and it is more preferable that both R³ and R⁴ are a sulfo group or a salt thereof.

In a case where at least one of R³ or R⁴ is a sulfo group or a salt thereof, the other thereof is preferably a hydrogen atom.

R⁵ and R⁶ are more preferably a hydrogen atom or a group having a preferred form of the above (1) or the above (2), and still more preferably a hydrogen atom or a group having a preferred form of the above (2).

Examples of the group having a preferred form of the above (1) include a group in which L³ is a linking group obtained by combining an alkylene group, Formula (1-1), and (1-4), and R¹¹¹ is a hydrogen atom.

Examples of the group having a preferred form of the above (2) include a group in which L³ is a single bond or a linking group obtained by combining an alkenylene group, an alkynylene group, and one or two or more kinds of groups represented by Formula (1-1), and R¹¹¹ is an alkyl group or an aryl group.

Examples of R⁵ and R⁶ include a hydrogen atom, an alkyl group, a carboxyalkyl group, an aryl group, an alkoxy group, an alkenylene - aryl group, an arylene - group represented by Formula (1-1) - alkyl group, an alkenylene - arylene- group represented by Formula (1-1) - alkyl group, and an alkynylene - arylene- group by Formula (1-1) - alkyl group, where an alkyl group or a carboxyalkyl group is preferable. Specific examples thereof include a methyl group and a CH₂CH₂COOH group.

### . X

X represents CR⁷ or N, and CR⁷ is preferable.

R⁷ represents a hydrogen atom, a halogen atom, a cyano group, or a group represented by Formula (A), and it is preferably a hydrogen atom or a group represented by Formula (A). The group represented by Formula (A) is preferably a group having a preferred form of the above (1) or the above (2).

Examples of the halogen atom that R⁷ can adopt include a fluorine atom, a chlorine atom, a bromine atom, and an iodine atom, where a fluorine atom is preferable.

In a case where X satisfies the above-described preferred form (that is, the form in which X is CR⁷), excellent light resistance can be imparted to a labeled biological substance in the biological tissue, and excellent fluorescence quantum yield can be imparted to a compound and a labeled biological substance. The reason for this is presumably due to a mechanism similar to the mechanism in which the photochemical degradation of the boron complex having bidentate dipyrromethene ligands easily occurs in a case where the meso position is N as described in The Journal of Physical Chemistry A, 2016, 120, p.2537-2546. In addition, as described in Analyst. 2014 Oct 7; 139 (19): p. 4862-4873, in a case where X is N, surrounding water molecules and N interacts with each other (for example, hydration) in the aqueous solution or the biological tissue, and the nonradiative deactivation such as vibration energy transition occurs, whereby the fluorescence quantum yield is generally low; however, in a case where X is CR⁷, the above interaction is suppressed, and thus the fluorescence quantum yield tends to be high.

The R⁷ is preferably a hydrogen atom, an alkyl group, an alkenyl group, an alkynyl group, an aryl group, a heteroaryl group, an aryloxy group, or a heteroaryloxy group.

Here, regarding the alkyl group, the alkenyl group, the alkynyl group, the aryl group, and the heteroaryl group, which are preferable as R⁷, the descriptions for the alkyl group, the alkenyl group, the alkynyl group, the aryl group, and the heteroaryl group in R¹¹¹ of Formula (A) can be applied respectively. Further, regarding the aryl group in the aryloxy group and the heteroaryl group in the heteroaryloxy group, which are preferable as R⁷ the descriptions for the aryl group and the heteroaryl group in R¹¹¹ of Formula (A) can be preferably applied, respectively.

The alkyl group, the alkenyl group, the alkynyl group, the aryl group, the heteroaryl group, the aryloxy group, and the heteroaryloxy group, which can be preferably adopted as R⁷, may be unsubstituted or have a substituent. The substituent which may be contained in the alkyl group, the alkenyl group, the alkynyl group, the aryl group, the heteroaryl group, the aryloxy group, and the heteroaryloxy group is not particularly limited; however, it is selected from a substituent group T described later. Preferred examples thereof include a halogen atom, an alkyl group, an alkenyl group, an alkoxy group, an alkylsulfanyl group, a hydrophilic group Pi described later, and a moiety bondable to a biological substance described later, where an alkyl group or a moiety bondable to a biological substance described later is more preferable. As the hydrophilic group Pi, the description for the hydrophilic group Pi described later can be preferably applied, and the hydrophilic group Pi is most preferably a group having a carboxy group or a salt thereof.

Specific examples of R⁷ include 3-carboxypropyl, 4-carboxy-2,6-dimethylphenyl, 4-carboxy-2, 6-dimethoxyphenyl, 4-carboxy-2, 6-difluorophenyl, and 4-carboxy-2, 6-dimethylphenyloxy.

The R⁷ is more preferably a hydrogen atom, an alkyl group, an aryl group, a heteroaryl group, an aryloxy group, or a heteroaryloxy group, still more preferably a hydrogen atom, an alkyl group, an aryl group, or a heteroaryl group, particularly preferably a hydrogen atom or an aryl group, and most preferably an aryl group.

Among the above, R⁷ is preferably an aryl group or a heteroaryl group from the viewpoint of inhibiting photolysis due to reactive oxygen by introducing a bulky substituent and further improving the light resistance (fading resistance) of the labeled biological substance in the biological tissue. Further, from the viewpoint of realizing a better fluorescence quantum yield, R⁷ is more preferably a hydrogen atom that is not a freely rotationable substituent or a group represented by Formula (C) in which the rotation of the benzene ring is suppressed, and R⁷ is still more preferably a group represented by Formula (C).

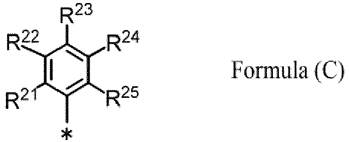

In the formula, R²¹ represents a substituent, R²² to R²⁵ represents a hydrogen atom or a substituent.
^{∗} represents a bonding portion.

The substituent in R²¹ is not particularly limited as long as it can suppress the rotation of the benzene ring in Formula (C), and it is preferably selected from a substituent group T described later. For example, R²¹ is preferably a halogen atom, an alkyl group, an alkenyl group, an alkynyl group, an alkoxy group, or an alkylsulfanyl group, and more preferably an alkyl group.

The substituent that can be adopted as R²² to R²⁵ is not particularly limited and is preferably selected from the substituent group T, which is described later. R²² to R²⁵ are preferably a halogen atom, an alkyl group, an alkenyl group, an alkynyl group, an alkoxy group, or an alkylsulfanyl group, and the substituent that can be adopted as R²² to R²⁵ is more preferably an alkyl group. In addition, preferred examples thereof include a hydrophilic group Pi described later and a moiety bondable to a biological substance described later.

The alkyl group, the alkenyl group, the alkynyl group, the alkoxy group, and the alkylsulfanyl group, which can be adopted as R²¹ to R²⁵, are respectively synonymous with the corresponding groups in the substituent group T, and the same applies to the preferred ones thereof.

The alkyl group, the alkenyl group, the alkynyl group, the alkoxy group, and the alkylsulfanyl group, which are substituents that can be adopted as R²¹ to R²⁵, may be an unsubstituted group or a group having a substituent. The substituent which may be contained in the above-described substituent (the alkyl group, the alkenyl group, the alkynyl group, the alkoxy group, or the alkylsulfanyl group) is not particularly limited; however, it is selected from, for example, a substituent group T described later. A hydrophilic group Pi described later or a moiety bondable to a biological substance described later is also preferable.

It is preferable that R²³ has a hydrophilic group Pi described later or a moiety bondable to a biological substance described later, and it is more preferable that R²³ has a carboxy group or a moiety bondable to a biological substance described later.

Among the above, it is more preferable that R²¹ is a substituent, R²³ has a hydrophilic group Pi described later or a moiety bondable to a biological substance described later, and at least R²⁵ of R²², R²⁴, and R²⁵ is a substituent, it is more preferable that R²¹ is a halogen atom, an alkyl group, an alkenyl group, an alkynyl group, an alkoxy group, or an alkylsulfanyl group, R²³ has a hydrophilic group Pi described later or a biological substance described later, and at least R²⁵ of R²², R²⁴, and R²⁵ is an alkyl group, an alkenyl group, an alkynyl group, an alkoxy group, or an alkylsulfanyl group, and it is particularly preferable that R²¹ is an alkyl group, R²³ has a carboxy group or a moiety bondable to a biological substance described later, and at least R²⁵ of R²², R²⁴, and R²⁵ is an alkyl group. In these cases, it suffices that R²² and R²⁴ is a hydrogen atom, a halogen atom, an alkyl group, an alkenyl group, an alkynyl group, an alkoxy group, or an alkylsulfanyl group, and a hydrogen atom is preferable.

### . Q¹ and Q²

Q¹ and Q² each independently represent a hydrogen atom, a halogen atom, a cyano group, or a group represented by Formula (A). However, at least one of Q¹ or Q² represents an alkyl group.

Examples of the halogen atom that Q¹, and Q² can adopt include a fluorine atom, a chlorine atom, a bromine atom, and an iodine atom, where a fluorine atom is preferable.

Q¹ is preferably a halogen atom, an alkyl group, an alkynyl group, an aryl group, a hydroxy group, or an alkoxy group, more preferably a halogen atom or an alkyl group, and still more preferably a halogen atom.

Q² is preferably an alkyl group and more preferably a halogenoalkyl group from the viewpoint of further improving the light resistance (in the aqueous solution) of the compound in addition to the excellent light resistance of the labeled biological substance in the biological tissue. It is noted that in a case where Q² is a halogenoalkyl group, it is possible to exhibit an excellent fluorescence quantum yield at the same level as that of the dipyrromethene boron complex in the related art, in which two fluorine atoms are coordinated to a boron atom. The number of carbon atoms of the alkyl group (including the halogenoalkyl group) is not particularly limited; however, it is preferably 1 to 15, more preferably 1 to 10, and still more preferably 1 to 4, from the viewpoint of achieving both the water solubility and the excellent light resistance of the labeled biological substance in the biological tissue.

In the halogenoalkyl group, at least one hydrogen atom of the alkyl group is substituted with a halogen atom. The number of halogen atoms that constitute the halogenoalkyl group is not particularly limited, and the halogenoalkyl group may be, for example, a perhalogenoalkyl group.

The alkyl group (including the halogenoalkyl group) may be linear or branched, and it may form a ring.

### <the group Pi of hydrophilic groups>

A carboxy group or a salt thereof, a sulfo group (-SO₃H) or a salt thereof, a phosphono group [-PO(OH)₂] or a salt thereof, an onio group, and a polyamino acid residue

At least one of R³, R⁴, R⁷, Q¹, or Q² has at least one group selected from the group Pi of hydrophilic groups.

The "salt" is meant to include a form in which a salt is formed in the molecule of the fluorescent compound represented by Formula (1).

Examples of the salt of carboxy group, sulfo group, and phosphono group include salts of alkali metals such as Na, Li, and K, salts of alkaline earth metals such as Mg, Ca, and Ba, and salts of organic amines such as tetraalkylammonium.

The number of the specific hydrophilic group Pi in the compound represented by Formula (1) is not particularly limited as long as any one of R³, R⁴, R⁷, Q¹, or Q² is included. The number of the hydrophilic groups Pi which can be contained in the compound represented by Formula (1) is preferably 1 to 6, more preferably 1 to 4, still more preferably 1 to 3, and particularly preferably 2 or 3, from the viewpoint of achieving both the hydrophilicity and the excellent light resistance of the labeled biological substance in the biological tissue.

In a case where the fluorescent compound according to the embodiment of the present invention, which is represented by Formula (1), has the above-described hydrophilic group Pi as a substituent that serves as a moiety bondable to a biological substance described later or a precursor thereof, the fluorescent compound according to the embodiment of the present invention, which is represented by Formula (1), practically has one or more hydrophilic groups Pi in addition to the group that serves as a moiety bondable to a biological substance, which is preferable. Specific examples thereof include a carboxy group or a salt thereof as a precursor of an NHS ester that can be a moiety bondable to a biological substance.

In a case where the fluorescent compound represented by Formula (1) contains a plurality of hydrophilic groups Pi or the like, the plurality of groups may have any one of a salt structure or a free acid structure and may be the same or different from each other.

Here, the "polyamino acid" means a compound in which two or more amino acids are linked by a peptide bond and has a concept including a peptide and a protein. The number of amino acids that constitute the "polyamino acid" is preferably 2 to 30, more preferably 4 to 20, and still more preferably 6 to 10. The "polyamino acid residue" is a group derived from the polyamino acid. The "polyamino acid residue" is preferably a group in which one of the hydrogen atoms of -NH₂, -CO₂H, -OH, and -SH contained in the amino acids constituting the polyamino acid is replaced with a bond (-) (for example, in a case where the hydrogen atom of -CO₂H is replaced with a bond, -CO₂H becomes -C(=O)-O-^{∗∗}. ^{∗∗} is a bonding portion served for the polyamino acid to be incorporated into the fluorescent compound of Formula (1)). Further, the group in which one of the hydrogen atoms of -NH₂, -CO₂H, -OH, and -SH contained in the amino acids that constitute the polyamino acid is replaced with a bond (-) may be incorporated as a polyamino acid residue via a bond such as >C=O and >NH.

Examples of the onio group include an ammonio group (including a cyclic ammonio such as a pyridinium salt, a piperidinium salt, a piperazinium salt, and a pyrrolidinium salt), a sulfonio group, and a phosphonio group, where an ammonio group is preferable.

Examples of the counter ion constituting the onio group include inorganic ions such as a sulfonate ion and an iodide ion.

The onio group may be in a form in which a constitutional counterion is contained as a substituent in the substituent or may be simply in a form in which it is contained simply as a counterion.

In the fluorescent compound according to the embodiment of the present invention, which is represented by Formula (1), it is preferable that at least one of R³, R⁴, or R⁷ has at least one group of the above group Pi of hydrophilic groups from the viewpoint of simultaneously imparting the excellent water solubility and the excellent light resistance of the labeled biological substance in the biological tissue.

Among the above, in a case of introducing an electron-withdrawing group among the above-described hydrophilic groups Pi into at least one of R³ or R⁴, which has a rich highest occupied molecular orbital (HOMO), an effect of deepening the oxidation potential of the whole molecule can be obtained, and as a result, it is conceived that the excellent light resistance of the excellent labeled biological substance in the biological tissue is imparted as a synergistic effect, in addition to water solubility (excellent hydrophilicity).

From the above viewpoint, it is preferable that at least one of R³ or R⁴ is a group represented by Formula (A), and the group represented by this Formula (A) has at least one group of the following group Pi-1 of hydrophilic groups.

### <the group Pi-1 of hydrophilic groups>

a carboxy group or a salt thereof, a sulfo group or a salt thereof, and a phosphono group or a salt thereof.

In the group represented by Formula (A), which contains at least one group among the group Pi-1 of hydrophilic groups, it is more preferable that L³ in Formula (A) is a linking group obtained by combining two or more kinds of groups among an alkylene group, an alkenylene group, an alkynylene group, an arylene group, a heteroarylene group, and groups respectively represented by Formula (1-1), Formula (1-3), and Formula (1-4), Formula (1-7), and Formula (1-8), and is a substituent having at least one group among the group Pi-1 of hydrophilic groups.

Among the above, at least one of R³ or R⁴ is more preferably a group selected from the group Pi-1 of hydrophilic groups, and still more preferably a sulfo group or a salt thereof.

Specific examples of the fluorescent compound according to the embodiment of the present invention, which is represented by Formula (1), are shown below; however, the present invention is not limited to these compounds. In the following specific examples, a group having a dissociative hydrogen atom such as a specific hydrophilic group Pi may adopt a salt structure by dissociating the dissociative hydrogen atom.

In the following exemplified compounds, Me represents a methyl group, iPr represents an isopropyl group, and Rf represents -CF(CF₃)₂.

Among the following exemplified compounds, the preferred ones are compounds corresponding to a compound (1) to a compound (7) described later. It is noted that the sulfo group in the compound (1) to the compound (7) described later is described in the form of a salt (-SO₃Na) having a sodium ion as a counterion. However, in addition to this form, the sulfo group may be a salt with another counterion described in the above-described group Pi of hydrophilic groups, or it may be a sulfo group (-SO₃H). Among them, the sulfo group in the compound (1) to the compound (7) described later is preferably in the form of a salt having a sodium ion as a counter ion.

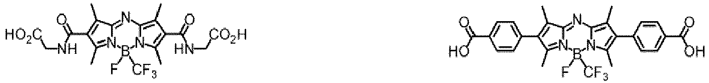

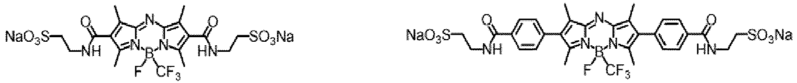

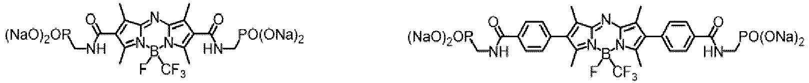

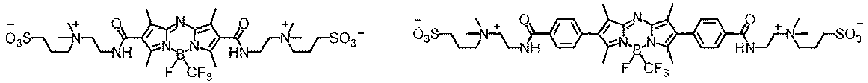

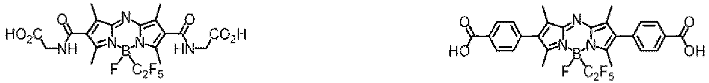

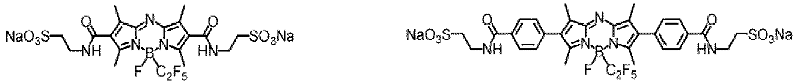

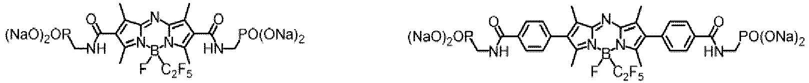

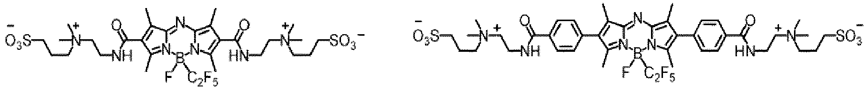

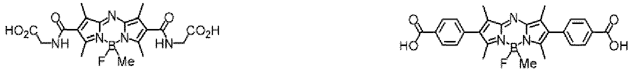

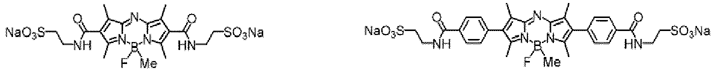

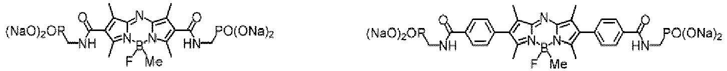

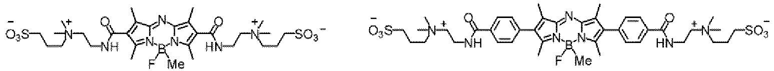

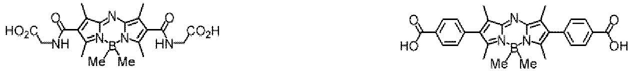

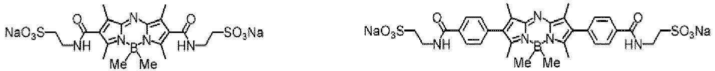

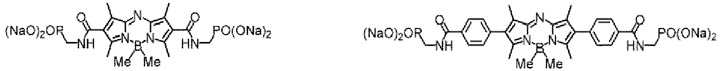

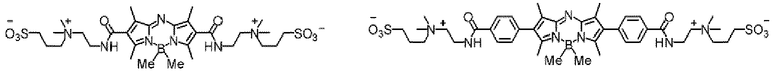

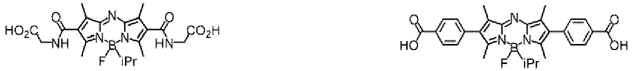

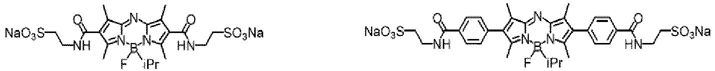

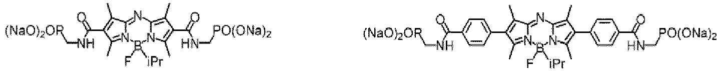

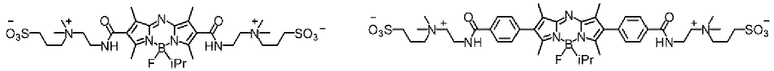

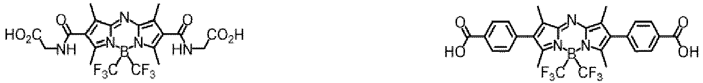

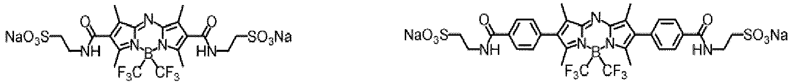

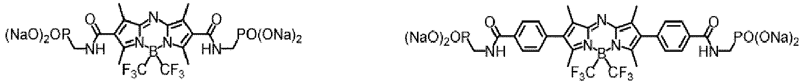

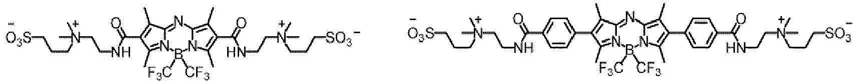

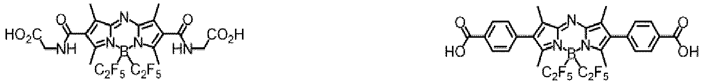

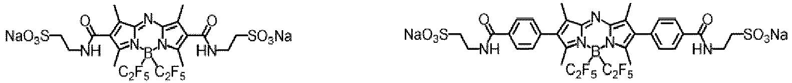

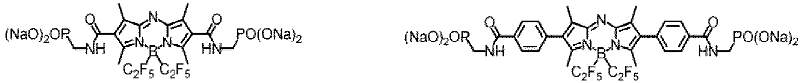

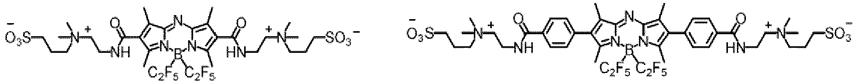

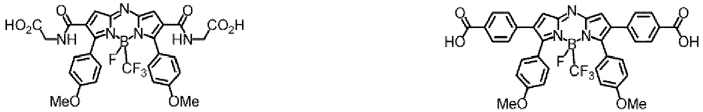

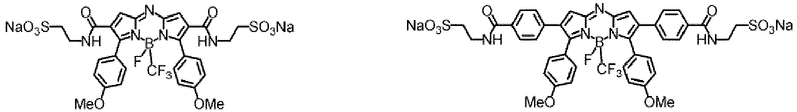

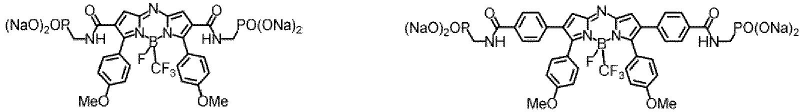

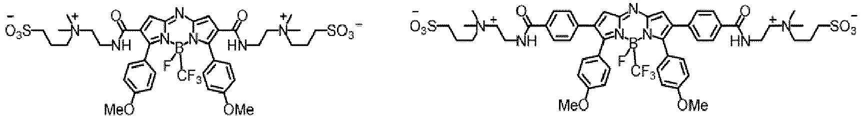

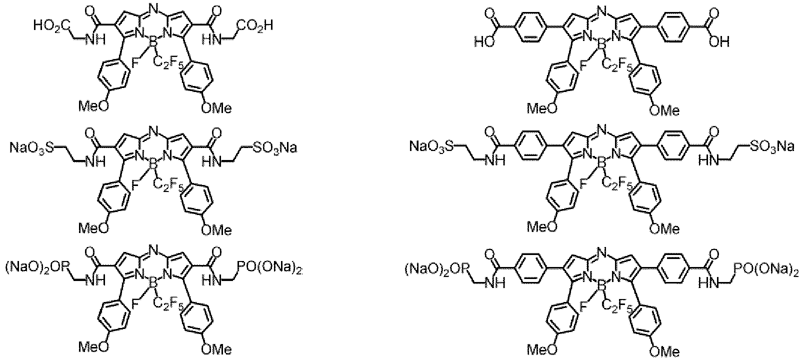

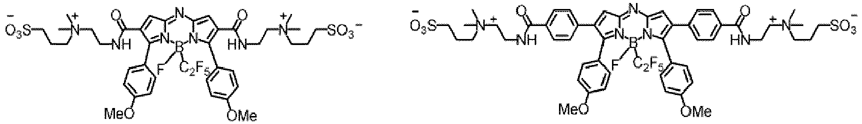

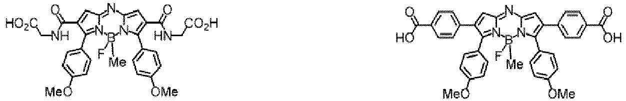

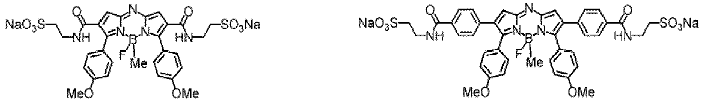

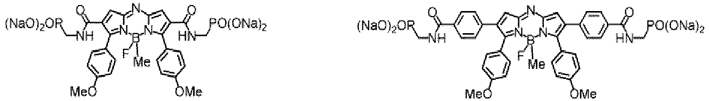

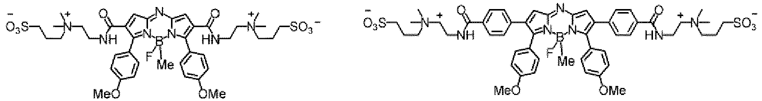

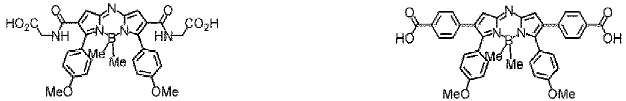

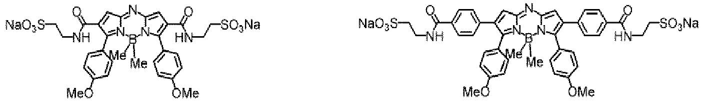

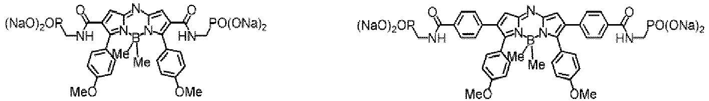

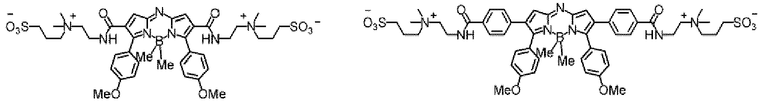

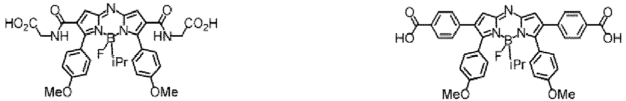

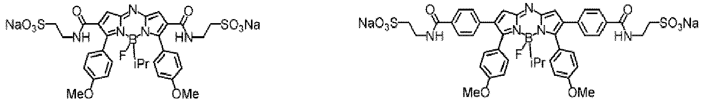

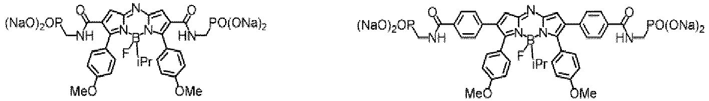

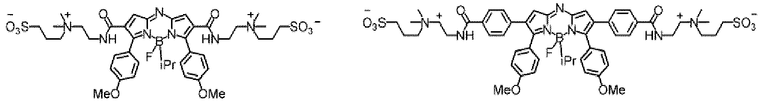

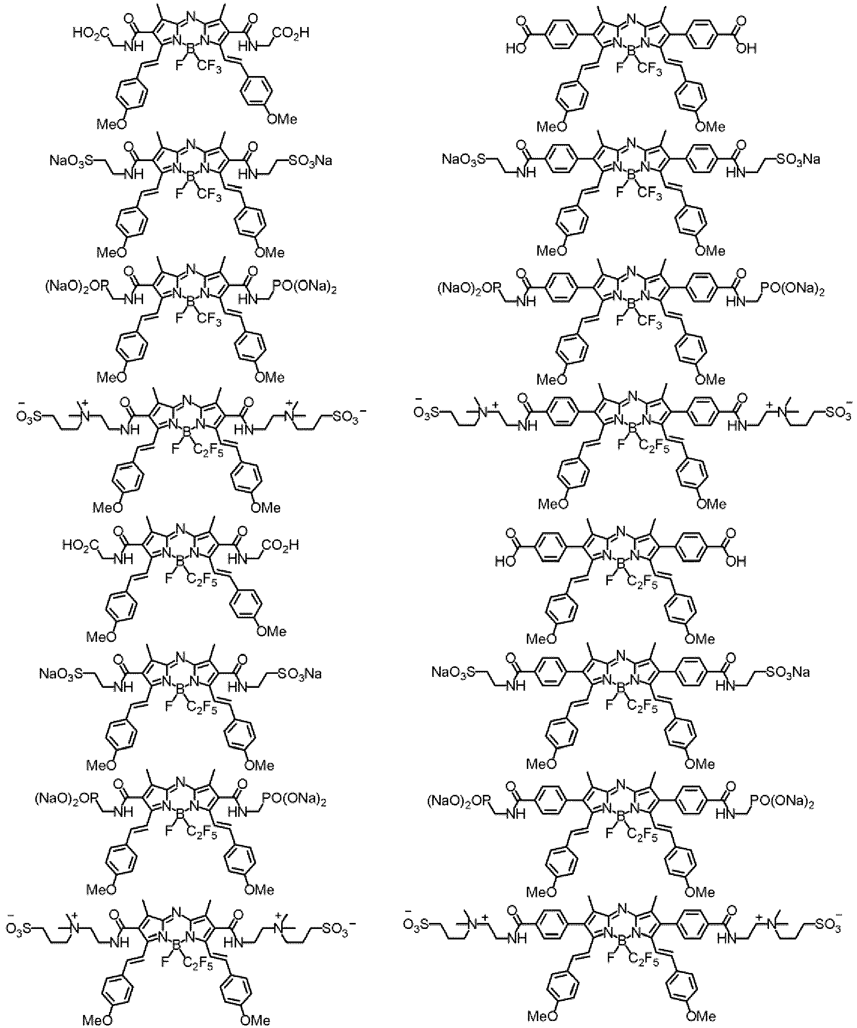

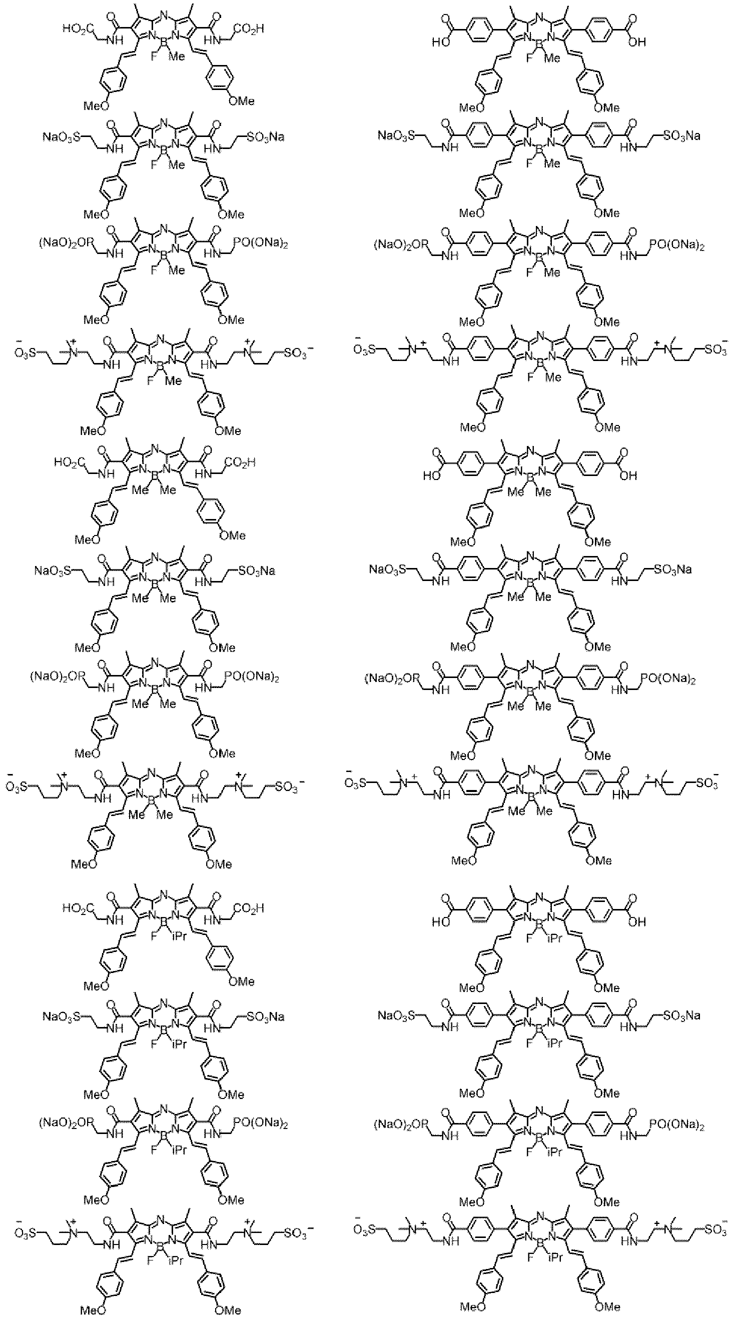

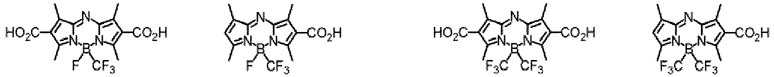

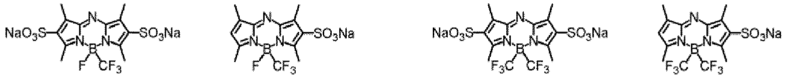

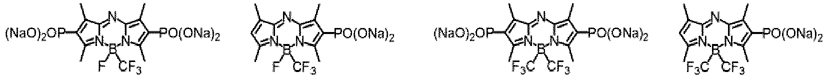

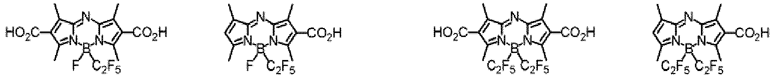

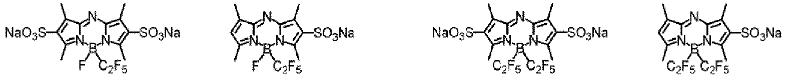

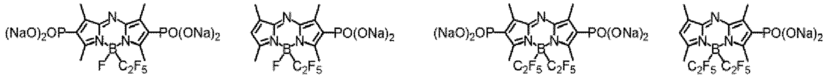

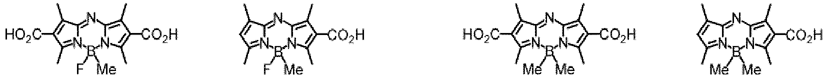

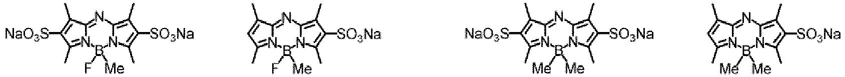

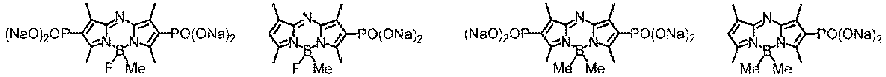

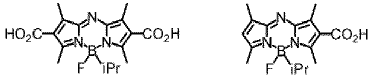

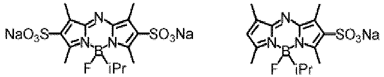

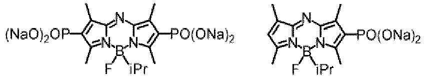

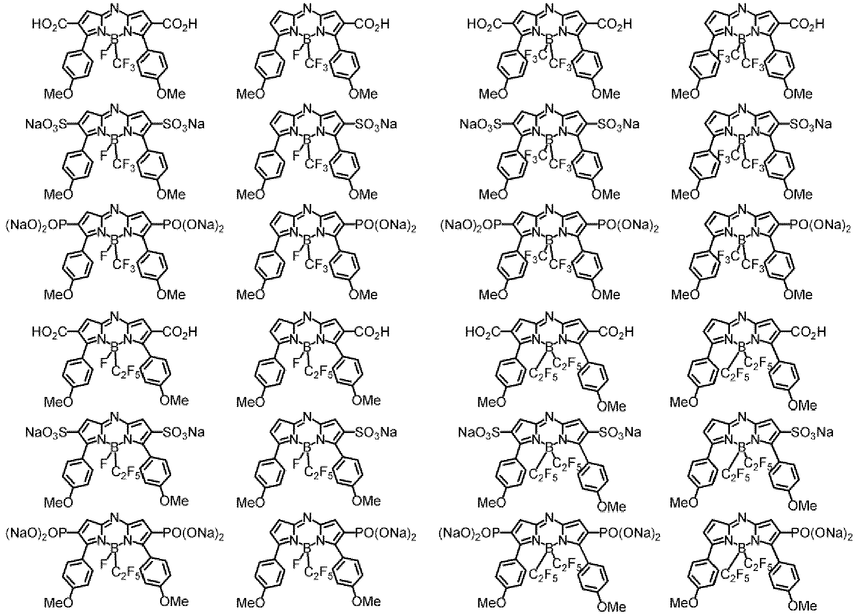

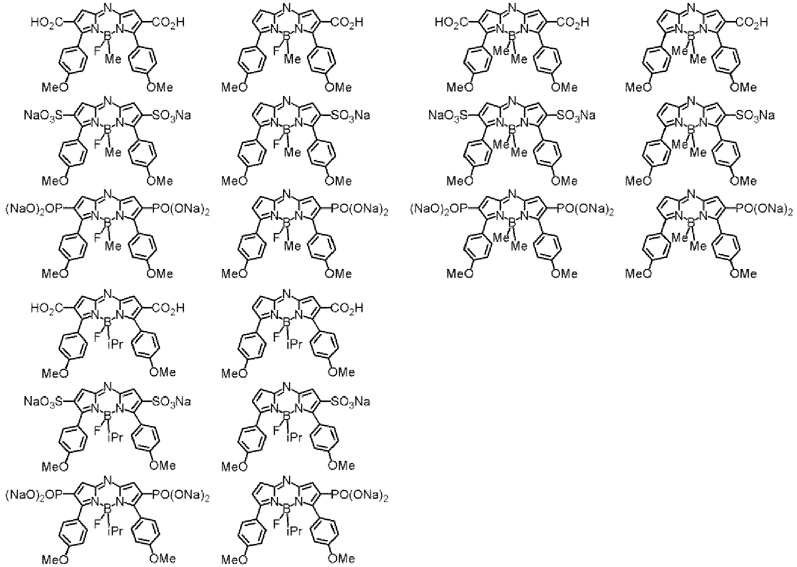

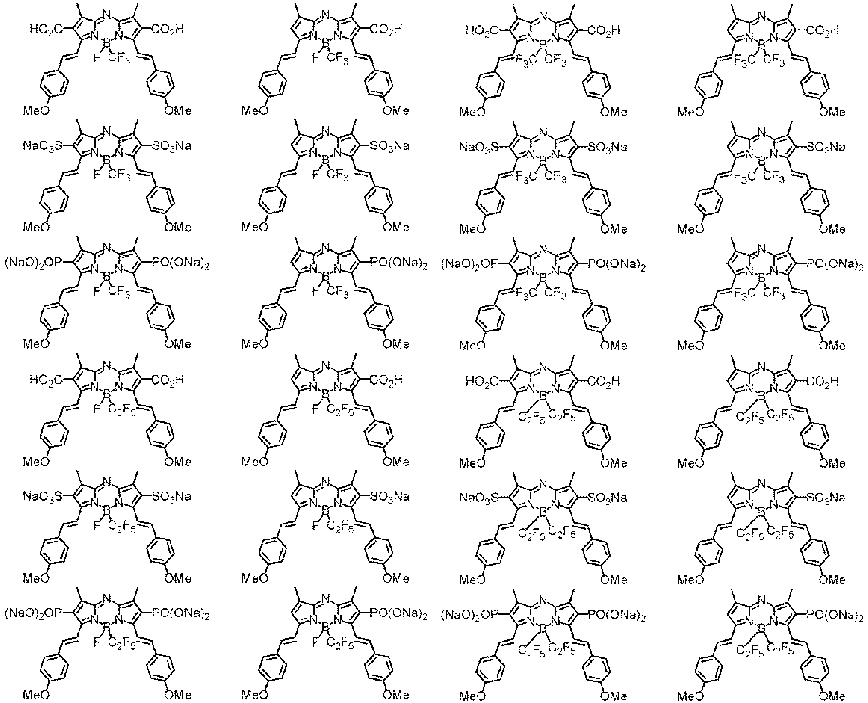

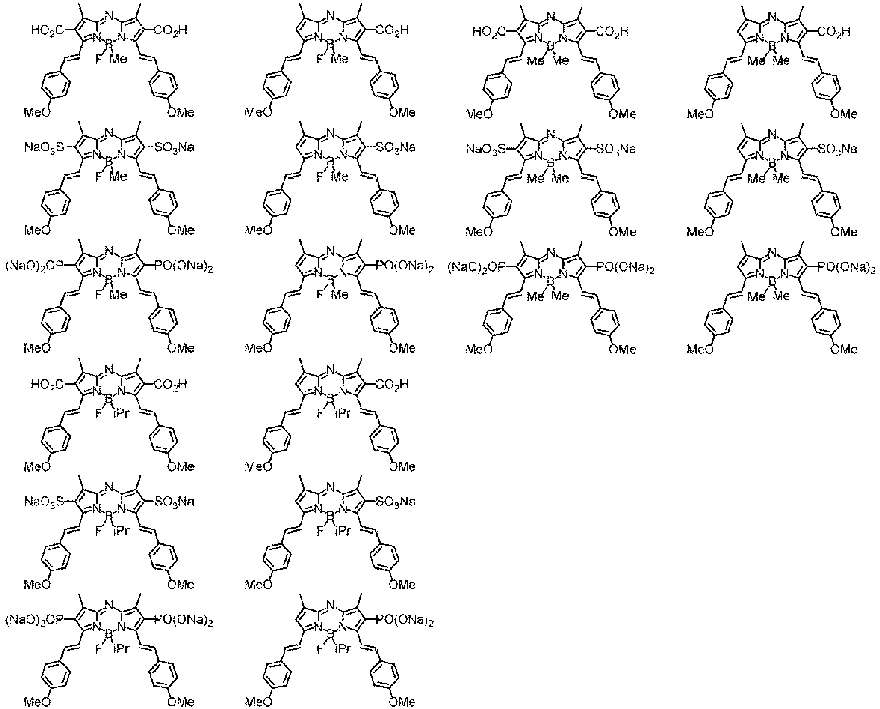

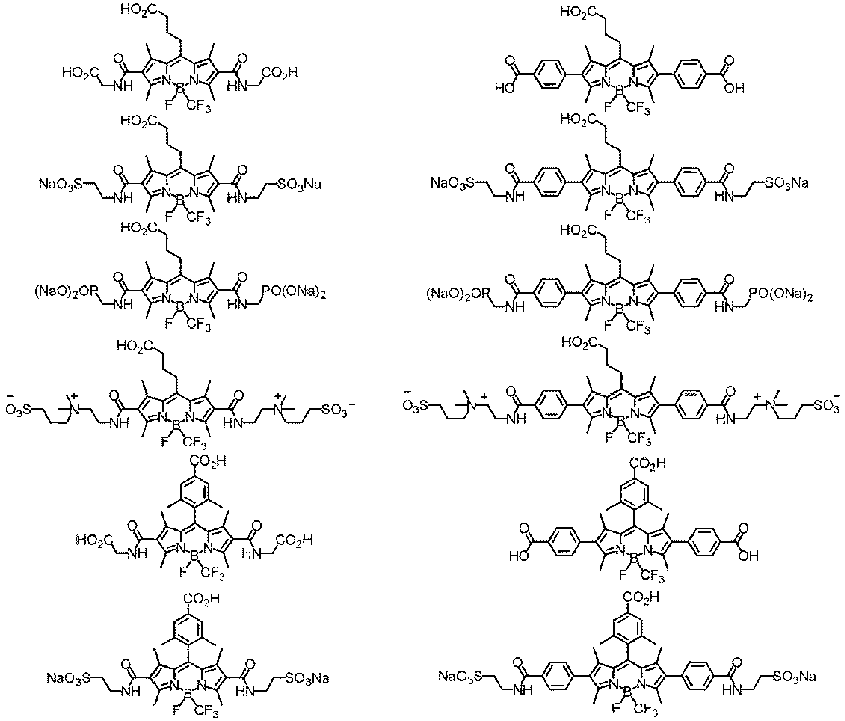

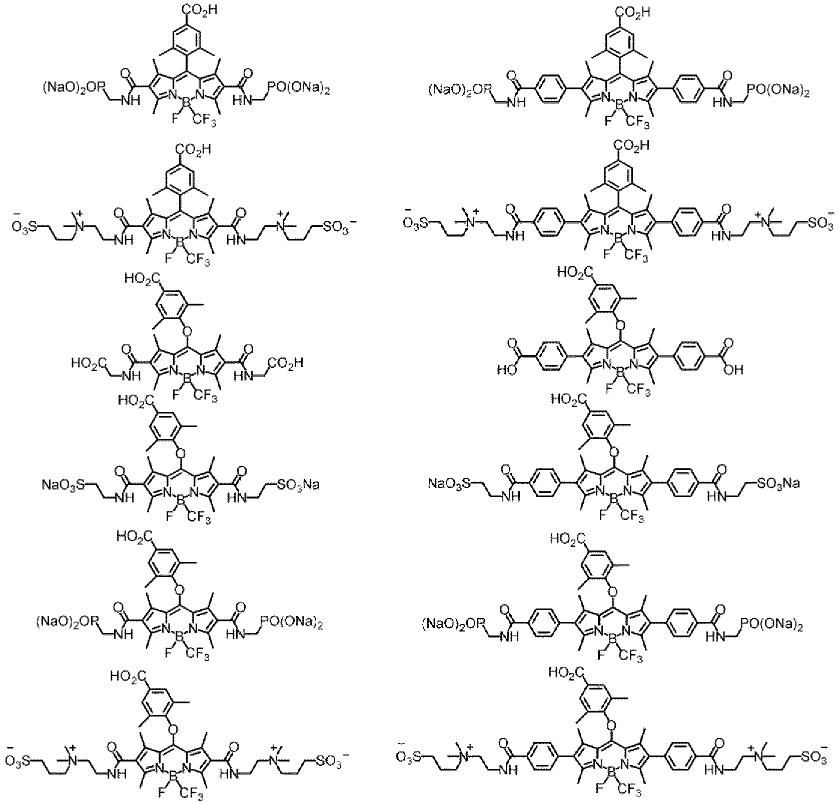

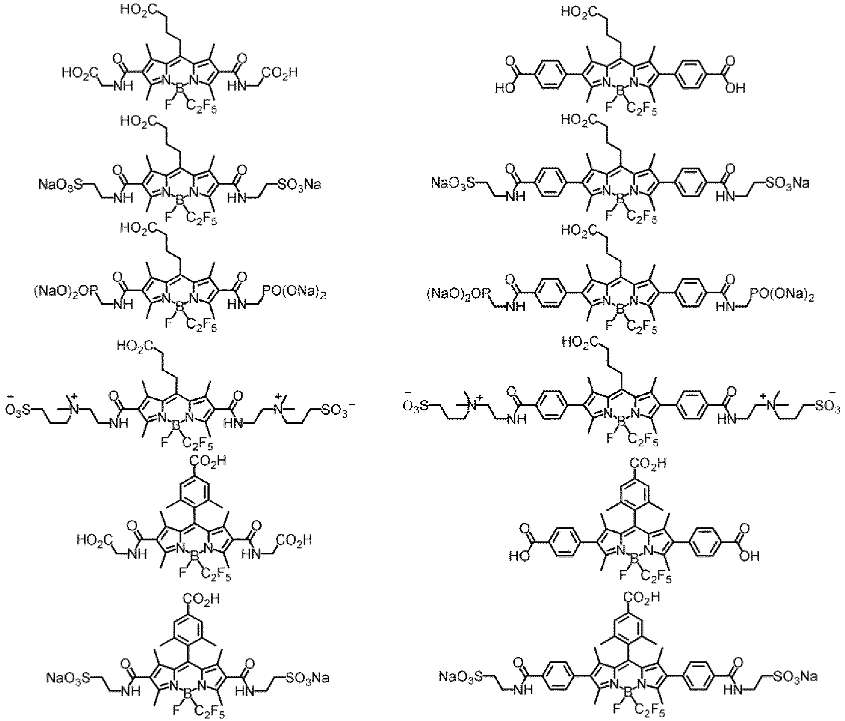

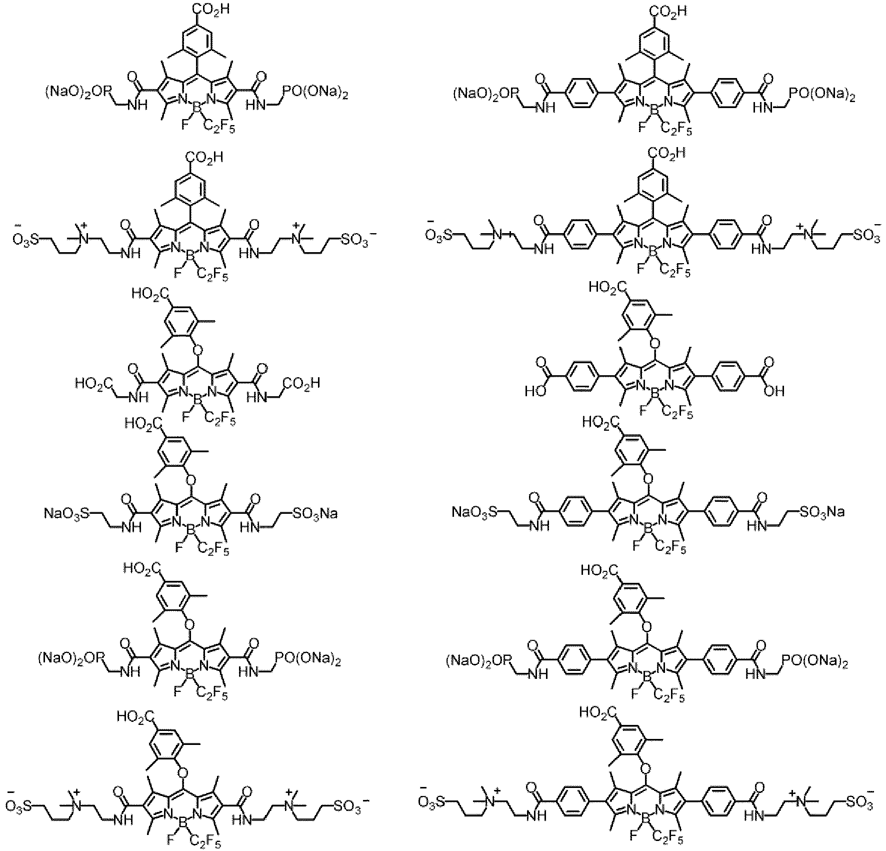

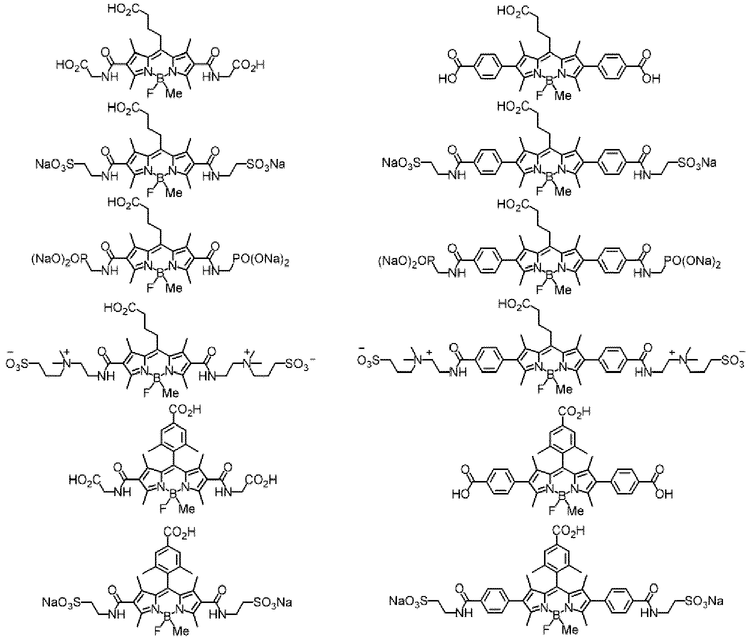

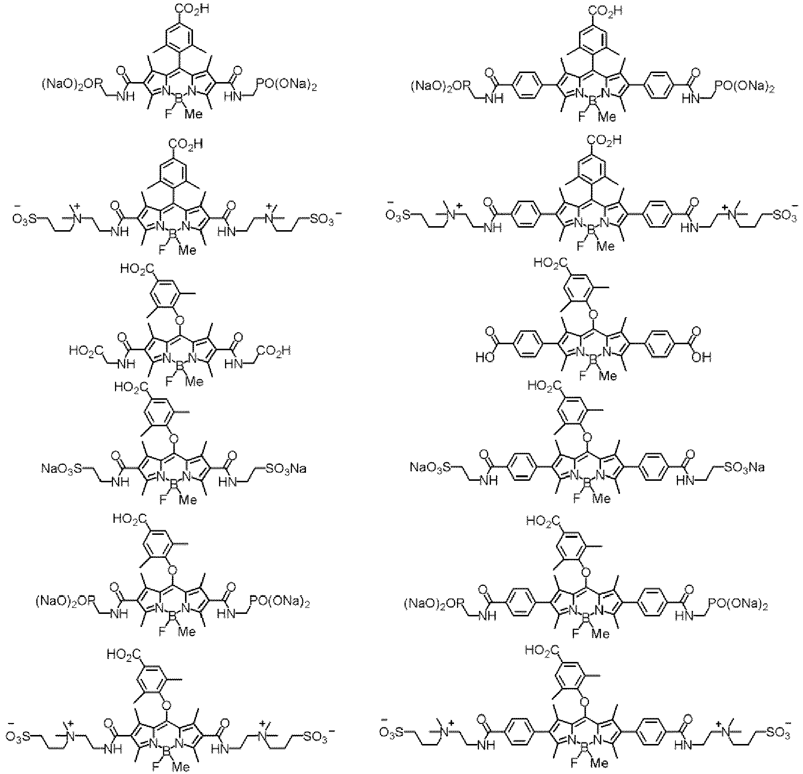

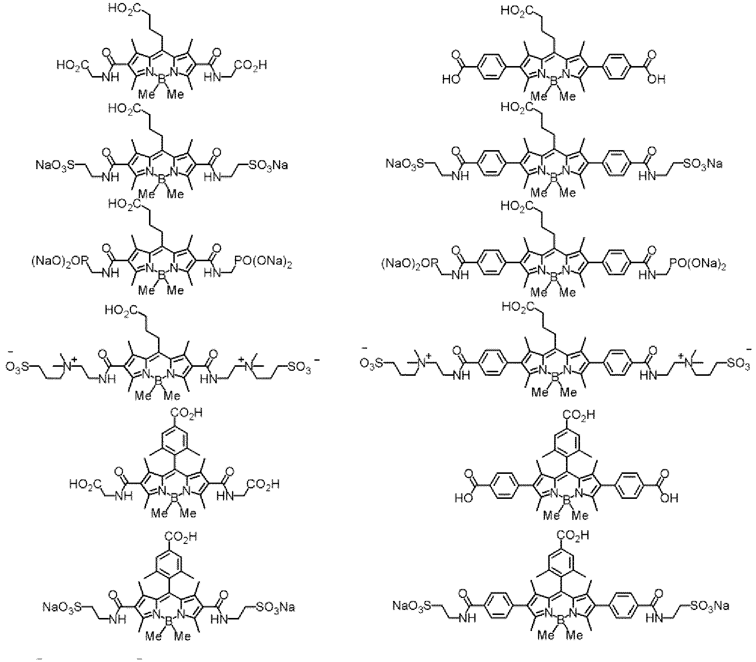

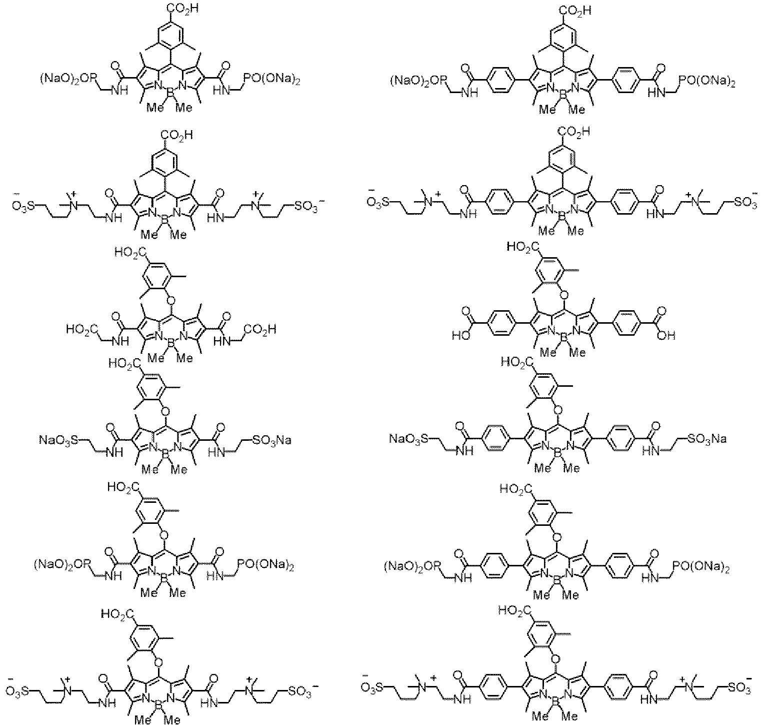

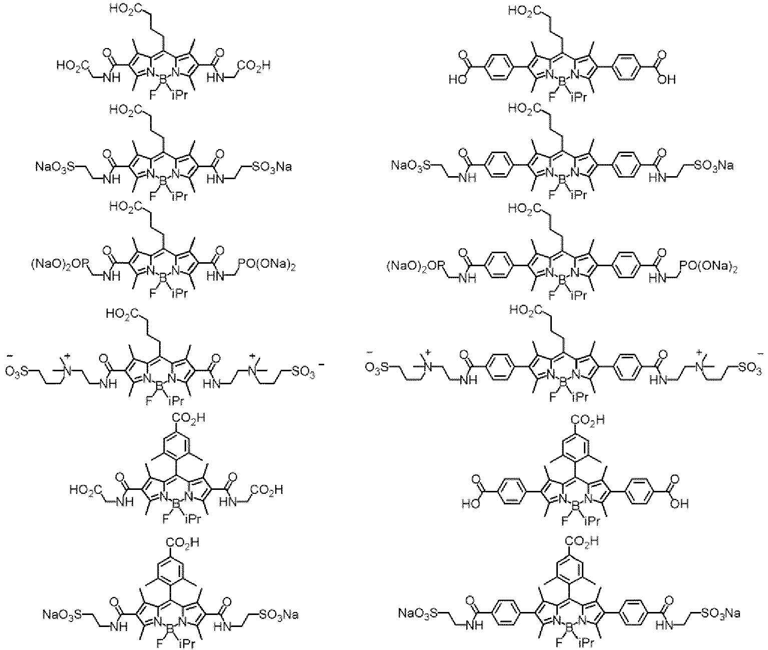

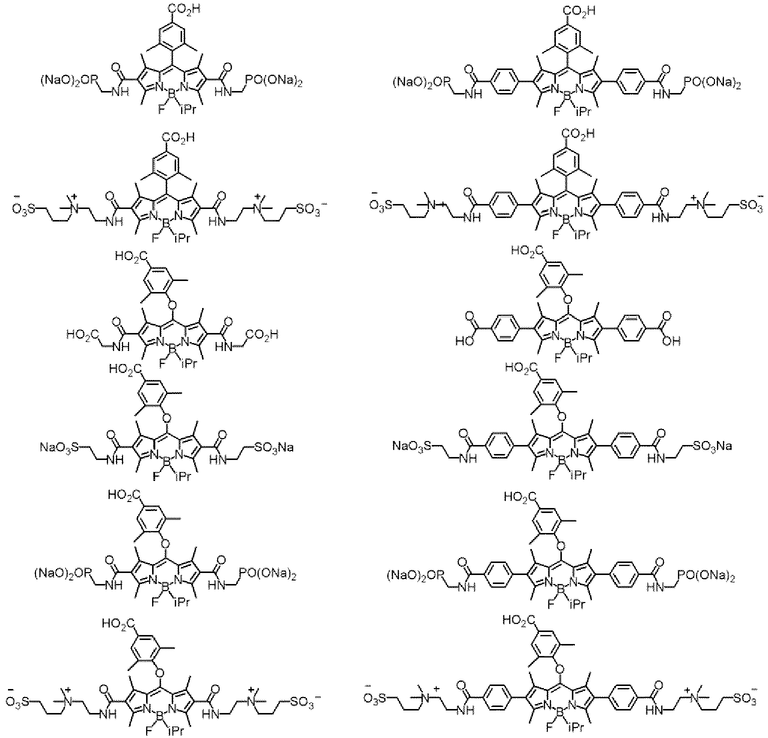

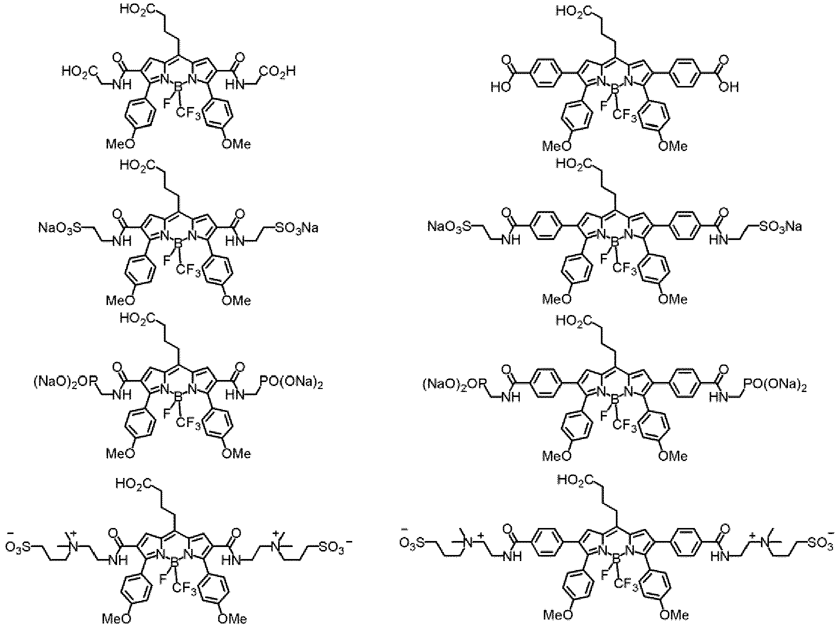

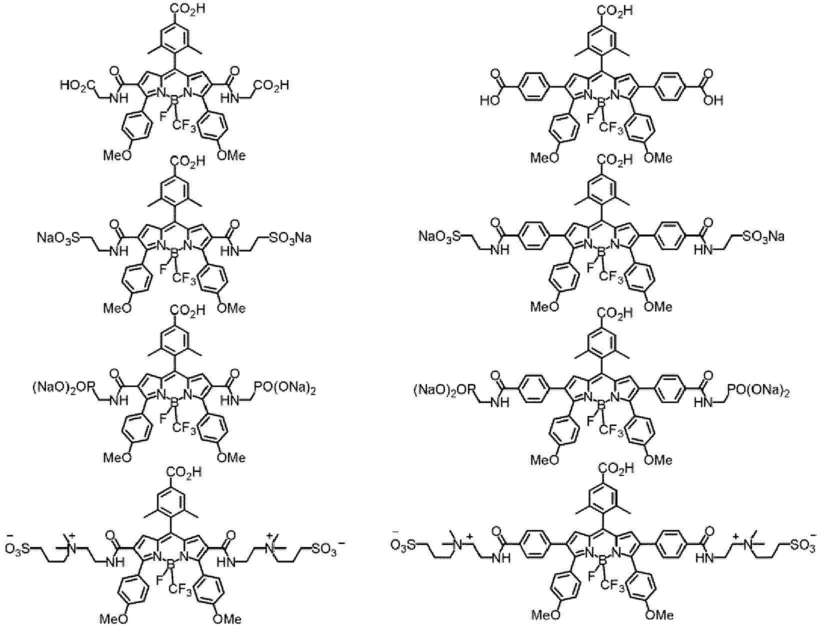

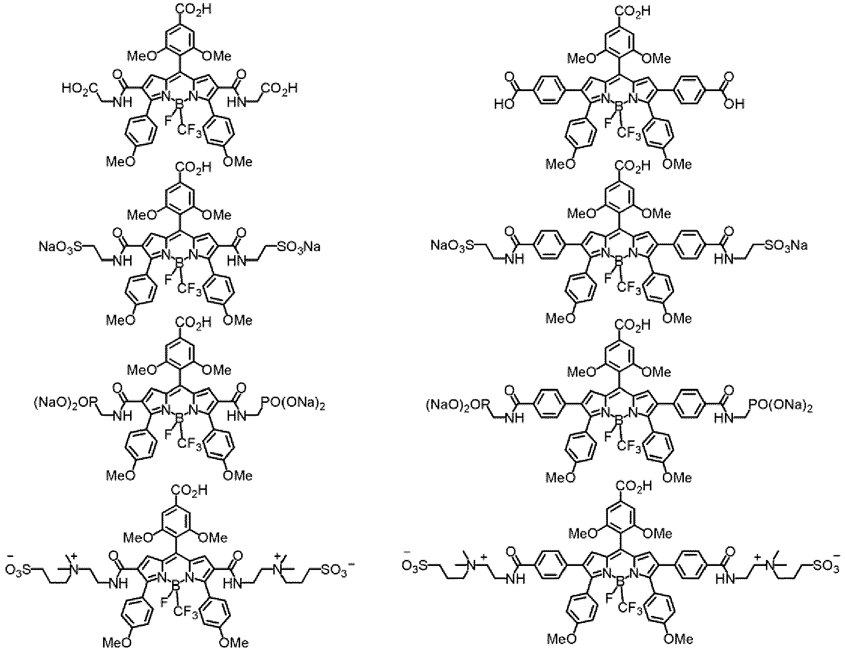

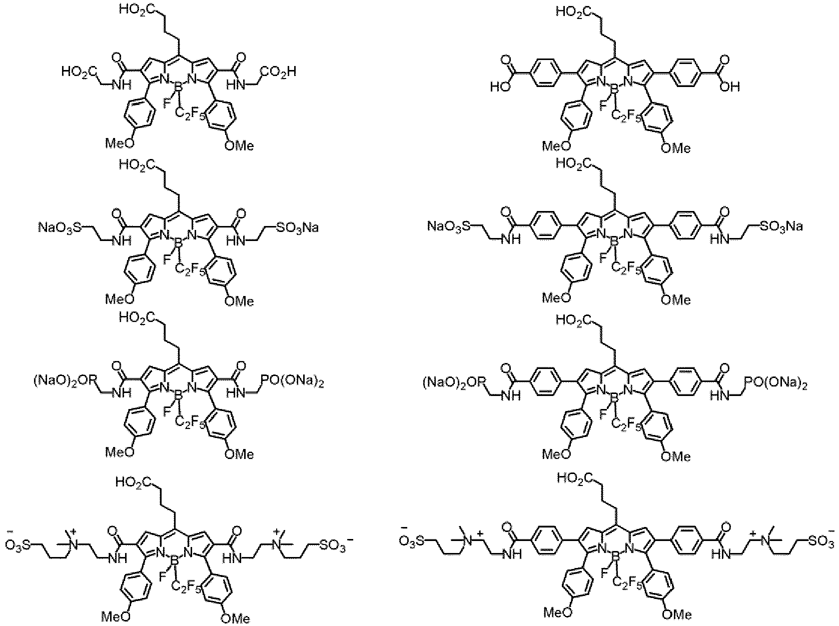

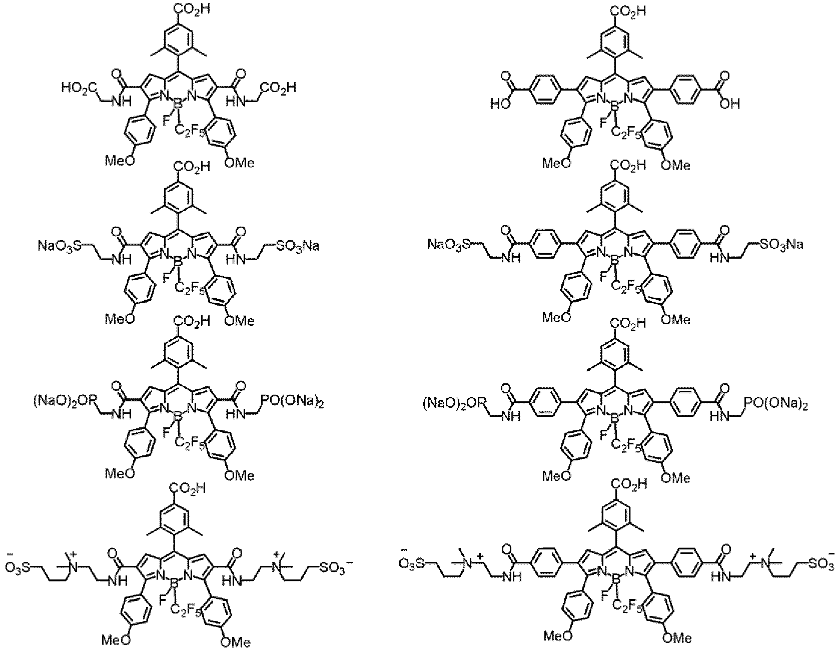

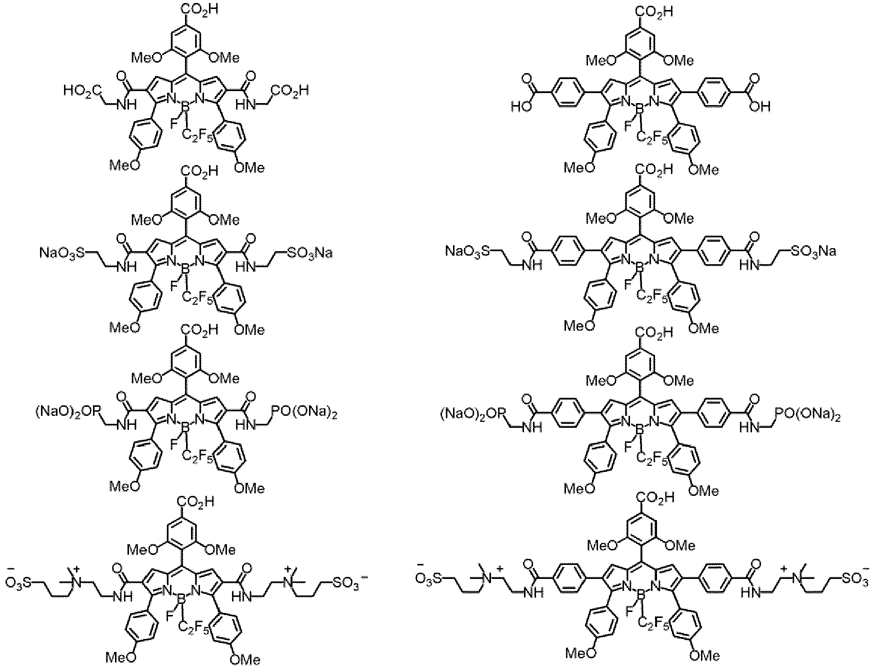

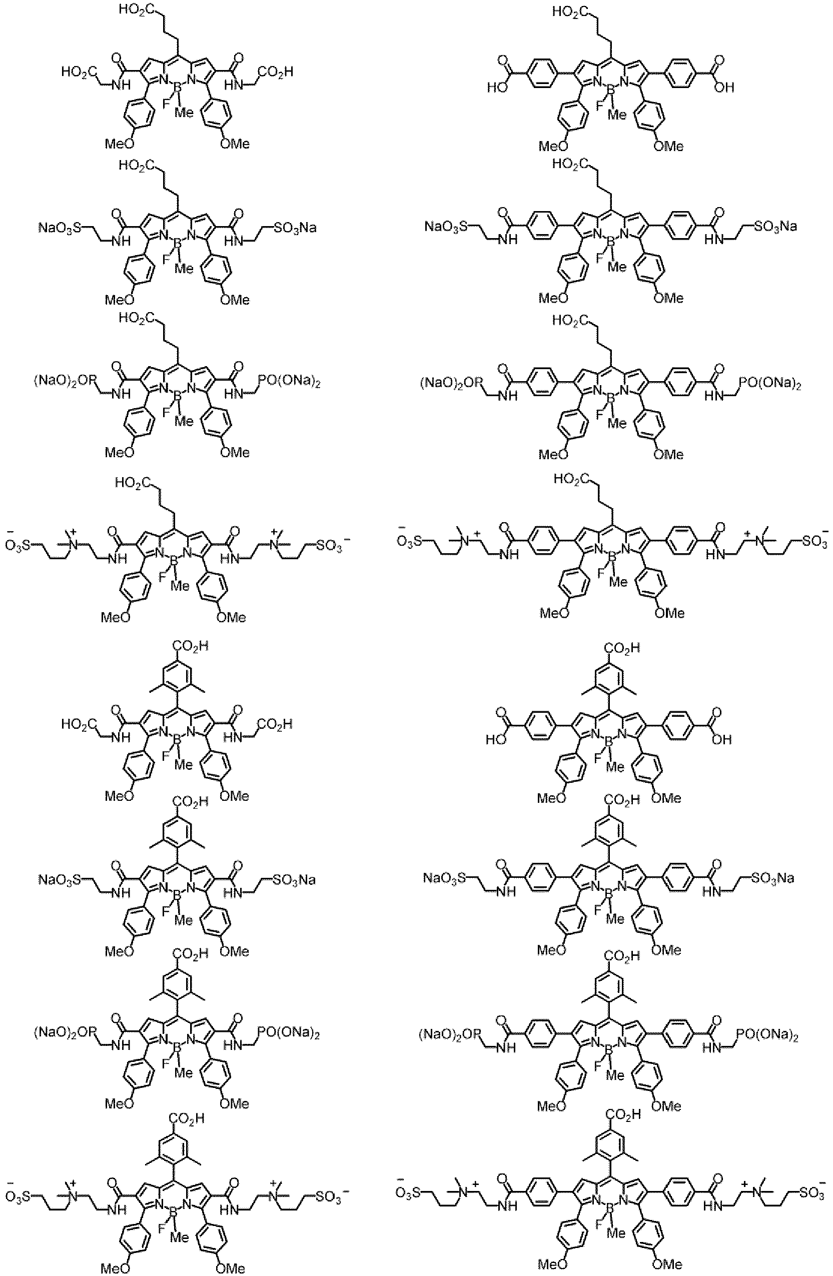

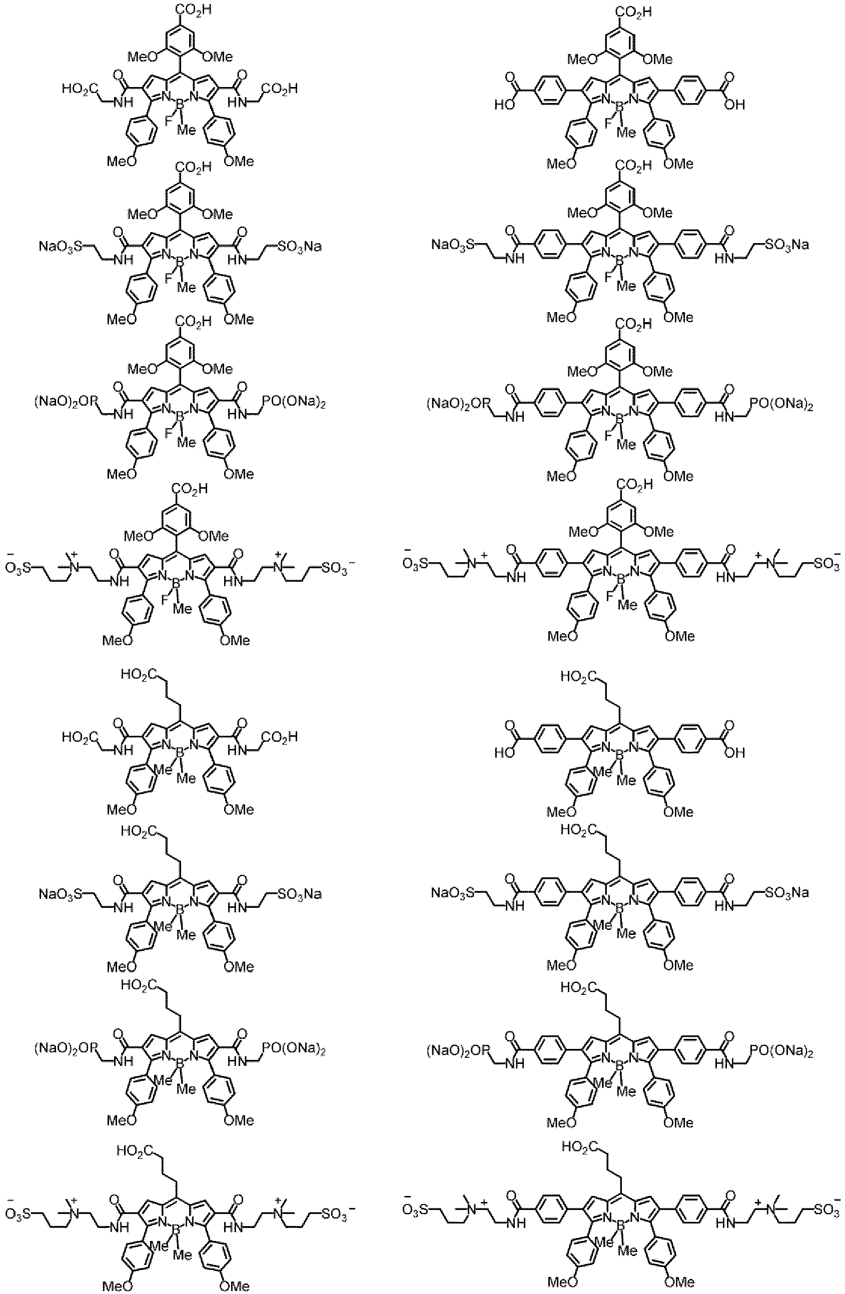

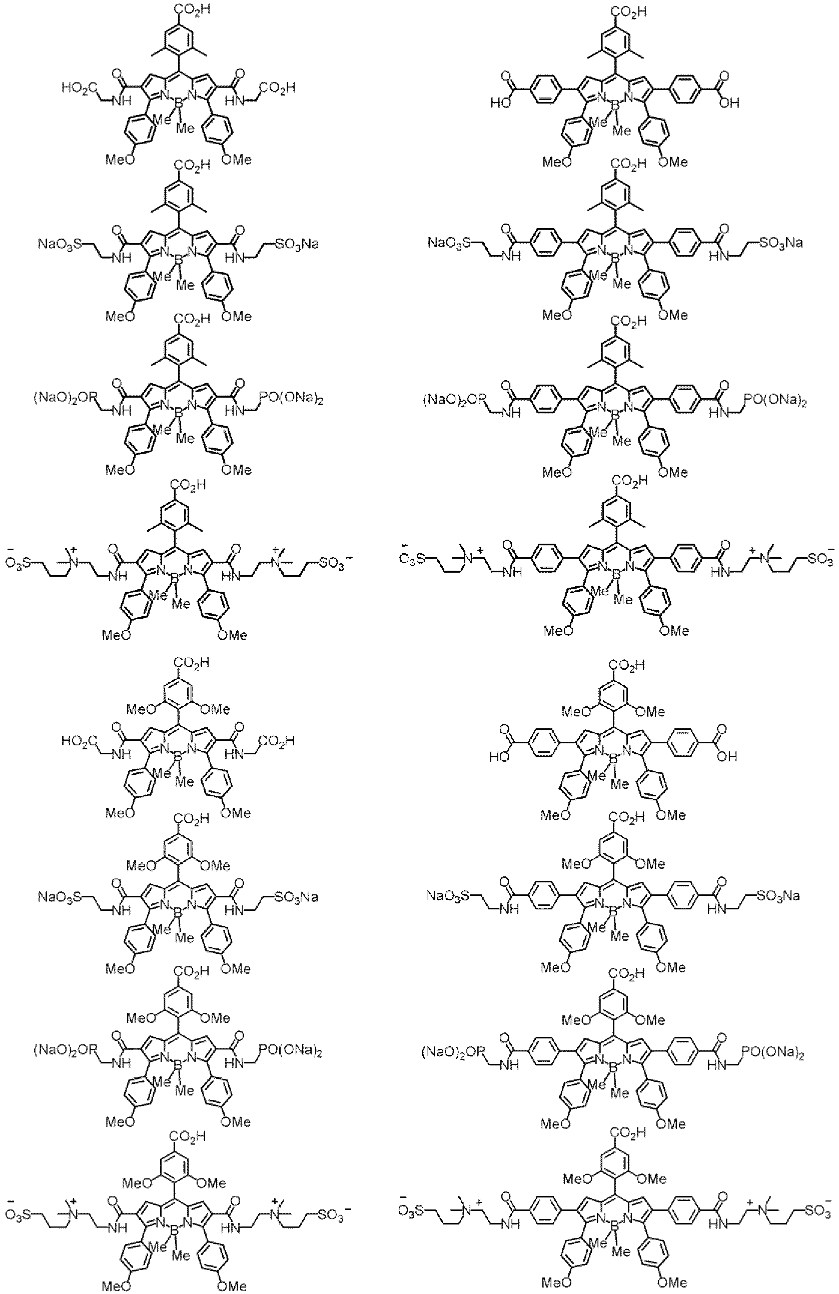

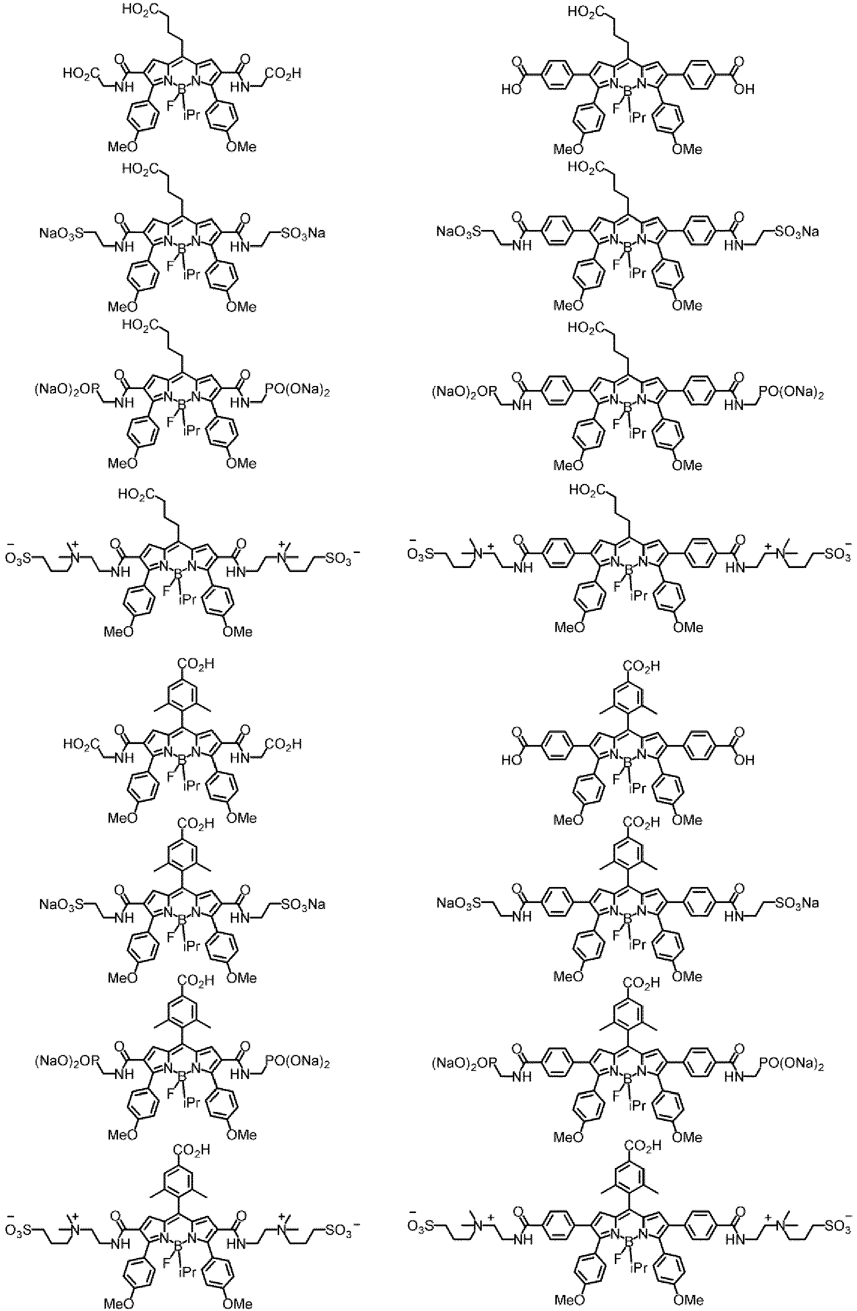

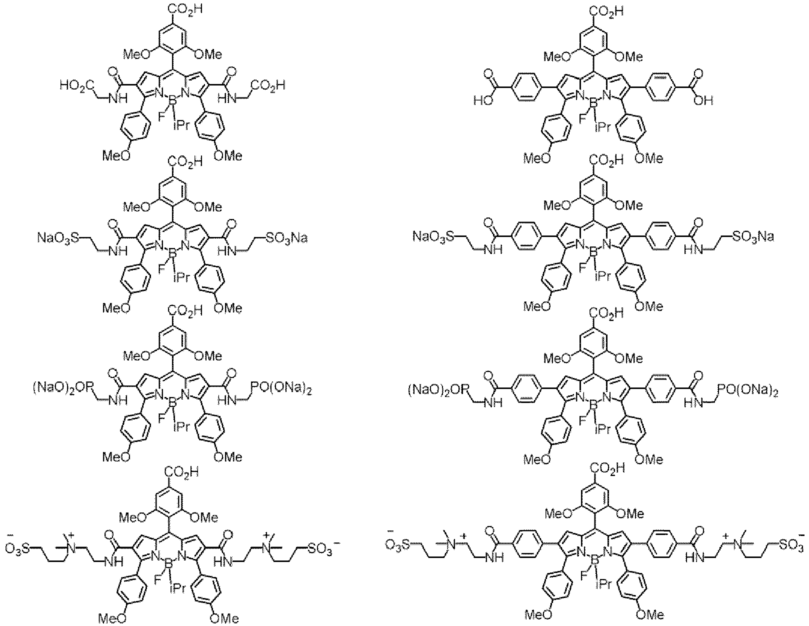

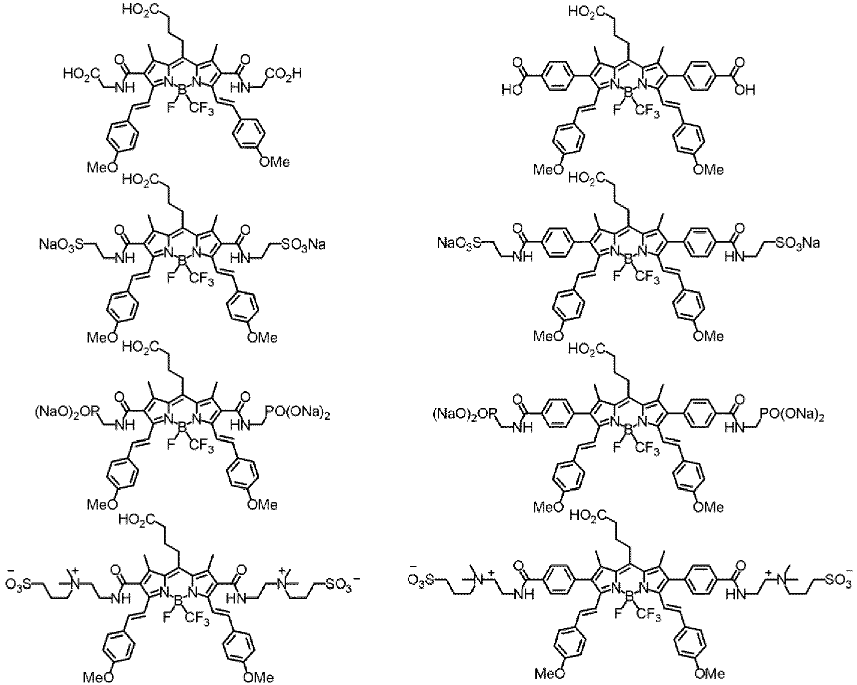

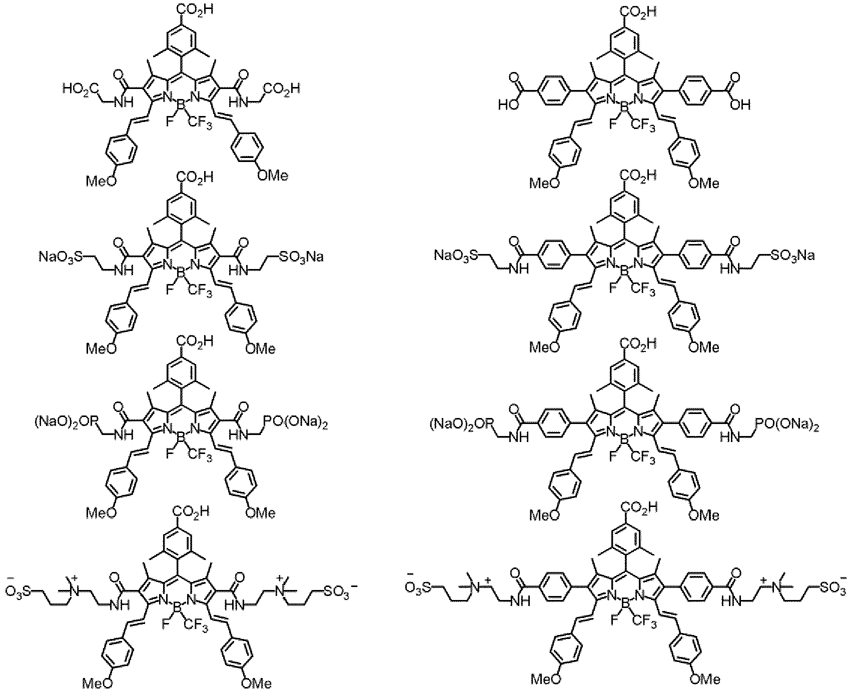

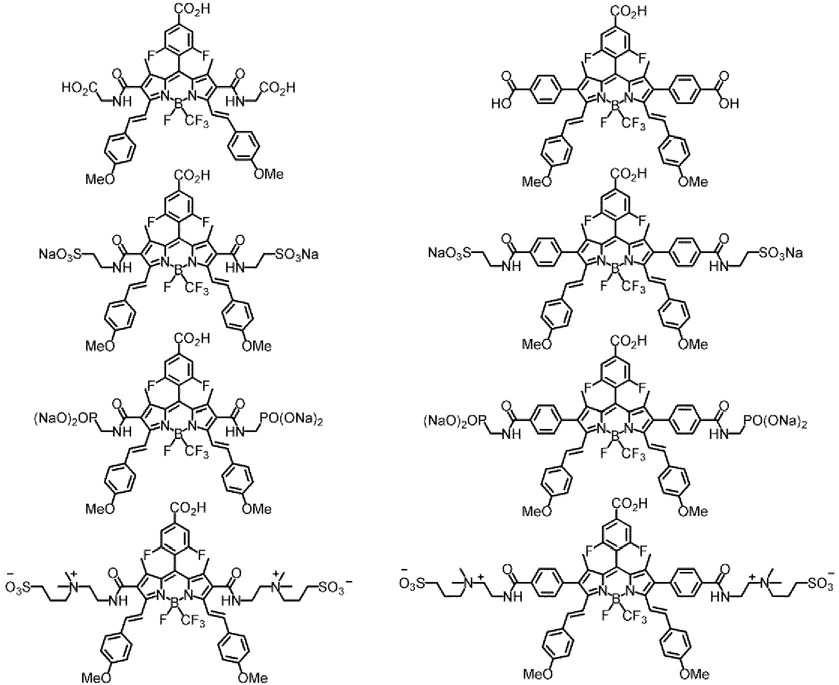

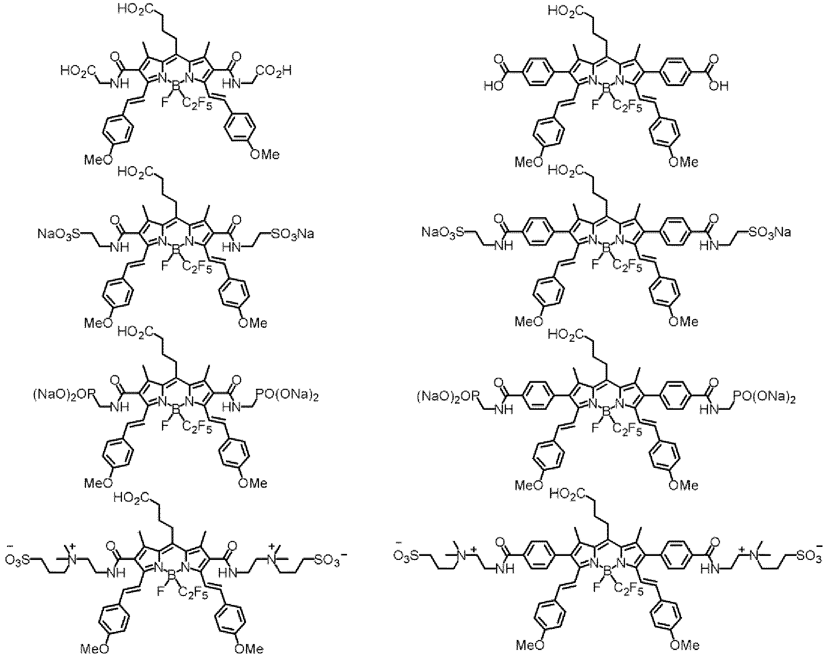

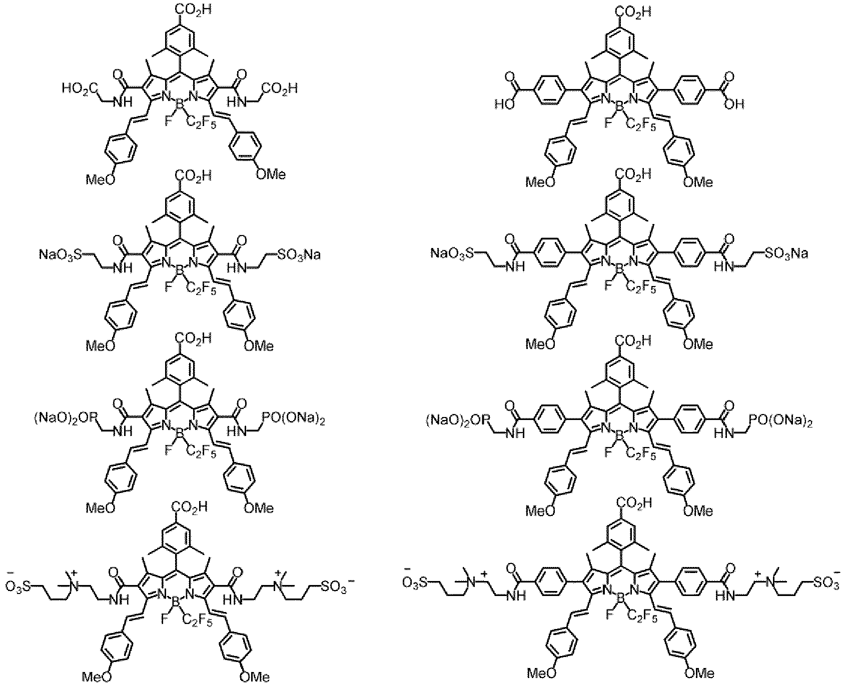

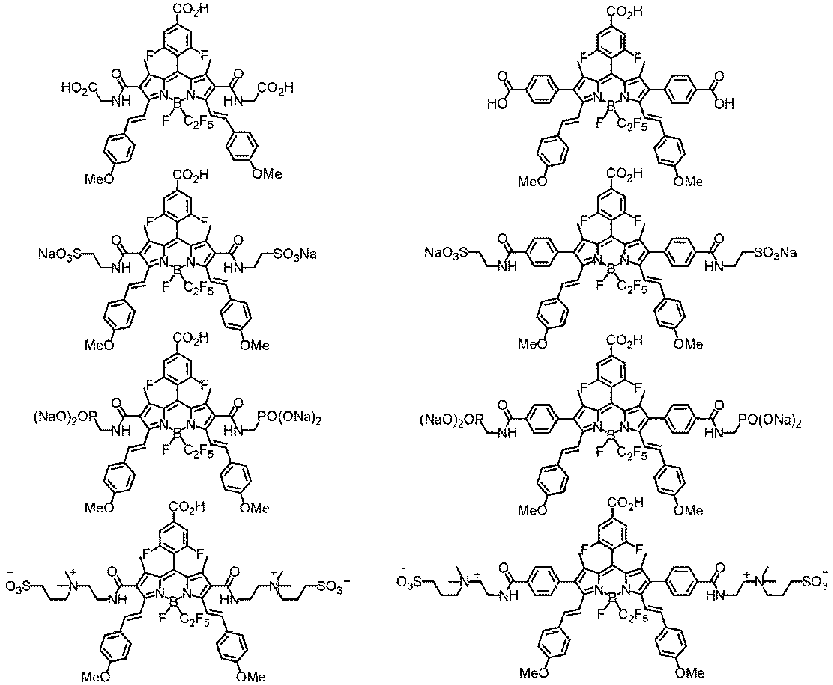

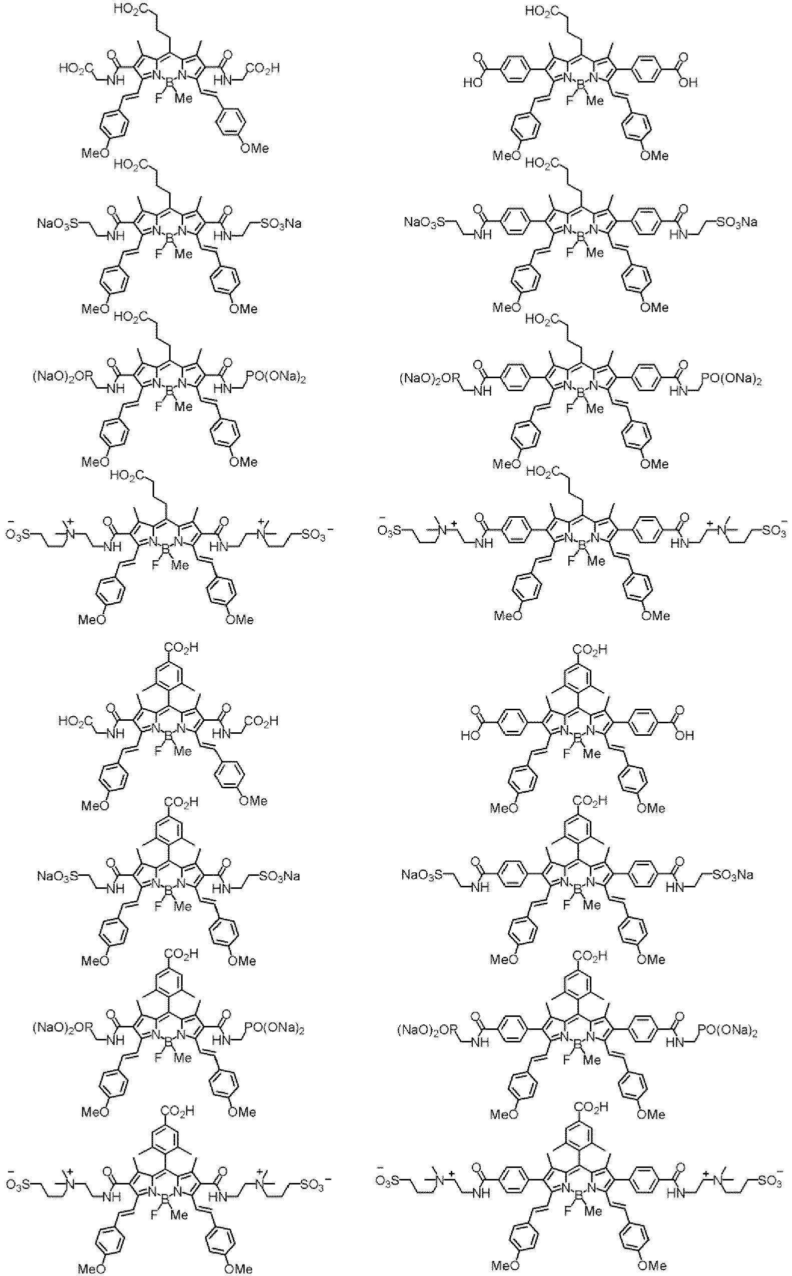

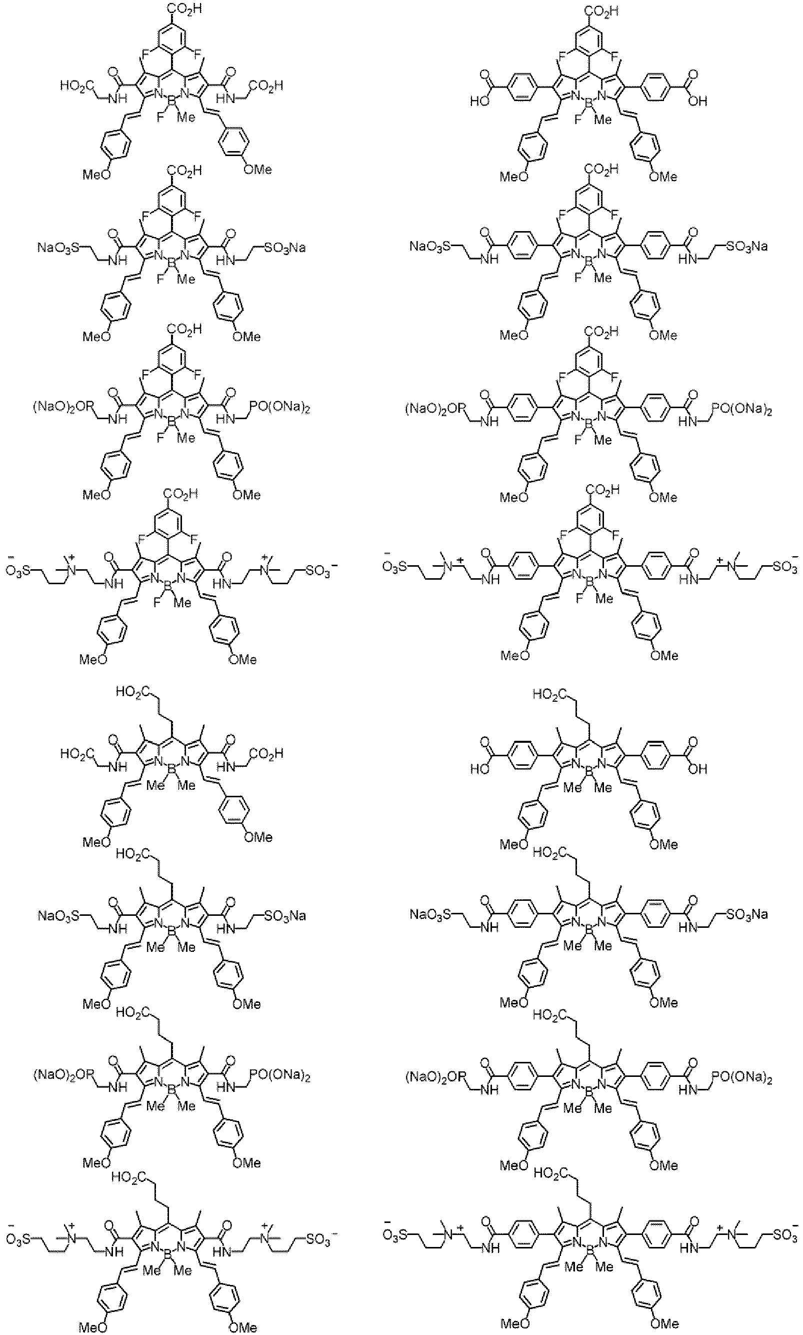

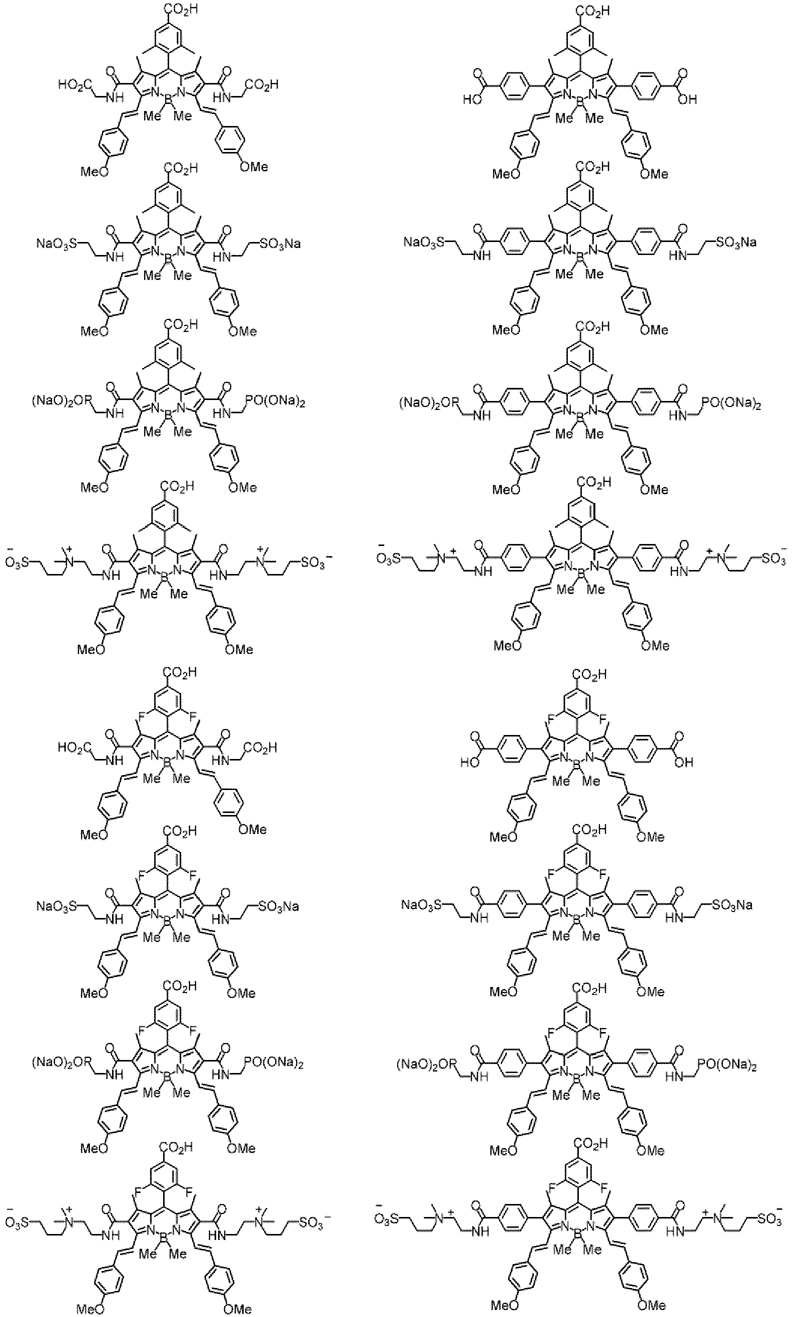

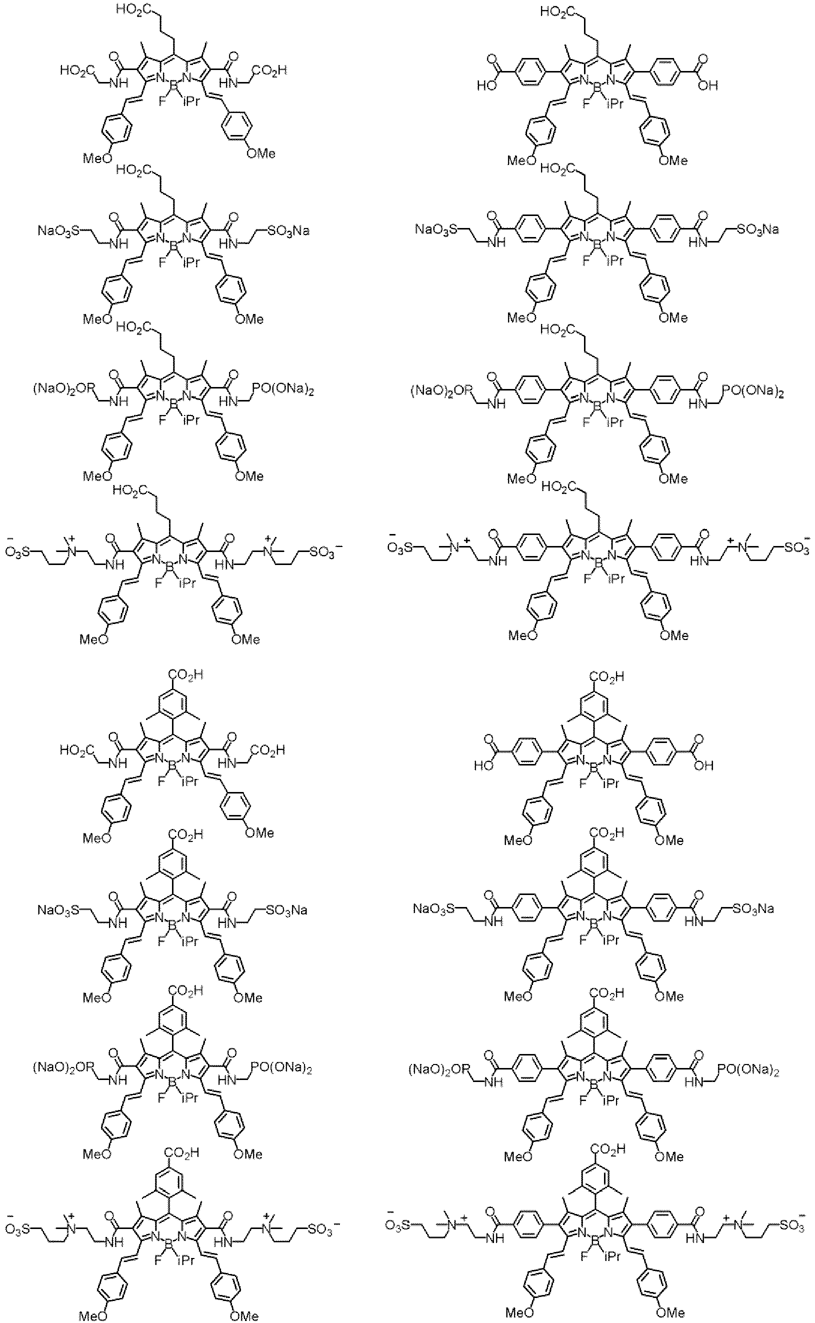

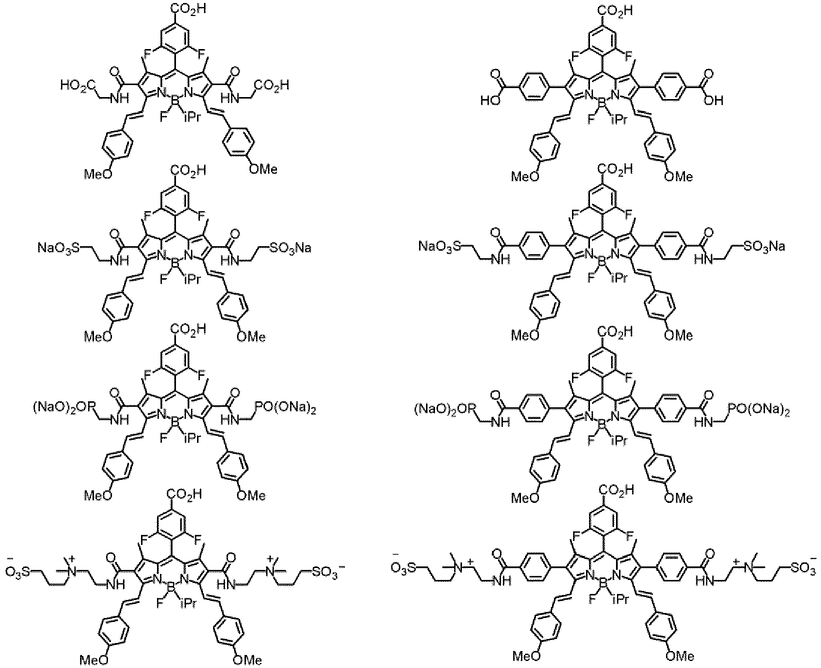

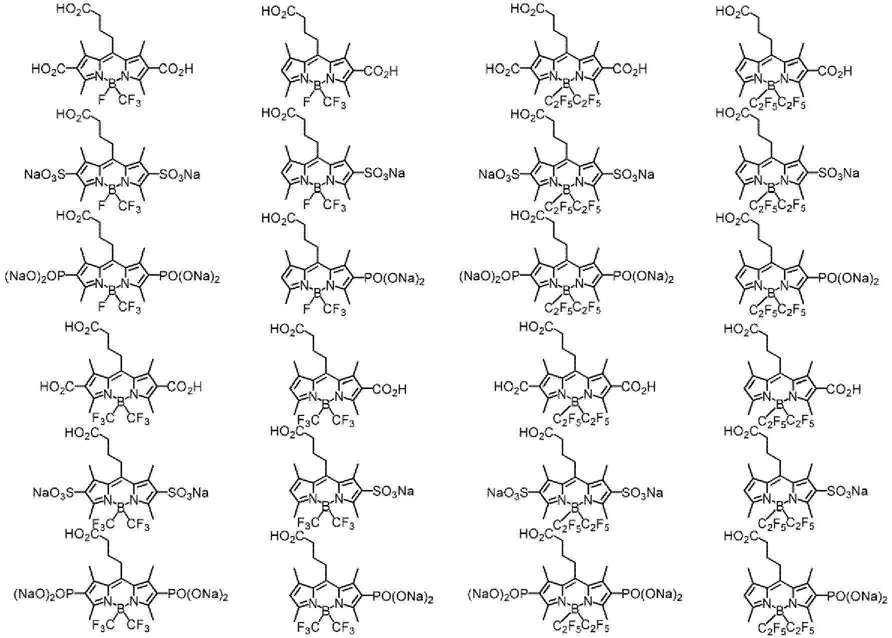

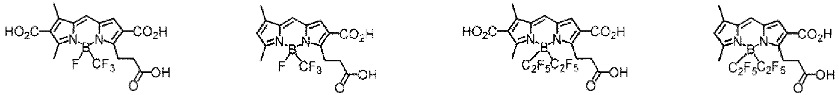

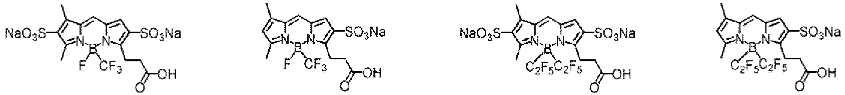

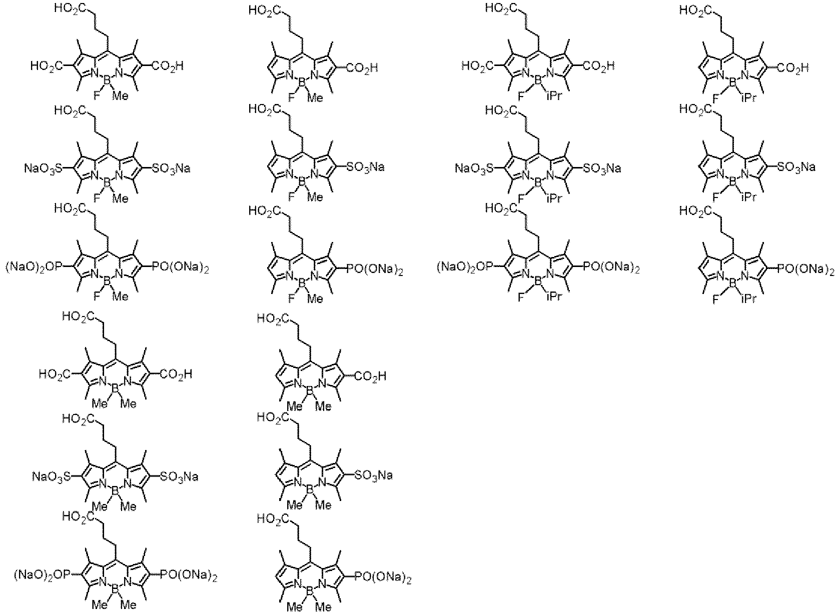

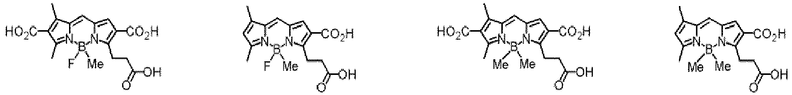

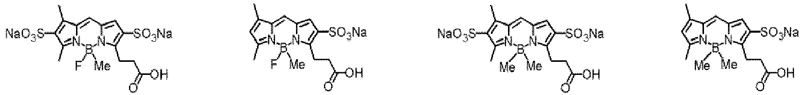

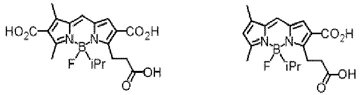

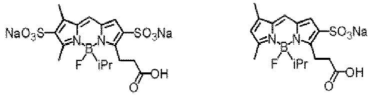

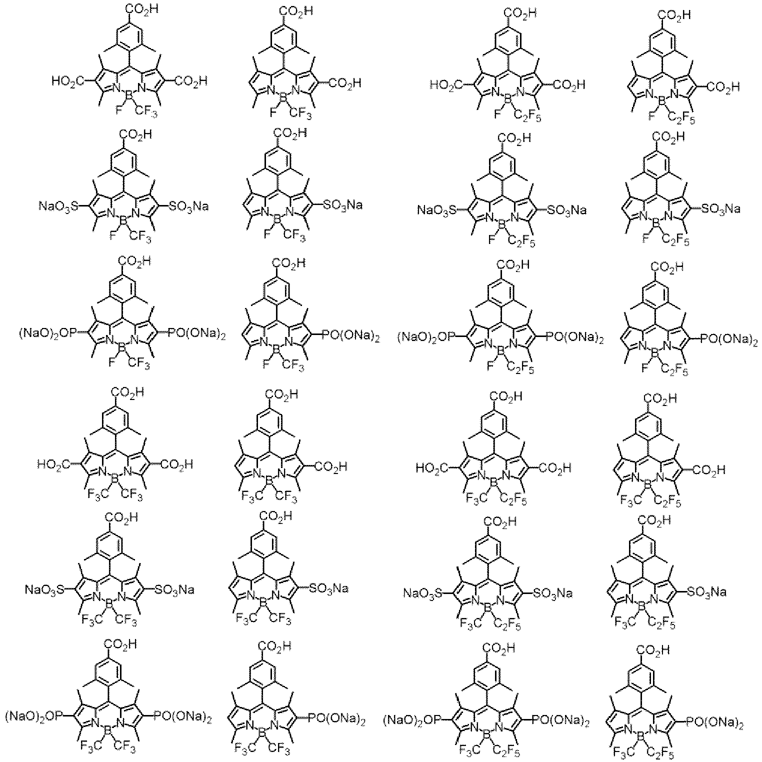

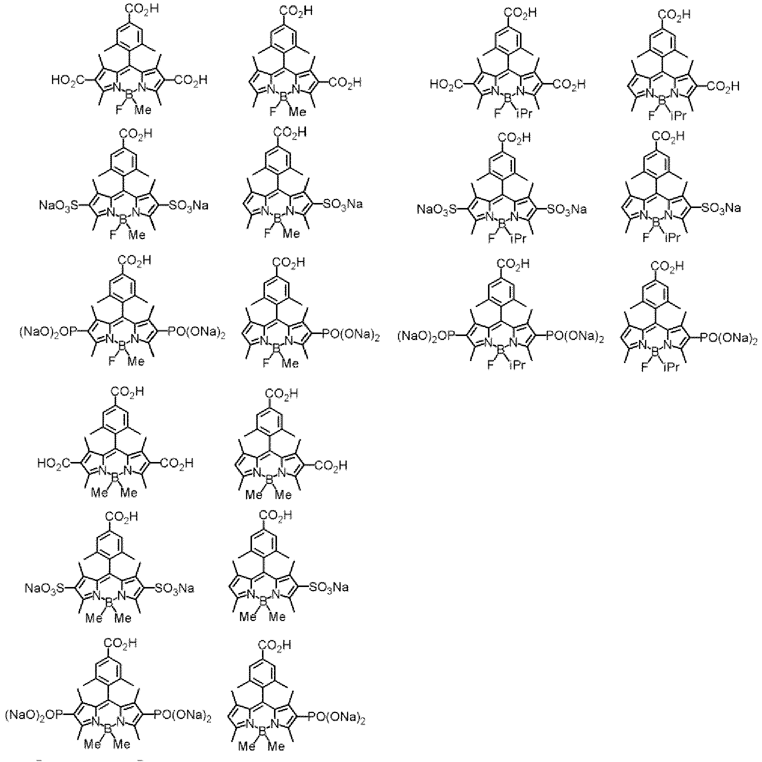

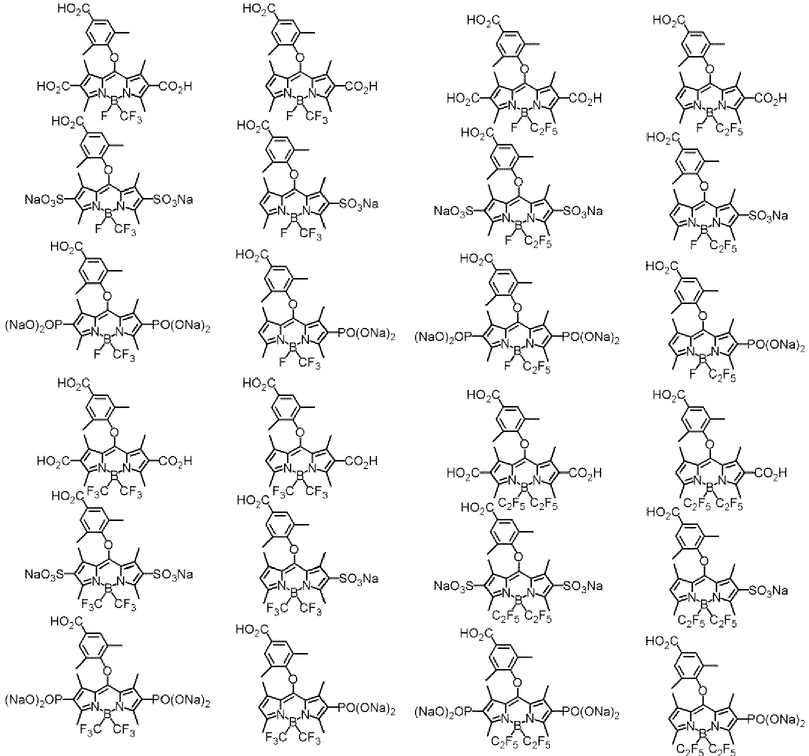

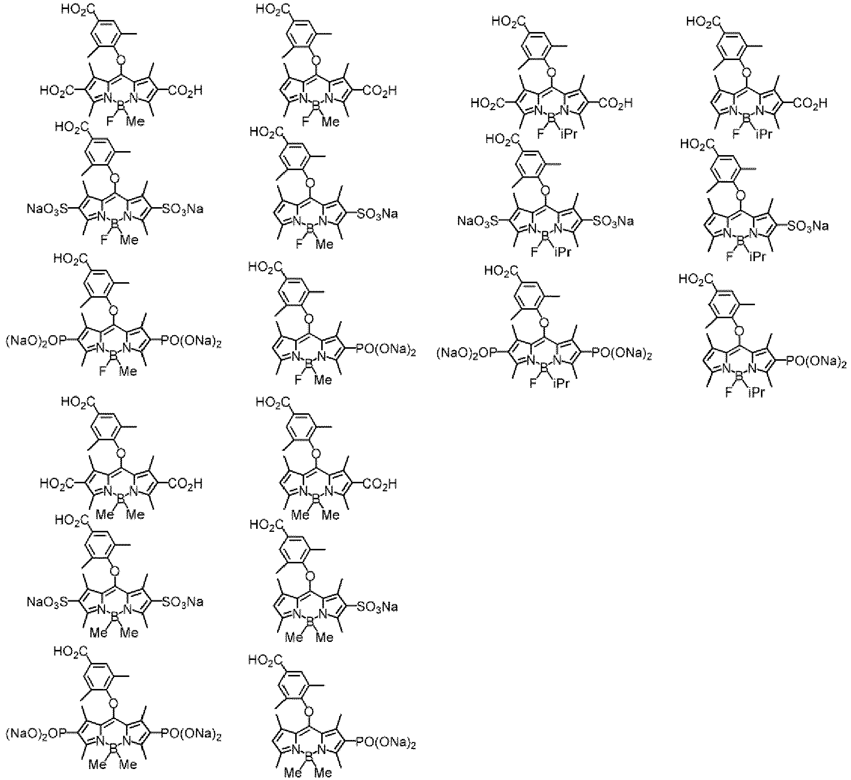

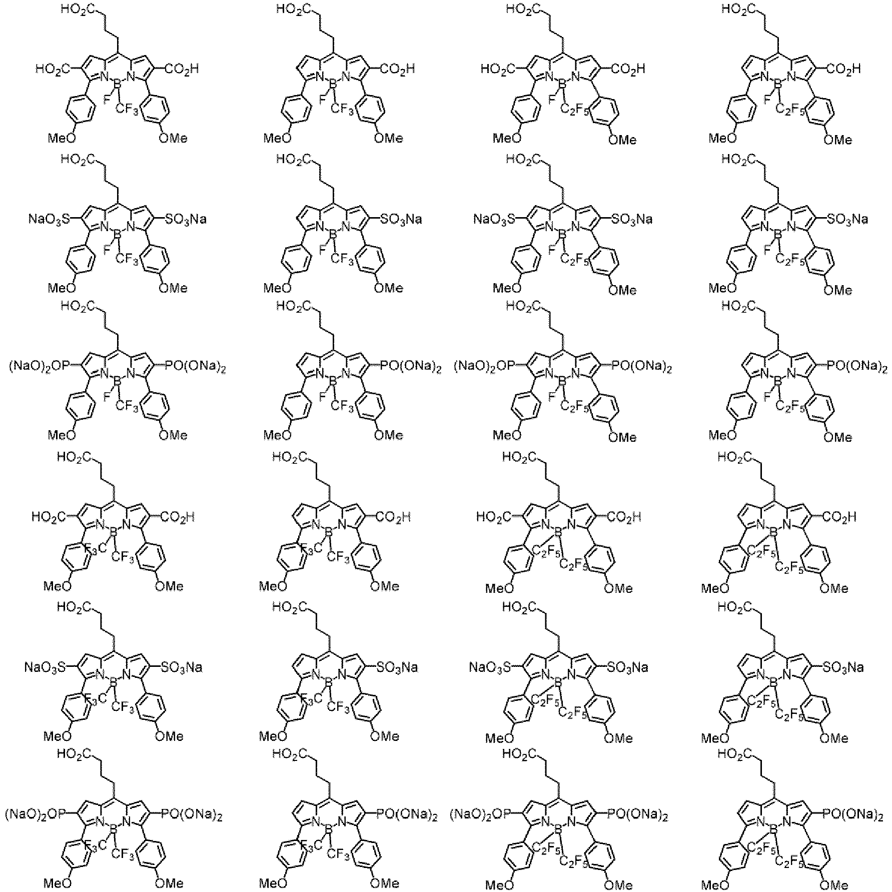

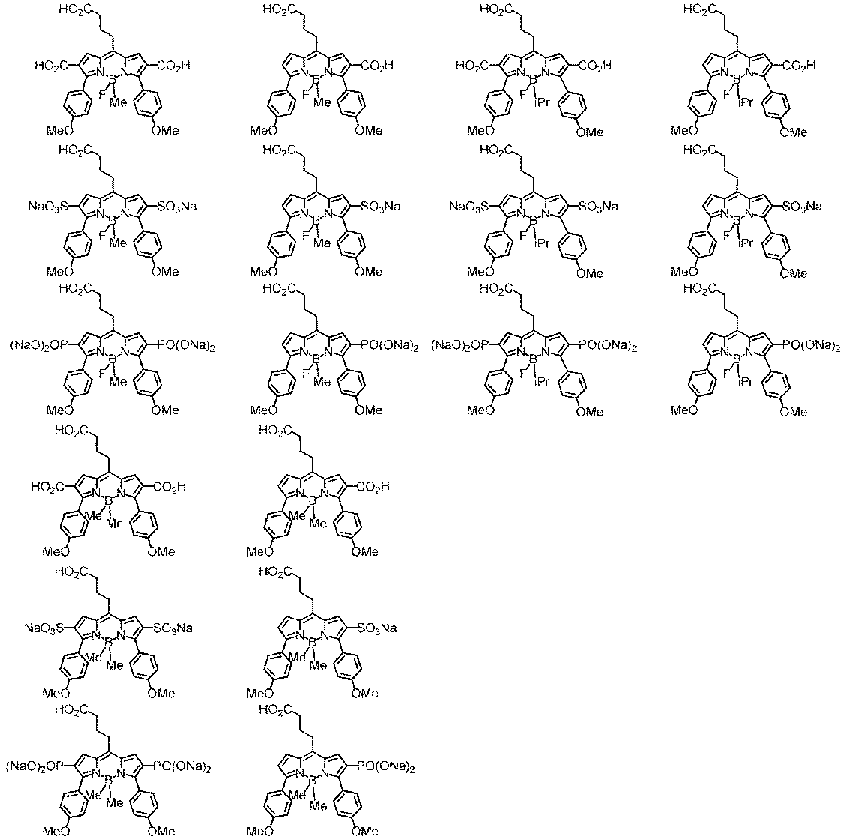

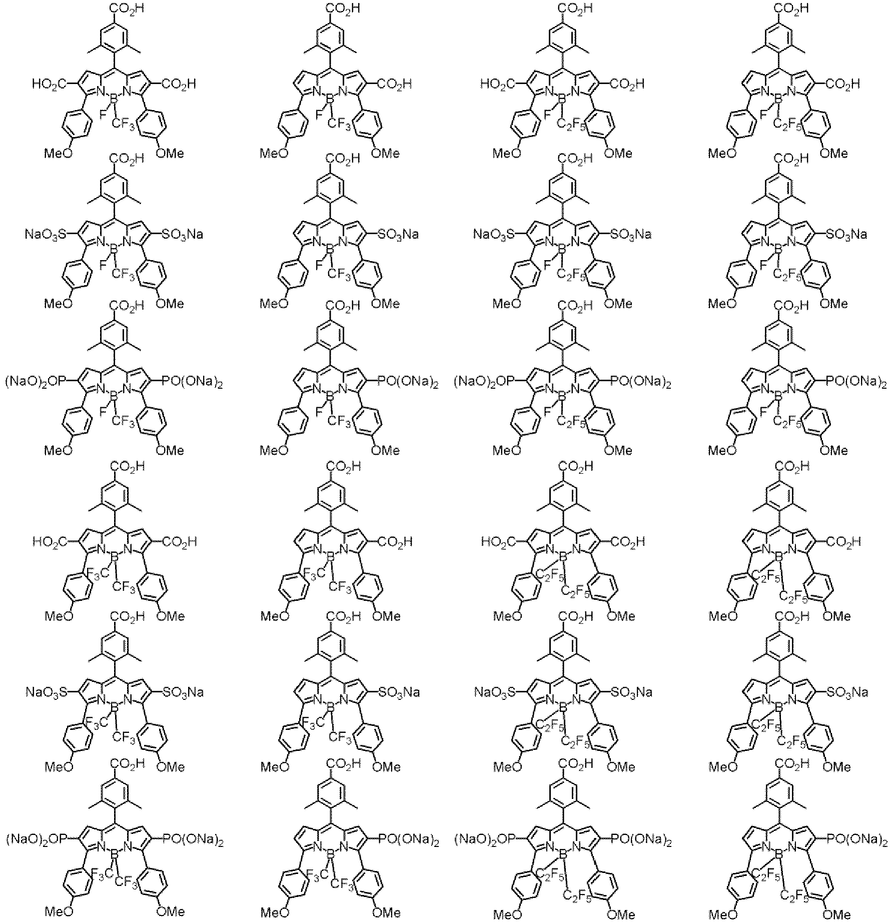

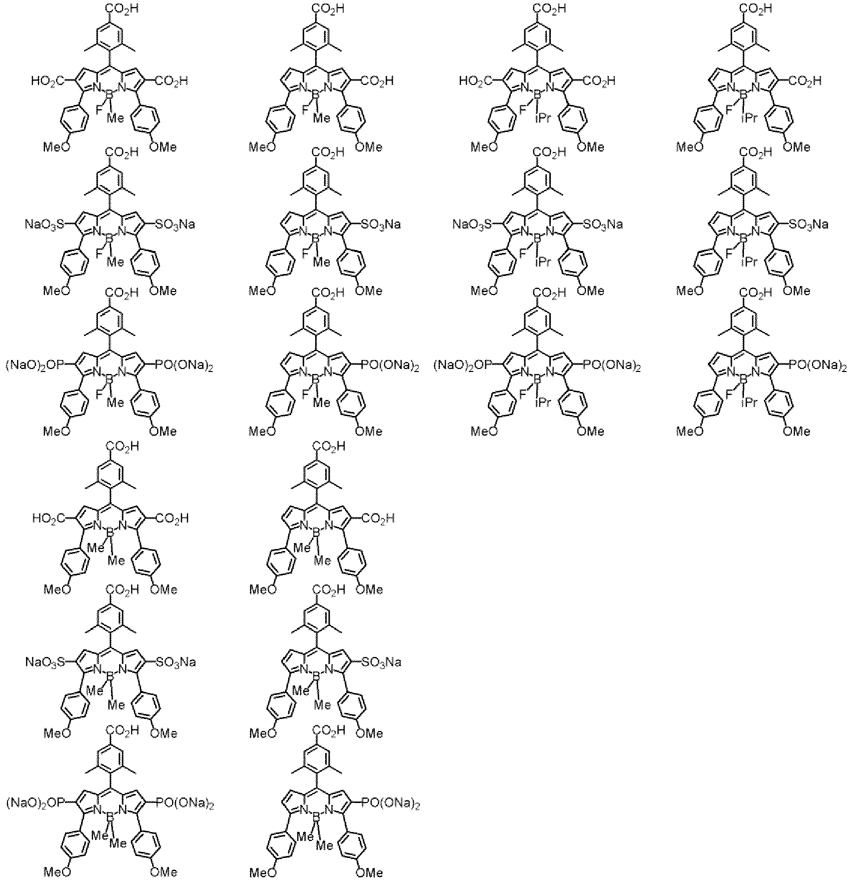

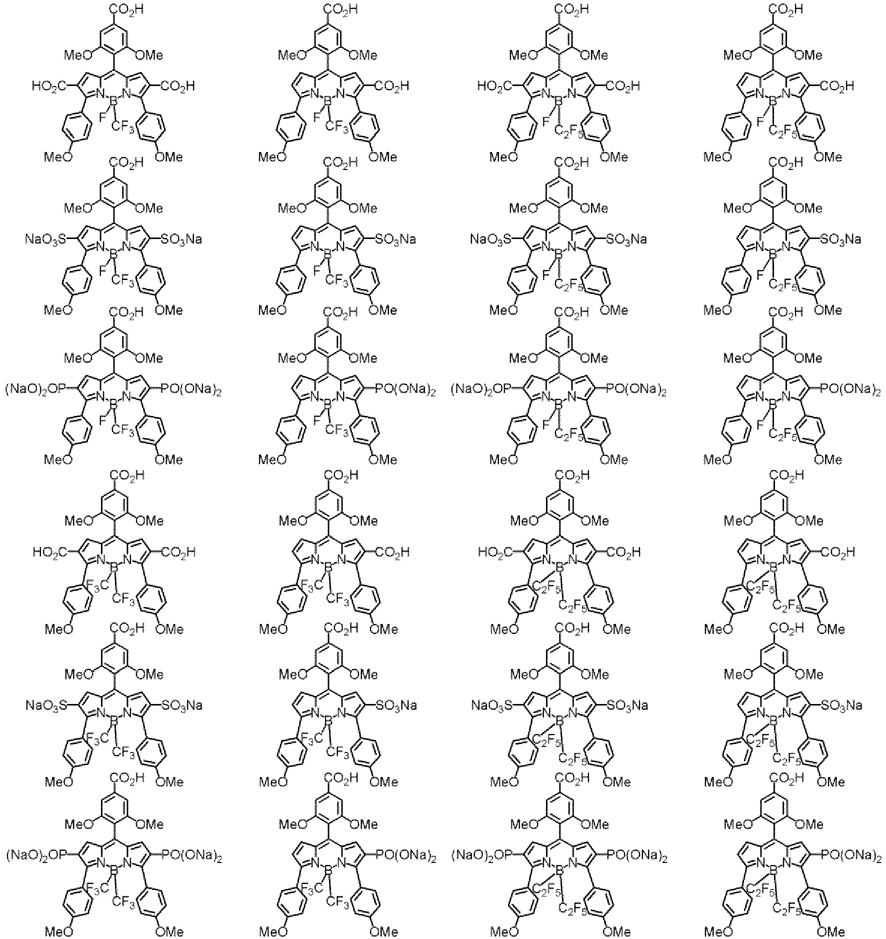

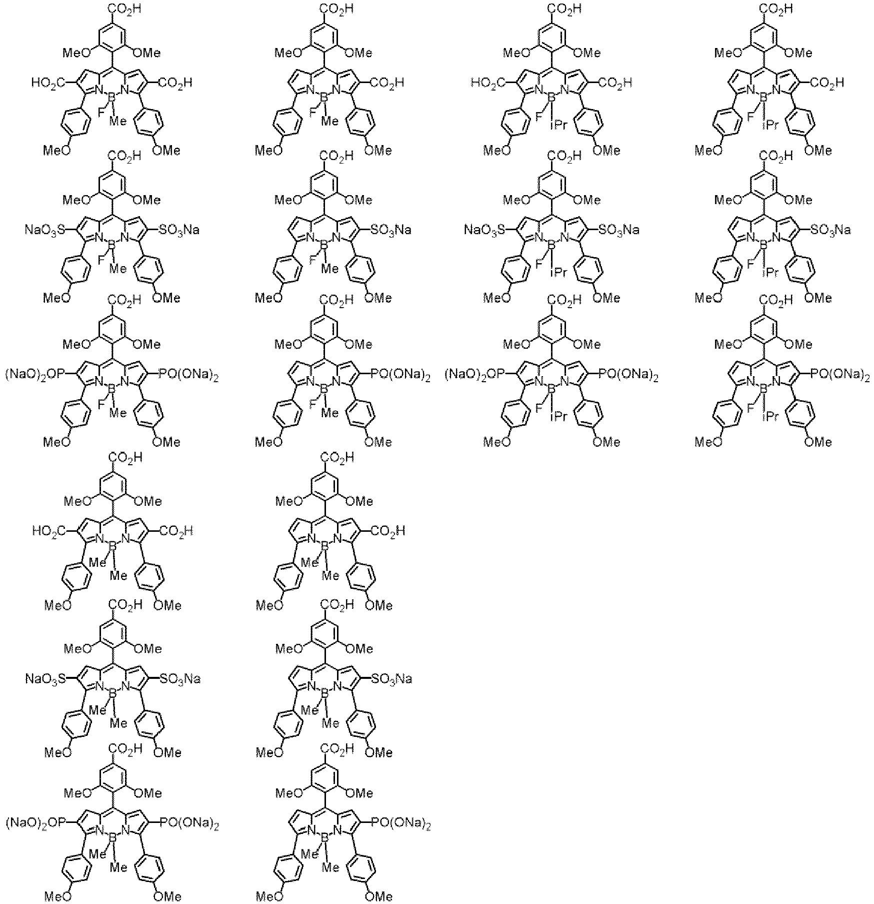

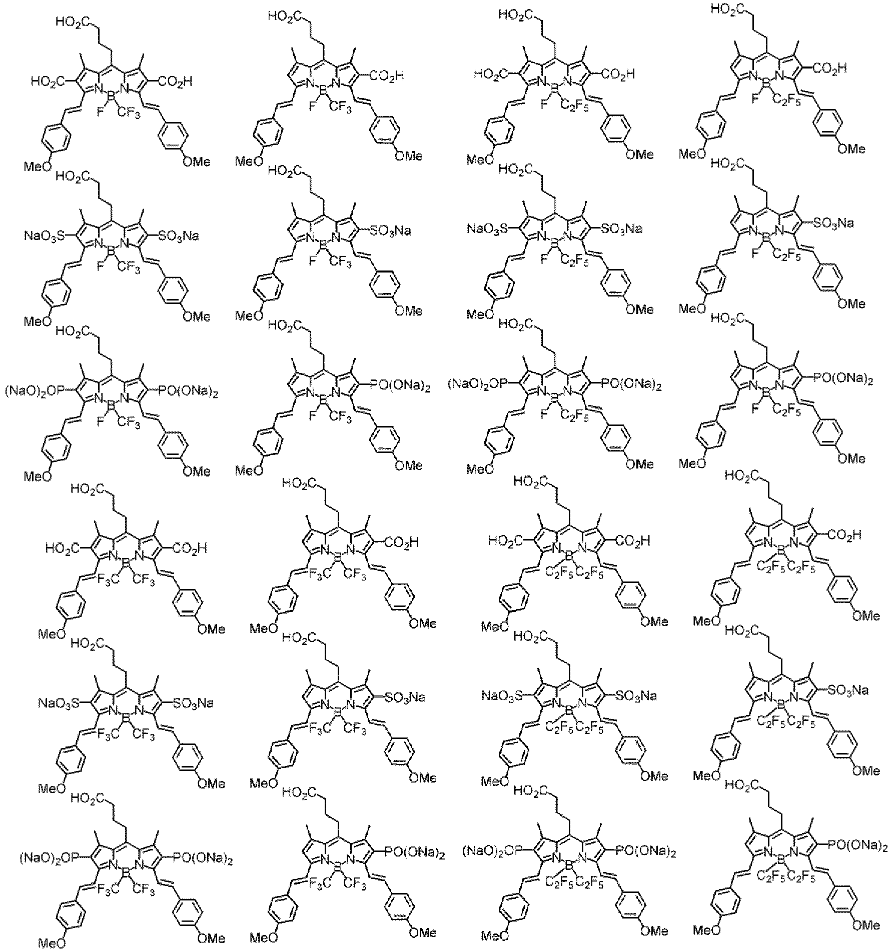

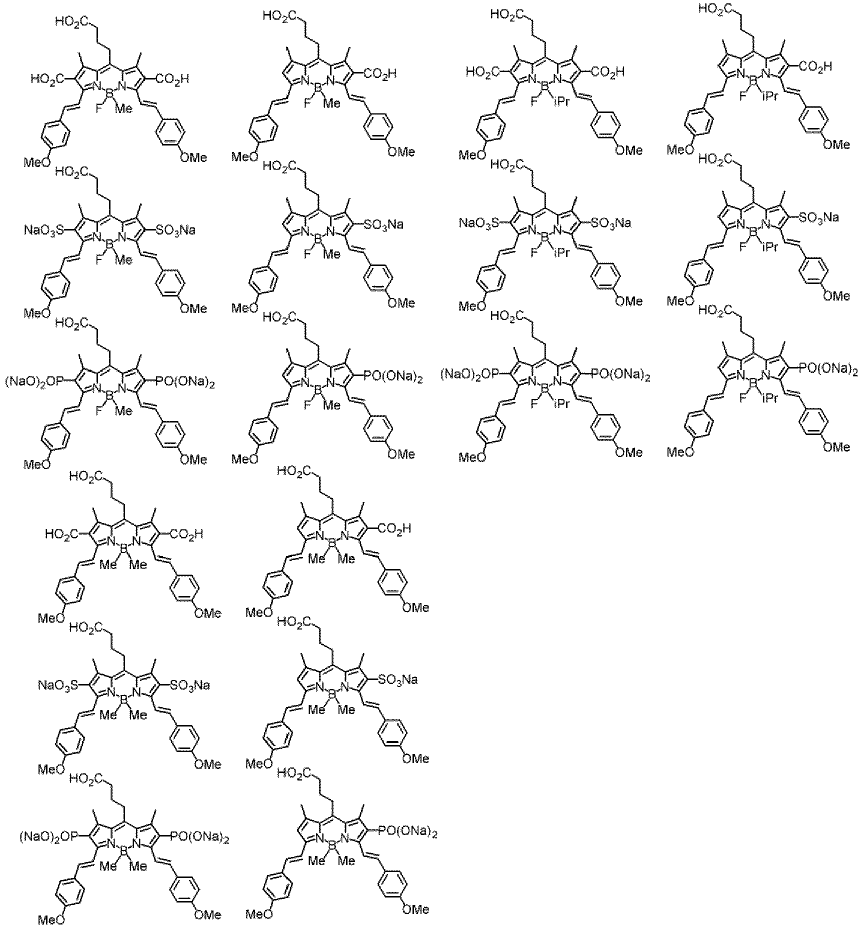

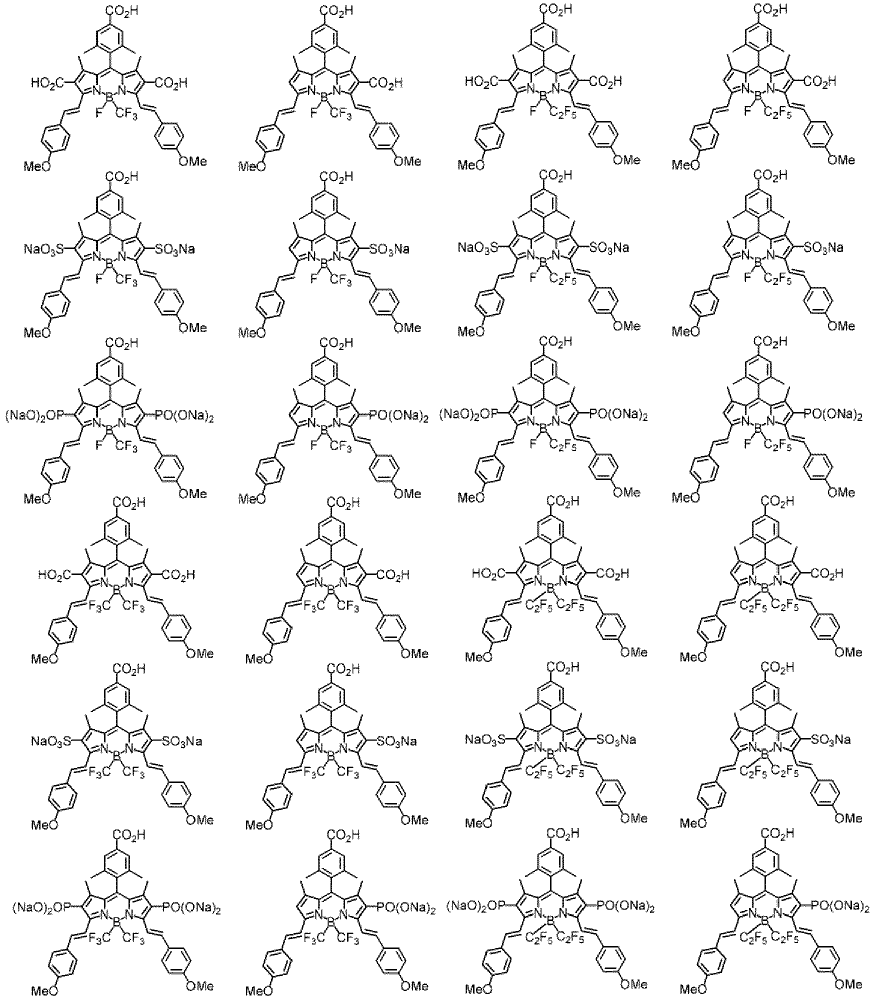

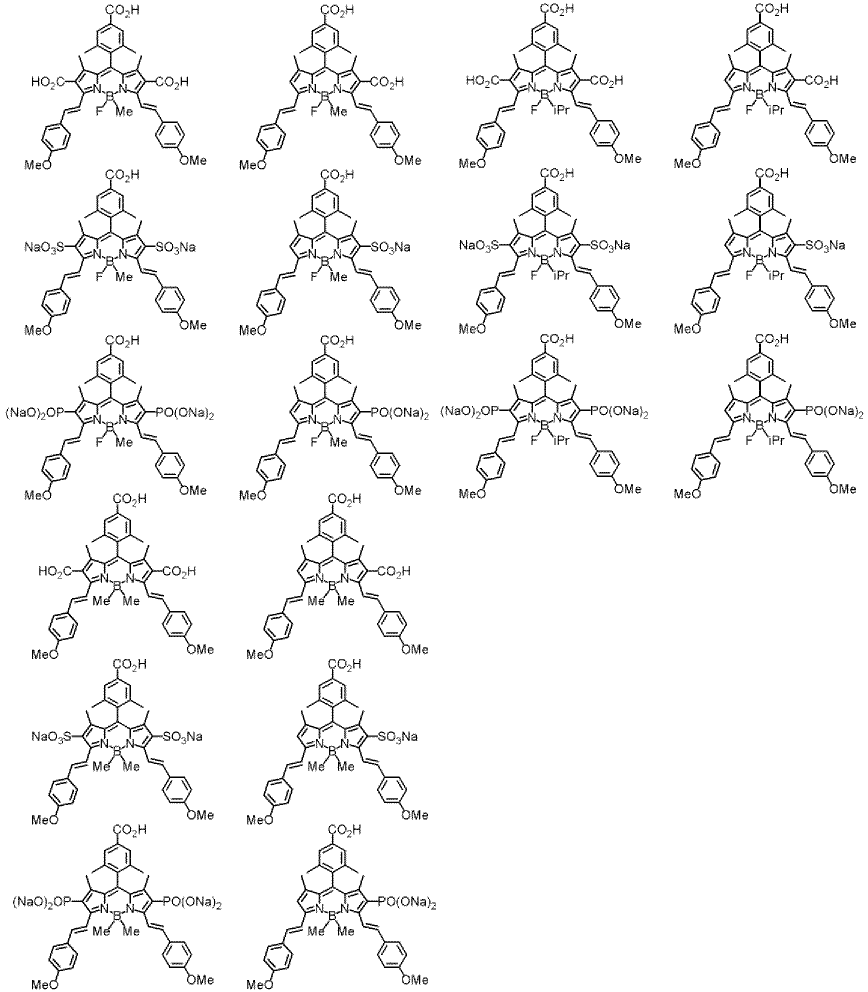

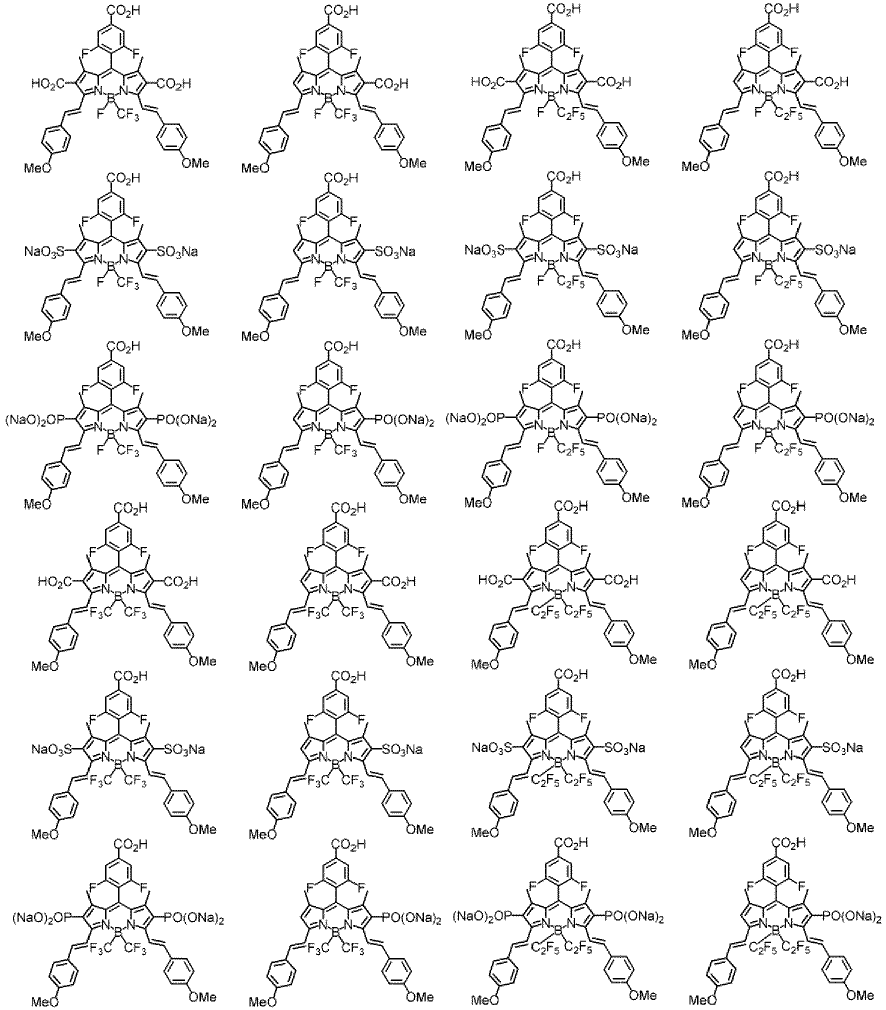

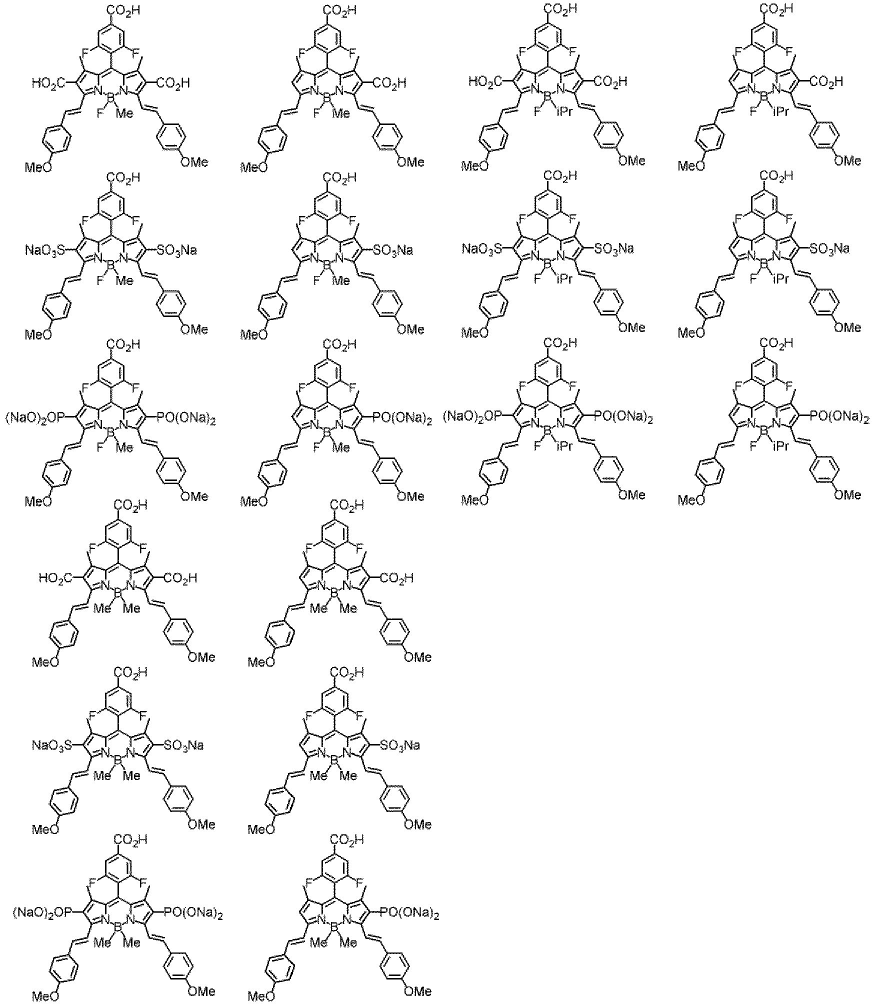

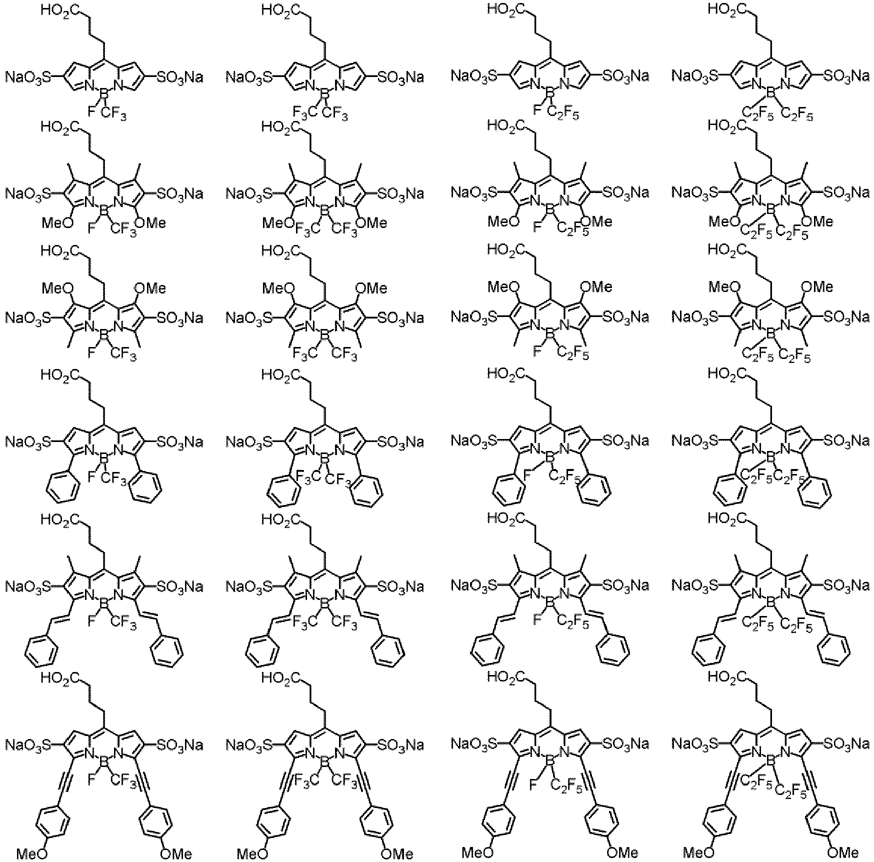

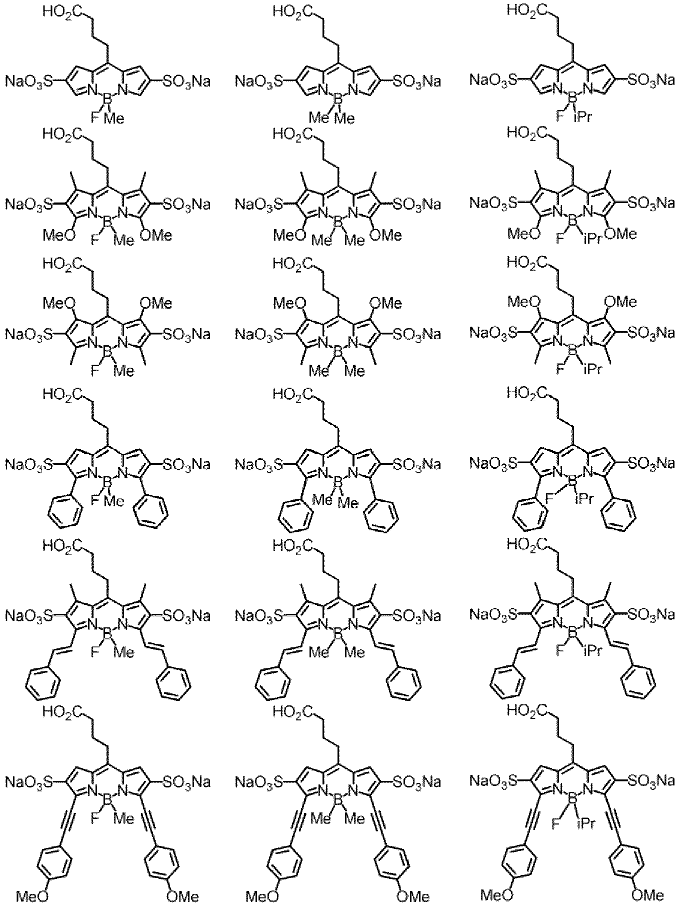

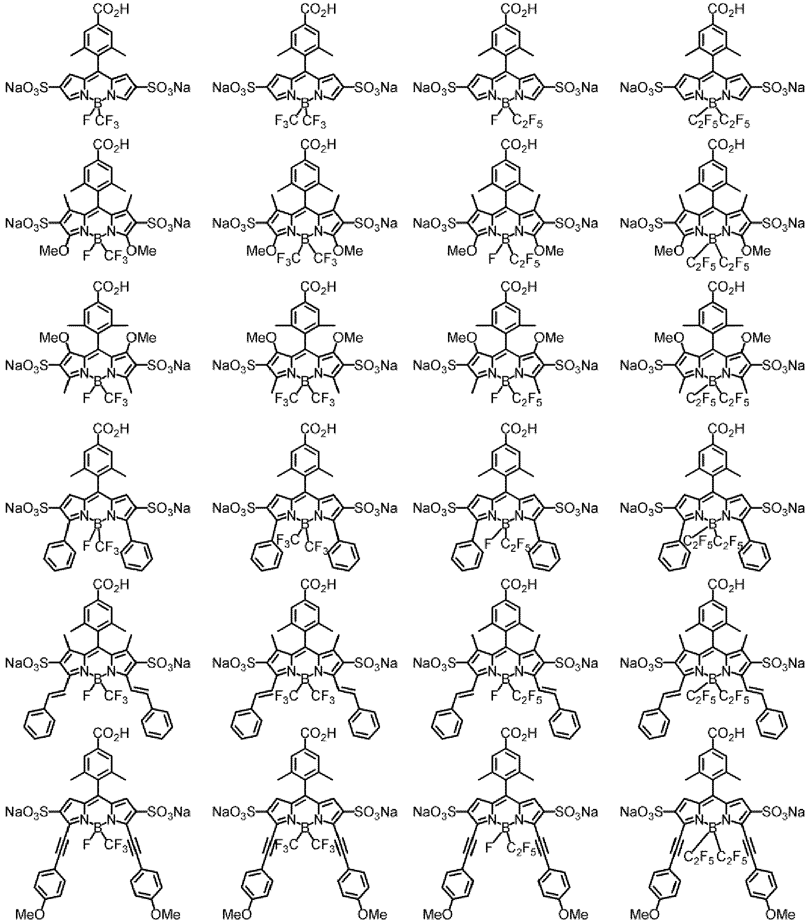

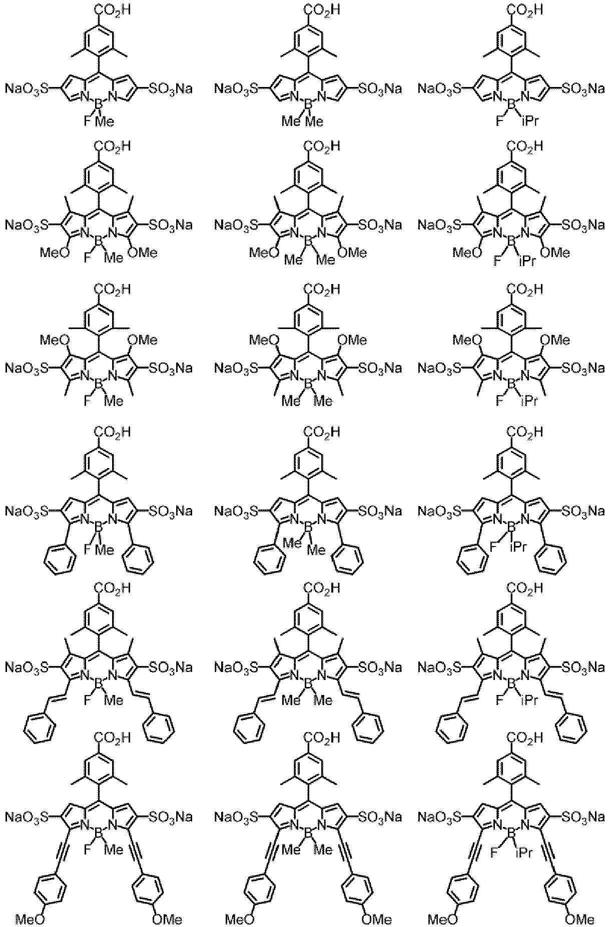

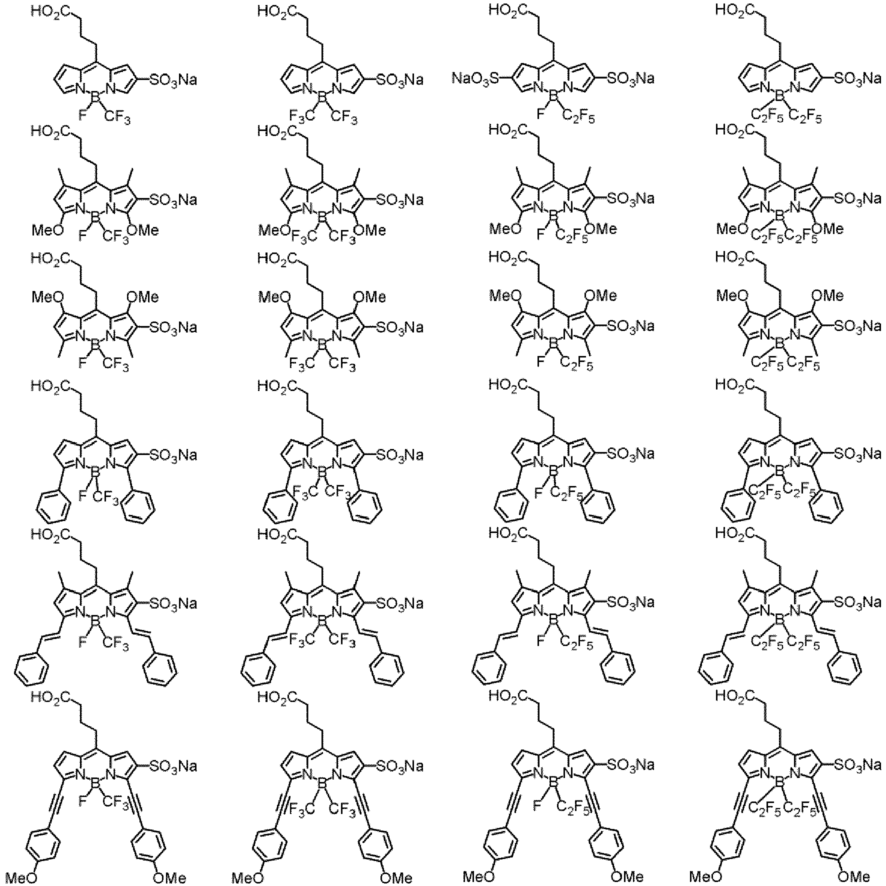

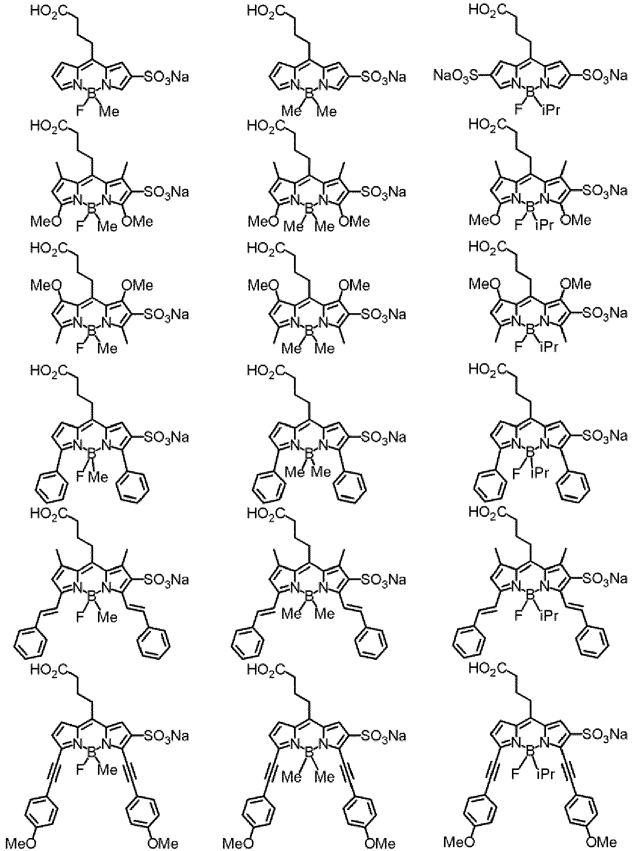

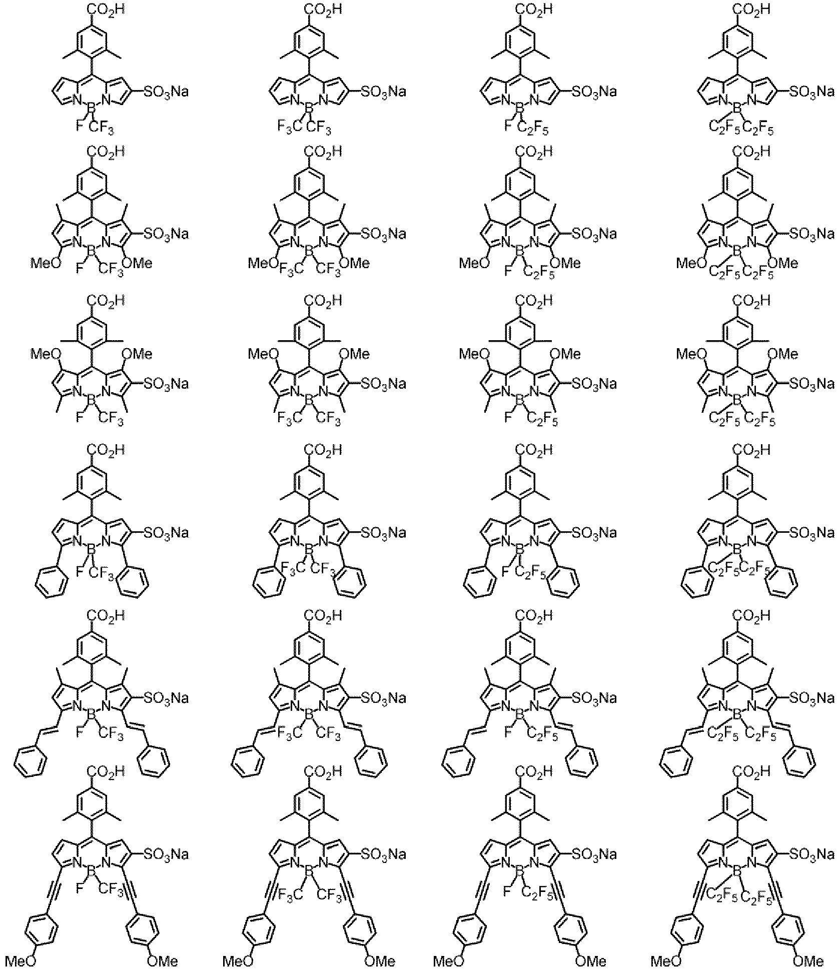

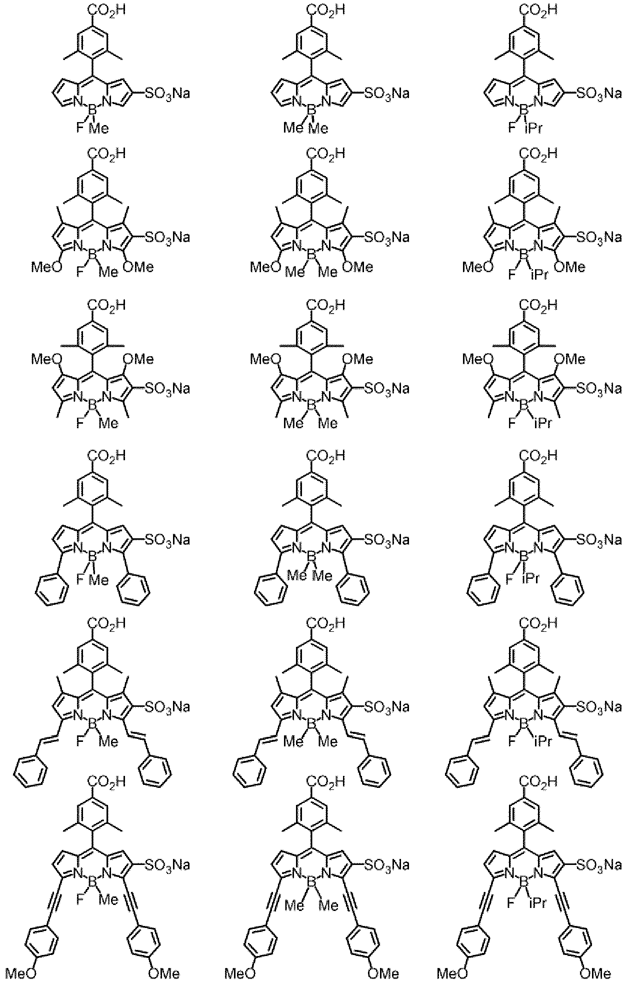

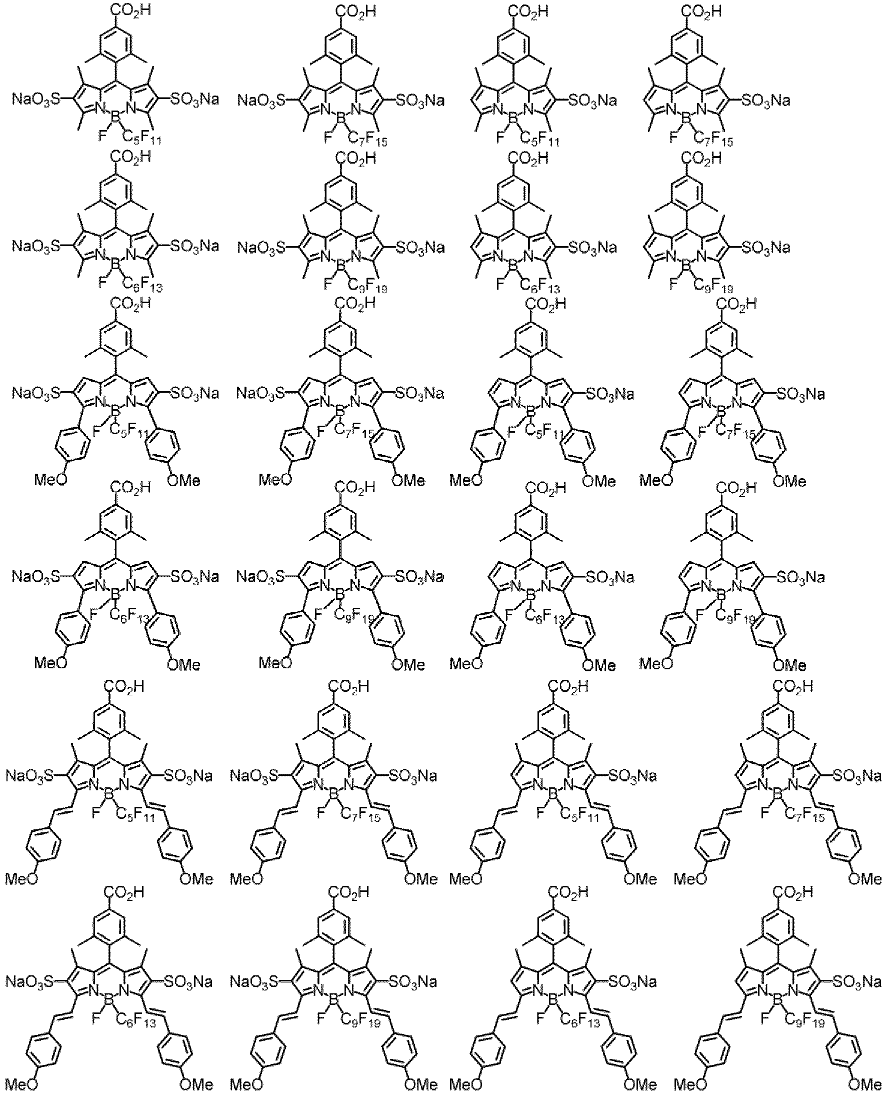

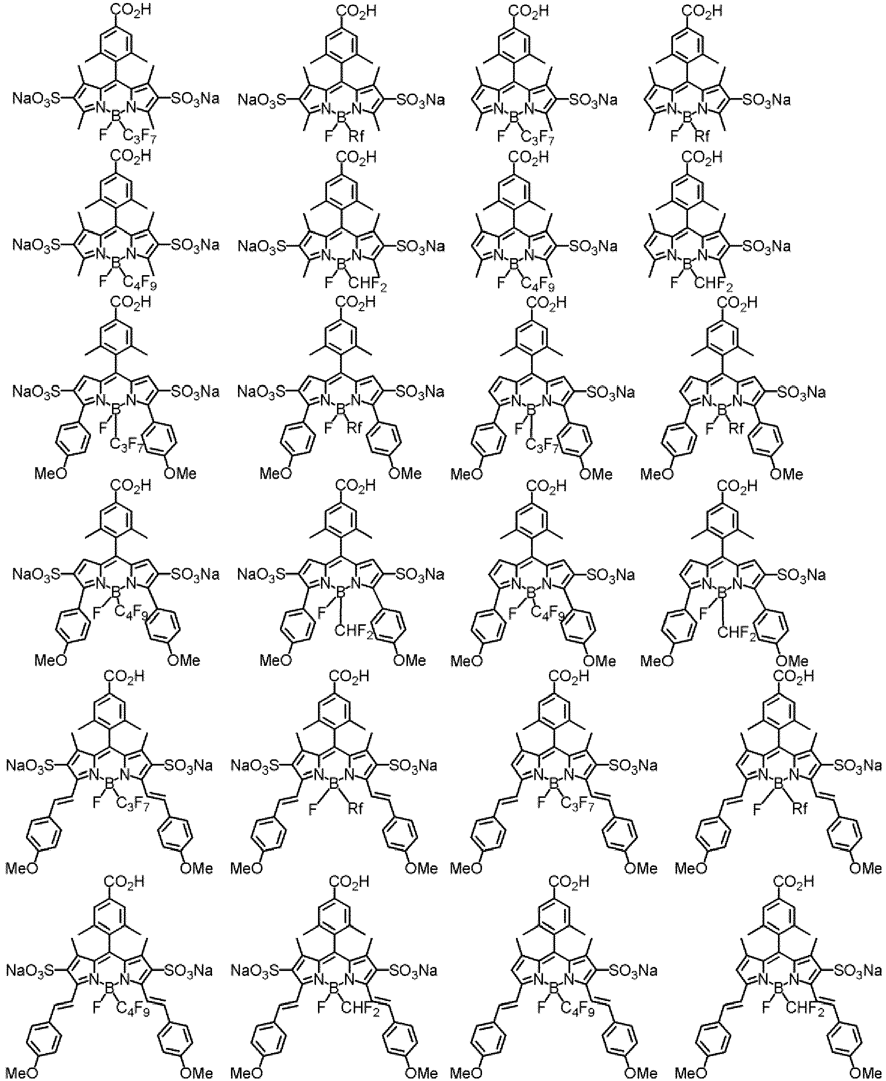

The fluorescent compound according to the embodiment of the present invention, which is represented by Formula (1), can be synthesized by a known method. Examples of the known method include methods described in, J. Org. Chem., 2008, 73(5), 1963-1970, and J. Mater. Chem. B, 2014, 2, 1576 to 1583.

The fluorescent compound according to the embodiment of the present invention, which is represented by Formula (1), has excellent hydrophilicity and thus can be used as a reagent for in vivo fluorescence imaging by being bonded to a biological substance such as a protein, a peptide, an amino acid, a nucleic acid, a sugar chain, and a lipid.

Further, the fluorescent compound according to the embodiment of the present invention, which is represented by Formula (1), can impart the excellent light resistance of the fluorescently labeled biological substance using this compound, in the biological tissue, and in particular, in a case where at least one of Q¹ or Q² is a halogenoalkyl group, the excellent light resistance (of the compound and the labeled biological substance) can be imparted even in a water-soluble solvent containing water (the water-soluble solvent is a solvent containing a relatively large amount of oxygen). As a result, as compared with the case of using the conventional fluorescent compound, it is possible to observe a living body with high resolution for a long time.

That is, the fluorescent compound according to the embodiment of the present invention, which is represented by Formula (1), includes a compound having a group capable of interacting (for example, physical adsorption and chemical bond) with a biological substance, and such a form is particularly preferable from the viewpoint of applying the fluorescent compound according to the embodiment of the present invention to the fluorescence labeling of the biological substance, such as in vivo fluorescence imaging.

Specifically, in the fluorescent compound according to the embodiment of the present invention, at least one of R¹ to R⁷, Q¹, or Q² preferably has a moiety bondable to a biological substance, and examples of the moiety bondable thereto include moieties described in the fluorescently labeled biological substance below.

Specific examples of the compound having a group capable of interacting with a biological substance are shown below, but the present invention is not limited to these compounds. In the following specific examples, a group having a dissociative hydrogen atom such as a specific hydrophilic group Pi may adopt a salt structure by dissociating the dissociative hydrogen atom.

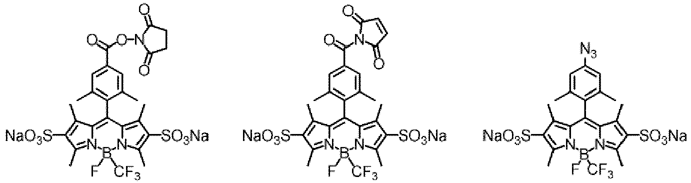

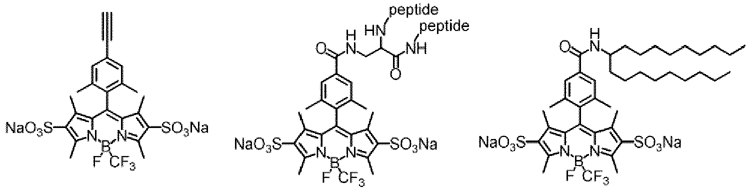

A compound having a group for acting (including adhesion) or bonding to a biological substance can be synthesized by a known method. For example, Bioconjugate Techniques (Third Edition, written by Greg T. Hermanson) can be referred to.

### «fluorescently labeled biological substance»

The fluorescently labeled biological substance according to the embodiment of the present invention (also simply referred to as the labeled biological substance) is a substance in which the fluorescent compound according to the embodiment of the present invention, which is represented by Formula (1), is bonded to a biological substance. The bond between the fluorescent compound represented by Formula (1) and a biological substance may have a form in which the fluorescent compound represented by Formula (1) and the biological substance are directly bonded or a form of being linked via a linking group.

Preferred examples of the biological substance include a protein, a peptide, an amino acid, a nucleic acid, a sugar chain, and a lipid. Preferred examples of the protein include an antibody, and preferred examples of the lipid include a phospholipid, a fatty acid, sterol. A labeled biological substance in which the fluorescent compound according to the embodiment of the present invention, which is represented by Formula (1), is bonded to an antibody is referred to as a labeled antibody.

Among the above biological substances, the clinically useful substance is not particularly limited, but examples thereof include immunoglobulins such as immunoglobulin (Ig) G, IgM, IgE, IgA, and IgD; plasma proteins such as complement, C-reactive protein (CRP), ferritin, α₁ microglobulin, β₂ microglobulin, and antibodies thereof; tumor markers such as α-fetoprotein, carcinoembryonic antigen (CEA), prostate acid phosphatase (PAP), carbohydrate antigen (CA) 19-9, and CA-125, and antibodies thereof; hormones such as luteinizing hormone (LH), follicle-stimulating hormone (FSH), human ciliated gonadotrobin (hCG), estrogen, and insulin, and antibodies thereof; and viral infection-related substances of viruses such HIV and ATL, hepatitis B virus (HBV)-related antigens (HBs, HBe, and HBc), human immunodeficiency virus (HIV), adult T-cell leukemia (ATL), and antibodies thereof.

The examples thereof further include bacteria such as Corynebacterium diphteriae, Clostridium botulinum, mycoplasma, and Treponema pallidum, and antibodies thereof; protozoa such as Toxoplasma, Trichomonas, Leishmania, Trypanosoma, and malaria parasites, and antibodies thereof; embryonic stem (ES) cells such as ELM3, HM1, KH2, v6.5, v17.2, v26.2 (derived from mice, 129, 129/SV, C57BL/6, and BALB/c), and antibodies thereof; antiepileptic drugs such as phenytoin and phenobarbital; cardiovascular drugs such as quinidine and digoxin; anti-asthma drugs such as theophylline; drugs such as antibiotics such as chloramphenicol and gentamicin, and antibodies thereof; and enzymes, extracellular toxins (for example, styrelidine O), and the like, and antibodies thereof. In addition, antibody fragments such as Fab'2, Fab, and Fv can also be used.

Examples of the specific form in which the fluorescent compound according to the embodiment of the present invention, which is represented by Formula (1), (hereinafter, also abbreviated as the fluorescent compound (1) and the biological substance interact with each other to be bonded include the forms described below,
i) Non-covalent bond (for example, hydrogen bond, ionic bond including chelate formation) or covalent bond between a peptide in the fluorescent compound (1) and a peptide in the biological substance,
ii) Van der Waals force between a long-chain alkyl group in the fluorescent compound (1) and a lipid bilayer, a lipid, or the like in the biological substance,
iii) an amide bond formed by reacting an N-hydroxysuccinimide ester (NHS ester) in the fluorescent compound (1) with an amino group in the biological substance,
iv) a thioether bond formed by reacting a maleimide group in the fluorescent compound (1) with a sulfanyl group (-SH) in the biological substance, and
v) a formation of a triazole ring, which is formed by Click reaction between an azido group in the fluorescent compound (1) and an acetylene group in the biological substance, or Click reaction between an acetylene group in the fluorescent compound (1)and an azido group in the biological substance.

In addition to the forms of the i) to v), the bond can be formed according to another form, for example, which is described in "Lucas C. D. de Rezende and Flavio da Silva Emery, A Review of the Synthetic Strategies for the Development of BODIPY Dyes for Conjugation with Proteins, Orbital: The Electronic Journal of Chemistry, 2013, Vol 5, No.1, p. 62-83". Further, the method described in the same document can be appropriately referred to for the production of the fluorescently labeled biological substance according to the embodiment of the present invention.

| Reactive group in fluorescent compound | Fluorescent compound | Reactive group in biological substance | Product (Bonding mode) |
|---|---|---|---|
| NHS ester | 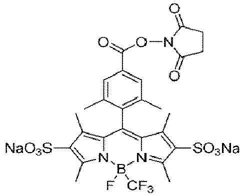 | Amino group (antibody or the like) | 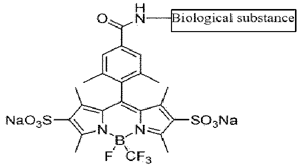 |
| Maleimide | 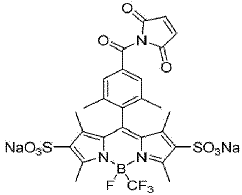 | Sulfanyl group (antibody or the like) | 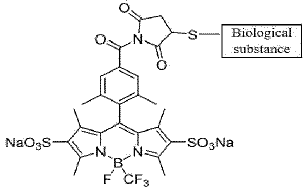 |
| Azide | 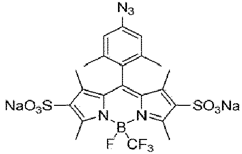 | Acetylene group (Click reaction) | 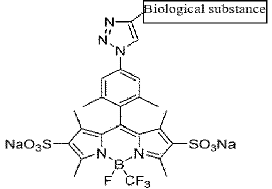 |
| Acetylene | 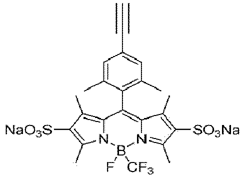 | Azide group (Click reaction) | 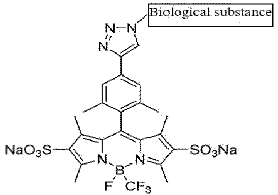 |
| Peptide (polyamino acid) | 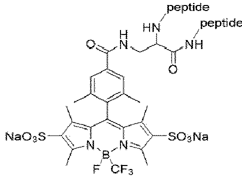 | Peptide bonding moiety (antibody or the like) | 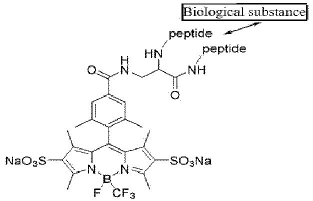 |
| Long chain alkyl | 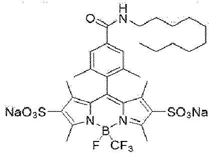 | Lipid bilayer Phospholipid or the like | Van der Waals force |

The fluorescently labeled biological substance according to the embodiment of the present invention has an excellent light resistance of the labeled biological substance in the biological tissue and is also excellent in hydrophilicity. Further, in a case where at least one of Q¹ or Q² of the fluorescently labeled biological substance according to the embodiment of the present invention is a halogenoalkyl group, a compound and a labeled biological substance are excellent in light resistance even in a water-soluble solvent containing water (a water-soluble solvent is a solvent containing a relatively large amount of oxygen), in addition to the excellent light resistance of the labeled biological substance in the biological tissue. As a result, as compared with the case of using the conventional fluorescently labeled biological substance having a dipyrromethene boron complex structure, such as a BODIPY (registered trademark) compound, it is possible to observe a living body with high resolution for a long time. Further, in a case where at least one of Q¹ or Q² of the fluorescently labeled biological substance according to the embodiment of the present invention is a halogenoalkyl group, since a compound and a labeled biological substance has excellent light resistance in a case of having a solution form dissolved in an aqueous medium such as physiological saline and a phosphate buffer solution, it also has excellent storage stability as the solution form.

### <Reagent containing fluorescently labeled biological substance>

In the reagent containing the fluorescently labeled biological substance according to the embodiment of the present invention, the form of the fluorescently labeled biological substance according to the embodiment of the present invention, for example, a solution form dissolved in an aqueous medium such as physiological saline and a phosphate buffer solution, and a solid form such as a fine particle powder or a lyophilized powder, is not particularly limited and can be appropriately selected depending on the purpose of use.

For example, in a case where the fluorescently labeled biological substance according to the embodiment of the present invention is used as a reagent for in vivo fluorescence imaging, it can be used as a reagent containing the fluorescently labeled biological substance having any one of the forms described above.

### <Use application of fluorescently labeled biological substance>

The fluorescently labeled biological substance according to the embodiment of the present invention, obtained from the fluorescent compound according to the embodiment of the present invention, which is represented by Formula (1), can stably detect fluorescence emitted from the fluorescent compound excited by light irradiation. Furthermore, it has sufficient hydrophilicity required for use as a reagent for in vivo fluorescence imaging. Accordingly, the fluorescently labeled biological substance according to the embodiment of the present invention is suitable as, for example, a reagent for in vivo fluorescence imaging.

Cells stained by using the fluorescently labeled biological substance according to the embodiment of the present invention as a fluorescent dye can maintain the fluorescence intensity for a long time since the fading is highly inhibited. Accordingly, the fluorescently labeled biological substance according to the embodiment of the present invention can be suitably used in vivo fluorescence imaging which requires excellent light resistance, for example, a long-term observation of a biological substance such as the microscopic observation by time-lapse measurement, and an observation of a biological substance by using high-resolution microscopes such as the confocal laser microscope and the super-resolution microscope such as the stimulated emission depletion microscope (STED microscope).

In vivo fluorescence imaging using the fluorescently labeled biological substance according to the embodiment of the present invention includes the following processes of (i) to (iii).
(i) A process of preparing a targeted biological substance (hereinafter, referred to as "target biological substance") and a fluorescently labeled biological substance according to the embodiment of the present invention in which a biological substance capable of bonding to the target biological substance is bonded to the fluorescent compound according to the embodiment of the present invention
(ii) A process of bonding the target biological substance to the fluorescently labeled biological substance according to the embodiment of the present invention
(iii) A process of irradiating a substance obtained by bonding the fluorescently labeled living body according to the embodiment of the present invention to the target biological substance with light having a range of the wavelength which is absorbed by the fluorescently labeled living body according to the embodiment of the present invention, and detecting the fluorescence emitted by the fluorescently labeled living body according to the embodiment of the present invention

In the in vivo fluorescence imaging described above, examples of the biological substance capable of bonding to the target biological substance include the above-described biological substance in the fluorescently labeled biological substance according to the embodiment of the present invention. The biological substance can be appropriately selected depending on the target biological substance (test object), and a biological substance capable of specifically binding to the test object can be selected.

Examples of the protein among the target biological substances include a protein, which is a so-called disease marker. The disease marker is not particularly limited, and examples thereof include α-fetoprotein (AFP), protein induced by vitamin K absence or antagonist II (PIVKA-II), breast carcinoma-associated antigen (BCA) 225, basic fetoprotein (BFP), carbohydrate antigen (CA) 15-3, CA19-9, CA72-4, CA125, CA130, CA602, CA54/61 (CA546), carcinoembryonic antigen (CEA), DUPAN-2, elastase 1, immunosuppressive acidic protein (IAP), NCC-ST-439, γ-seminoprotein (γ-Sm), prostate specific antigen (PSA), prostatic acid phosphatase (PAP), nerve specific enolase (NSE), Iba1, amyloid β, tau, squamous cell carcinoma associated antigen (SCC antigen), sialyl LeX-i antigen (SLX), SPan-1, tissue polypeptide antigen (TPA), serial Tn antigen (STN), cytokeratin (CYFRA) pepsinogen (PG), C-reactive protein (CRP), serum amyloid A protein (SAA), myoglobin, creatine kinase (CK), troponin T, and ventricular muscle myosin light chain I.

Examples of the bacterium among the above-described target biological substances include a bacterium to be subjected to a cellular and microbiological test, which are not particularly limited. Specific examples thereof include Escherichia coli, Salmonella, Legionella, and bacteria causing problems in public health.

The virus among the above-described target biological substances is not particularly limited, and examples thereof include hepatitis virus antigens such as hepatitis C and B virus antigens, p24 protein antigen of HIV virus, and pp65 protein antigen of cytomegalovirus (CMV), and E6 and E7 proteins of papillomavirus (HPV).

In the above (i), the target biological substance is not particularly limited and can be prepared according to a conventional method.

In addition, the fluorescently labeled biological substance according to the embodiment of the present invention is not particularly limited and can be prepared by bonding a biological substance capable of binding to a target biological substance to the fluorescent compound according to the embodiment of the present invention, according to a conventional method. Examples of the form of the bond and the reaction for forming the bond include the form in which the bond is formed by interaction and the reaction which are described in «fluorescently labeled biological substance» according to the embodiment of the present invention.

In the above (ii), the fluorescently labeled biological substance according to the embodiment of the present invention may be directly bonded to the target biological substance or may be bonded via another biological substance which is different from the fluorescently labeled biological substance according to the embodiment of the present invention and the target biological substance. In vivo fluorescence imaging using the fluorescently labeled biological substance according to the embodiment of the present invention is not particularly limited, and examples thereof include fluorescent cell staining. The fluorescent cell staining includes a direct method in which a fluorescently labeled antibody is used as a primary antibody and an indirect method in which a primary antibody is reacted with a secondary antibody that is used as a fluorescently labeled antibody. The fluorescently labeled biological substance according to the embodiment of the present invention can be used as a fluorescently labeled antibody in both the direct method and the indirect method but is preferably used as a fluorescently labeled antibody in the indirect method.

The binding of the fluorescently labeled biological substance according to the embodiment of the present invention to the target biological substance is not particularly limited and can be carried out according to a conventional method.

In the above (iii), the wavelength for exciting the fluorescently labeled living body according to the embodiment of the present invention is not particularly limited as long as the wavelength is an emission wavelength (wavelength light) capable of exciting the fluorescently labeled living body according to the embodiment of the present invention. Generally, the wavelength for excitation is preferably 300 to 1,000 nm and more preferably 400 to 800 nm.

The fluorescence excitation light source used in the present invention is not particularly limited as long as it emits an emission wavelength (wavelength light) capable of exciting the fluorescently labeled living body according to the embodiment of the present invention, and various laser light sources can be used. Examples of the laser light source include a He-Ne laser, a CO₂ laser, an Ar ion laser, a Kr ion laser, a He-Cd laser, an excimer laser, a gas laser such as nitrogen laser, a ruby laser, a yttrium-aluminum-garnet (YAG) laser, a solid-state laser such as glass laser, a dye laser, and a semiconductor laser. In addition, various optical filters can be used to obtain a preferred excitation wavelength or detect only fluorescence.

Other matters in the above (i) to (iii) are not particularly limited and can be appropriately selected depending on conditions such as a method, a reagent, and an apparatus, which are usually used.

In the in vivo fluorescence imaging using the fluorescently labeled biological substance according to the embodiment of the present invention, the fading of the substance obtained by bonding the fluorescently labeled living body according to the embodiment of the present invention to the target biological substance is highly inhibited, whereby a biological substance can be observed while maintaining the fluorescence intensity for a long time. Further, even in a case where a high resolution microscope or a super resolution microscope is used, observation can be carried out while maintaining the fluorescence intensity.

Further, in addition to the above, the fluorescently labeled biological substance according to the embodiment of the present invention can be suitably used even in the long-term storage of stained cells, by appropriately adjusting the storage conditions.

### - Substituent group T -

In the present invention, the preferred substituents include those selected from the following substituent group T.

In addition, in the present specification, in a case where it is simply described as a substituent, the substituent refers to the substituent group T, and in a case where an individual group, for example, an alkyl group is only described, a corresponding group in the substituent group T is preferably applied.

Further, in the present specification, in a case where an alkyl group is described separately from a cyclic (cyclo)alkyl group, the alkyl group is used to include a linear alkyl group and a branched alkyl group. On the other hand, in a case where an alkyl group is not described separately from a cyclic alkyl group, and unless otherwise specified, the alkyl group is used to include a linear alkyl group, a branched alkyl group, and a cycloalkyl group. This also applies to groups (alkoxy group, alkylthio group, alkenyloxy group, and the like) containing a group capable of having a cyclic structure (alkyl group, alkenyl group, alkynyl group, and the like) and compounds containing a group capable of having a cyclic structure. In a case where a group is capable of forming a cyclic skeleton, the lower limit of the number of atoms of the group forming the cyclic skeleton is 3 or more and preferably 5 or more, regardless of the lower limit of the number of atoms specifically described below for the group that can adopt this structure,

In the following description of the substituent group T, a group having a linear or branched structure and a group having a cyclic structure, such as an alkyl group and a cycloalkyl group, are sometimes described separately for clarity.

As the groups included in the substituent group T, the following groups are included.

An alkyl group (preferably having 1 to 30 carbon atoms, more preferably 1 to 20 carbon atoms, still more preferably 1 to 12 carbon atoms, still more preferably 1 to 8 carbon atoms, still more preferably 1 to 6 carbon atoms, and particularly preferably 1 to 3 carbon atoms), an alkenyl group (preferably having 2 to 30 carbon atoms, more preferably 2 to 20 carbon atoms, still more preferably 2 to 12 carbon atoms, still more preferably 2 to 6 carbon atoms, and even still more preferably 2 to 4 carbon atoms), an alkynyl group (preferably having 2 to 30 carbon atoms, still more preferably 2 to 20 carbon atoms, still more preferably 2 to 12 carbon atoms, still more preferably 2 to 6 carbon atoms, and even still more preferably 2 to 4 carbon atoms), a cycloalkyl group (preferably having 3 to 20 carbon atoms), a cycloalkenyl group (preferably having 5 to 20 carbon atoms), an aryl group (it may be a monocyclic group or may be a condensed ring group (preferably a condensed group in which 2 to 6 rings are condensed); in a case of a condensed ring group, it consists of a 5-membered to 7-membered ring; and the aryl group preferably has 6 to 40 carbon atoms, more preferably 6 to 30 carbon atoms, still more preferably 6 to 26 carbon atoms, and particularly preferably 6 to 10 carbon atoms), a heterocycle group (it has, as a ring-constituting atom, at least one nitrogen atom, an oxygen atom, a sulfur atom, a phosphorus atom, a silicon atom, or selenium atom, may be a monocyclic ring, or may be a condensed ring group (preferably a condensed group in which 2 to 6 rings are condensed); in a case of a monocyclic group, the monocyclic ring is preferably a 5-membered to 7-membered ring and more preferably a 5-membered or 6-membered ring; the heterocycle group preferably has 2 to 40 carbon atoms and more preferably 2 to 20 carbon atoms; and the heterocyclic group includes an aromatic heterocyclic group (a heteroaryl group) and an aliphatic heterocyclic group (an aliphatic heterocyclic group), an alkoxy group (preferably having 1 to 20 carbon atoms, and more preferably having 1 to 12 carbon atoms), an alkenyloxy group (preferably having 2 to 20 carbon atoms, and more preferably having 2 to 12 carbon atoms), and an alkynyloxy group (preferably having 2 to 20 carbon atoms, and more preferably having 2 to 12 carbon atoms), a cycloalkyloxy group (preferably having 3 to 20 carbon atoms), an aryloxy group (preferably having 6 to 40 carbon atoms, more preferably having 6 to 26 carbon atoms, and still more preferably having 6 to 14 carbon atoms), a heterocyclic oxy group (preferably having 2 to 20 carbon atoms),
an alkoxycarbonyl group (preferably having 2 to 20 carbon atoms), a cycloalkoxycarbonyl group (preferably having 4 to 20 carbon atoms), an aryloxycarbonyl group (preferably having 6 to 20 carbon atoms), an amino group (preferably having 0 to 20 carbon atoms; the amino group includes an unsubstituted amino group (-NH₂), a (mono- or di-) alkylamino group, a (mono- or di-) alkenylamino group, a (mono- or di-) alkynylamino group, a (mono- or di-) cycloalkylamino group, a (mono- or di-) cycloalkenylamino group, a (mono- or di-) arylamino group, or a (mono- or di-) heterocyclic amino group, where each of the above groups substituting an unsubstituted amino group has the same definition as the corresponding group in the substituent group T), a sulfamoyl group (preferably having 0 to 20 carbon atoms; the sulfamoyl group is preferably an alkyl, cycloalkyl, or aryl sulfamoyl group), an acyl group (preferably having 1 to 20 carbon atoms, and more preferably having 2 to 15 carbon atoms), an acyloxy group (preferably having 1 to 20 carbon atoms), a carbamoyl group (preferably having 1 to 20 carbon atoms; the carbamoyl group is preferably an alkyl, cycloalkyl, or aryl carbamoyl group),
an acylamino group (preferably having 1 to 20 carbon atoms), a sulfonamide group (preferably having 0 to 20 carbon atoms and preferably an alkyl, cycloalkyl, or aryl sulfonamide group), an alkylthio group (preferably having 1 to 20 carbon atoms and more preferably 1 to 12 carbon atoms), a cycloalkylthio group (preferably having 3 to 20 carbon atoms), an arylthio group (preferably having 6 to 40 carbon atoms, more preferably 6 to 26 carbon atoms, and still more preferably 6 to 14 carbon atoms), a heterocyclic thio group (preferably having 2 to 20 carbon atoms), an alkyl, cycloalkyl, or aryl sulfonyl group (preferably having 1 to 20 carbon atoms),
a silyl group (preferably having 1 to 30 carbon atoms and more preferably 1 to 20 carbon atoms, and preferably substituted with an alkyl, aryl, alkoxy, or aryloxy), a silyloxy group (preferably having 1 to 20 carbon atoms and preferably substituted with an alkyl, aryl, alkoxy, or aryloxy), a hydroxy group, a cyano group, a nitro group, a halogen atom (for example, a fluorine atom, a chlorine atom, a bromine atom or an iodine atom), an oxygen atom (specifically replacing >CH₂ which constitutes a ring with >C=O), a carboxy group (-CO₂H), a phosphono group [-PO(OH)₂], a phosphonooxy group [-O-PO(OH)₂], a sulfo group (-SO₃H), a boric acid group [-B(OH)₂], an onio group (an ammonio group including a cyclic ammonio group, which contains a sulfonio group(-SH₂+) or a phosphonio group (-PH3+), and preferably has 0 to 30 carbon atoms and more preferably 1 to 20 carbon atoms), a sulfanyl group (-SH), an amino acid residue, or a polyamino acid residue.

Further, the substituent group T includes a carboxy group, a phosphono group, a sulfo group, an onio group, an amino acid residue, or the above-described alkyl group, alkenyl group, alkynyl group, cycloalkyl group, cycloalkenyl group, aryl group, heterocycle group, alkoxy group, alkenyloxy group, alkynyloxy group, cycloalkyloxy group, aryloxy group, heterocyclic oxy group, alkoxycarbonyl group, cycloalkoxycarbonyl group, aryloxycarbonyl group, amino group, sulfamoyl group, acyl group, acyloxy group, carbamoyl group, acylamino group, sulfonamide group, alkylthio group, cycloalkylthio group, arylthio group, heterocyclic thio group, and an alkyl, cycloalkyl, or aryl sulfonyl group, where this above-described group has a polyamino acid residue as a substituent.

The substituent selected from the substituent group T is more preferably an alkyl group, an alkenyl group, a cycloalkyl group, an aryl group, a heterocycle group, an alkoxy group, a cycloalkoxy group, an aryloxy group, an alkoxycarbonyl group, a cycloalkoxycarbonyl group, an amino group, an acylamino group, a cyano group or a halogen atom, and particularly preferably an alkyl group, an alkenyl group, an aryl group, a heterocycle group, an alkoxy group, an alkoxycarbonyl group, an amino group, an acylamino group, or a cyano group.

The substituent selected from the substituent group T also includes a group obtained by combining a plurality of the above groups, unless otherwise specified. For example, in a case where a compound, a substituent, or the like contains an alkyl group, an alkenyl group, or the like, the alkyl group, the alkenyl group, or the like may be substituted or unsubstituted. In addition, in a case where a compound, a substituent, or the like contains an aryl group, a heterocycle group, or the like, the aryl group, the heterocycle group, or the like may be a monocyclic ring or a condensed ring, and may be substituted or unsubstituted.

### Examples

Hereinafter, the present invention will be described in more detail based on Examples, but the present invention is not limited thereto. In the present invention, room temperature means 25°C.

The compounds and labeled antibodies used as Examples and Comparative Examples are shown below.

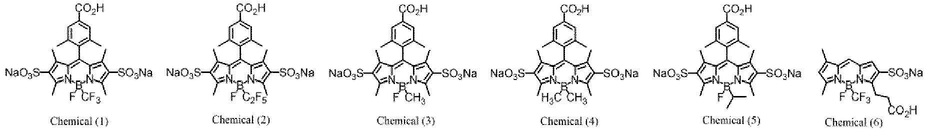

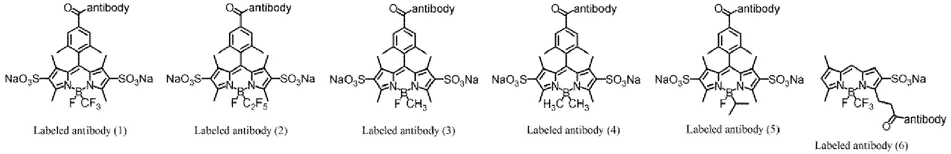

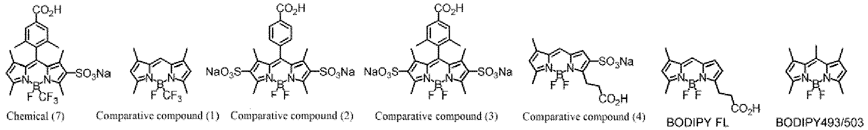

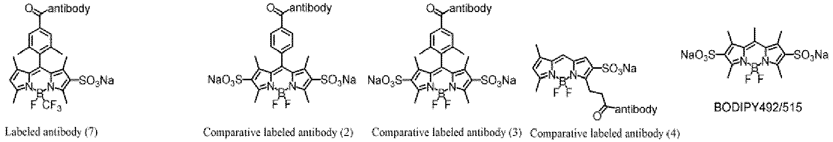

The comparative compound (1) is the compound 3 described in paragraph [0065] of WO2016/143699A.

The comparative compound (2) is the compound described in RSC Advances, 2016, vol.6, 38, p.32070-32073.

The comparative compounds (1) and (2) were synthesized according to the methods described in the respective documents.

The comparative labeled antibody (2) was synthesized according to the same method as the method of synthesizing the labeled antibody (1) described later.

BODIPY FL, BODIPY 493/503, and BODIPY 492/515 are all commercially available products (product names) manufactured by Thermo Fisher Scientific, Inc.

The methods of synthesizing a compound and a labeled antibody will be described in detail below; however, the starting materials, the dye intermediates, and the synthetic routes are not limited thereto.

Unless otherwise specified, as the carrier in the silica gel column chromatography, SNAP KP-Sil Cartridge (manufactured by Biotage, LLC) and High-Flash column W001, W002, W003, W004, or W005 [manufactured by Yamazen Corporation] were used. As NH silica, SNAP KP-NH Cartridge (manufactured by Biotage, LLC) was used. The mixing ratio in the eluent is based on a volume ratio. For example, "ethyl acetate:normal hexane = from 0:100 to 100:0" means that the eluent of "ethyl acetate:normal hexane = 0:100" is changed to an eluent of "ethyl acetate:normal hexane = 100:0".

The MS spectrum was measured by ACQUITY SQD LC/MS System [manufactured by Waters Corporation, ionization method: electrospray Ionization (ESI)] or LCMS-2010EV [manufactured by Shimadzu Corporation, ionization method: an ionization method simultaneously carrying out ESI and atomospheric pressure chemical ionization (APCI)].

Unless otherwise specified, the synthesized compound and the labeled antibody used in each Example were those stored under the light-shielded conditions in a case where they were not used immediately after preparation. In addition, the commercially available compound and labeled antibody were stored under the light-shielded conditions after purchase until use.

The abbreviations used are summarized below.
DDQ: 2,3-dichloro-5,6-dicyano-1,4-benzoquinone
DCC: dicyclohexylcarbodiimide
NHS: N-hydroxysuccinimide
TMSOTf: trimethylsilyl trifluoromethanesulfonate
TFA: trifluoroacetic acid
DIPEA: diisopropylethylamine
DCM: dichloromethane
DMAP: 4-dimethylaminopyridine
DMF: N,N-dimethylformamide
DMSO: dimethyl sulfoxide
PBS: phosphate buffered saline
Boc or BOC: tert-butoxycarbonyl group
Me: methyl group
Et: ethyl group
TMS: trimethylsilyl group

### [Synthesis Example 1: Synthesis of compound (1) and labeled antibody (1)]

A compound (1) and a labeled antibody (1) were synthesized based on the following scheme.

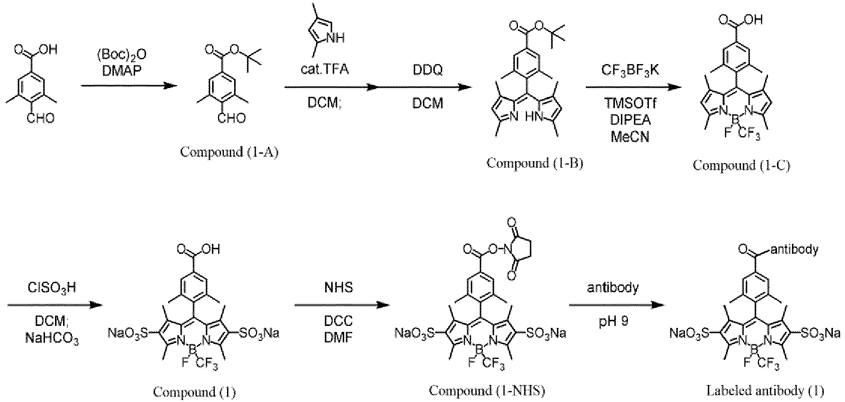

### <Synthesis of compound (1-A)>

1.0 g of 3,5-dimethyl-4-formyl benzoic acid (AOBChem USA), 2.6 mL of di-tert-butyl dicarbonate (FUJIFILM Wako Pure Chemical Corporation), 1.0 g of N,N-dimethyl-4-aminopyridine (FUJIFILM Wako Pure Chemical Corporation), 5.4 mL of tert-butanol (FUJIFILM Wako Pure Chemical Corporation), and 5.3 mL of tetrahydrofuran (FUJIFILM Wako Pure Chemical Corporation) were added to a 50 mL eggplant flask, and the resultant mixture was stirred at room temperature for 16 hours. After adding water, extraction was carried out with ethyl acetate, drying was carried out with sodium sulfate, and the solvent was distilled off under reduced pressure. The obtained residue was purified by silica gel column chromatography (ethyl acetate:normal hexane = 5:95), the solvent was distilled off under reduced pressure, whereby 0.95 g of a compound (1-A) (yield: 72%, white solid) was obtained.

### <Synthesis of compound (1-B)>

0.75 g of 2,4-dimethyl-1H-pyrrole (FUJIFILM Wako Pure Chemical Corporation), 0.74 g of the compound (1-A), and 32 mL of methylene chloride were added to a 50 mL eggplant flask, and after adding 0.02 mL of trifluoroacetic acid (FUJIFILM Wako Pure Chemical Corporation), the mixture was stirred at room temperature for 1 hour. Subsequently, 1.15 g of 2,3-dichloro-5,6-dicyano-1,4-benzoquinone (FUJIFILM Wako Pure Chemical Corporation) was added under ice-cooling, the temperature was raised to room temperature, and the mixture was stirred for 30 minutes. Subsequently, water was added, the mixture was extracted with methylene chloride, and purification was carried out by silica gel column chromatography (ethyl acetate: normal hexane = 15:85). The solvent was distilled off under reduced pressure to obtain 0.7 g (yield: 55%, brown solid) of a compound (1-B).
[M + H⁺]⁺: 405

### <Synthesis of compound (1-C)>

0.11 g of the compound (1-B) and 6 mL of methylene chloride were added to a 50 mL eggplant flask, and after adding 0.14 mL of N,N-diisopropylethylamine (FUJIFILM Wako Pure Chemical Corporation) under a nitrogen atmosphere, the resultant mixture was stirred at room temperature for 10 minutes. Subsequently, a solution obtained by mixing 0.18 g of potassium trifluoro(trifluoromethyl)borate (FUJIFILM Wako Pure Chemical Corporation), 0.46 g of trimethylsilyl trifluoromethanesulfonate (FUJIFILM Wako Pure Chemical Corporation), and 1.5 mL of acetonitrile was added under ice-cooling, the temperature was raised to room temperature, and the mixture was stirred for 3 hours. The reaction mixture was directly charged on silica gel and purified by silica gel column chromatography (ethyl acetate: normal hexane = from 0:100 to 100:0). The solvent was distilled off under reduced pressure to obtain 0.13 g (yield: 99%, reddish brown solid) of a compound (1-C).
[M + H⁺]⁺: 447

### <Synthesis of compound (1)>

0.07 g of the compound (1-C) and 14 mL of methylene chloride were added to a 50 mL eggplant flask, 0.09 mL of chlorosulfonic acid (FUJIFILM Wako Pure Chemical Corporation) was added thereto under ice-cooling, and the resultant mixture was stirred for 5 minutes. Subsequently, 0.59 g of sodium hydrogen carbonate (FUJIFILM Wako Pure Chemical Corporation) and 4.7 mL of distilled water were added, and after distilling off methylene chloride under reduced pressure, the mixed solution was purified by reverse phase silica gel column chromatography (manufactured by Biotage, LLC, a C18 column, distilled water only), and the solvent was removed by evaporation and freeze-drying to obtain 0.05 g (yield: 53%, red solid) of a compound (1).
[M - 2Na⁺ + H⁺]⁻: 605

### <Synthesis of compound (1-NHS)>

0.05 g of the compound (1), 0.04 g of N-hydroxysulfuric acid imide (FUJIFILM Wako Pure Chemical Corporation), 0.13 g of dicyclohexylcarbodiimide (FUJIFILM Wako Pure Chemical Corporation), and 0.64 mL of dimethylformamide was added to a 50 mL eggplant flask, and the resultant mixture was stirred at room temperature for 1 hour. Subsequently, a saturated aqueous sodium chloride solution was added, and the mixed solution is purified by reverse phase silica gel column chromatography (Biotage, LLC, C18 column, acetonitrile: distilled water = 10:90), and the solvent was removed by evaporation and freeze-drying to distill off the compound. 0.03 g (yield: 60%, red solid) of (1-NHS) was obtained.
[M - 2Na⁺ + H⁺]⁻: 702

### <Synthesis of labeled antibody (1)>

40 µL of a carbonate pH standard solution (pH = 10.01) (FUJIFILM Wako Pure Chemical Corporation) and 3.2 µL of a DMSO solution having a concentration of the compound (1-NHS) of 20 mM were added to 400 µL of an anti-rabbit IgG antibody [host: goat, 2.4 mg/mL, catalog number: 111-005-003, manufactured by Jackson ImmunoResearch Inc.], and the resultant mixture was stirred and allowed to stand at room temperature for 1 hour. Subsequently, the reaction solution was directly applied onto a Sephadex G-25 column [catalog number: 17085101, manufactured by GE Healthcare] and purified by using PBS [pH = 7.4, manufactured by FUJIFILM Wako Pure Chemical Corporation], thereby obtaining the labeled antibody (1).

### [Synthesis Example 2: Synthesis method for compound (2) and labeled antibody (2)]

A compound (2) and a labeled antibody (2) were synthesized based on the following scheme.

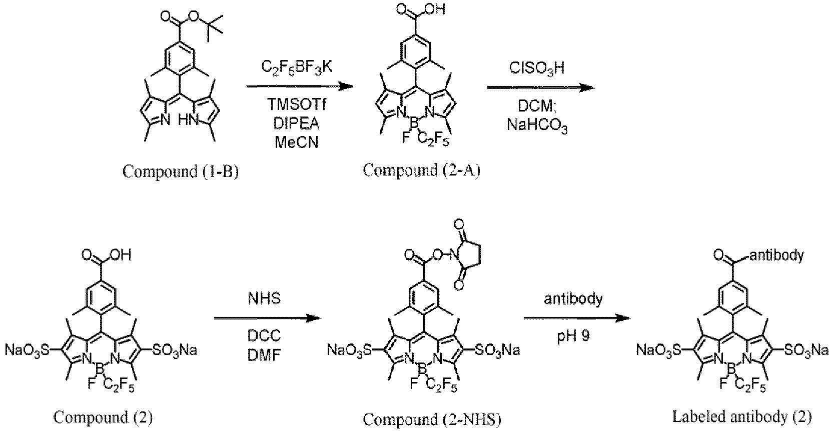

### <Synthesis of compound (2-A)>

0.13 g (reddish brown solid) of a compound (2-A) was obtained in the same manner except that in the synthesis method for the compound (1-C), potassium pentafluroroethyltrifluoroborate (manufactured by Sigma-Aldrich Co., LLC) was used instead of potassium trifluoro(trifluoromethyl)borate.
[M + H⁺]⁺: 497

### <Synthesis of compound (2)>

0.02 g (reddish solid) of a compound (2) was obtained in the same manner except that in the synthesis method for the compound (1), the compound (2-A) was used instead of the compound (1-C).
[M - 2Na⁺ + H⁺]⁻: 655

### <Synthesis of compound (2-NHS)>

0.01 g (reddish solid) of a compound (2-NHS) was obtained in the same manner except that in the synthesis method for the compound (1-NHS), the compound (2) was used instead of the compound (1).
[M - 2Na⁺ + H⁺]⁻: 752

### <Synthesis of labeled antibody (2)>

A labeled antibody (2) was obtained in the same manner except that in the synthesis method for the labeled antibody (1), the compound (2-NHS) was used instead of the compound (1-NHS).

### [Synthesis Example 3: Synthesis of compound (3) and labeled antibody (3)]

A compound (3) and a labeled antibody (3) were synthesized based on the following scheme.

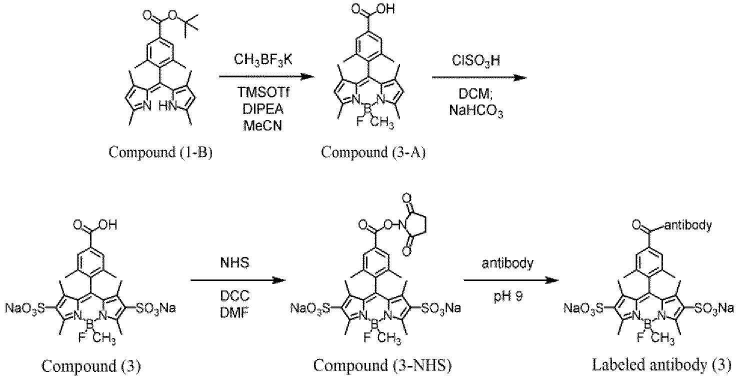

### <Synthesis of compound (3-A)>

0.11 g (reddish brown solid) of a compound (3-A) was obtained in the same manner except that in the synthesis method for the compound (1-C), potassium methyltrifluoroborate (manufactured by Sigma-Aldrich Co., LLC) was used instead of potassium trifluoro(trifluoromethyl)borate.
[M + H⁺]⁺: 393

### <Synthesis of compound (3)>

0.03 g (reddish solid) of a compound (3) was obtained in the same manner except that in the synthesis method for the compound (1), the compound (3-A) was used instead of the compound (1-C).
[M - 2Na⁺ + H⁺]⁻: 551

### <Synthesis of compound (3-NHS)>

0.01 g (reddish solid) of a compound (3-NHS) was obtained in the same manner except that in the synthesis method for the compound (1-NHS), the compound (3) was used instead of the compound (1).
[M - 2Na⁺ + H⁺]⁻: 648

### <Synthesis of labeled antibody (3)>

A labeled antibody (3) was obtained in the same manner except that in the synthesis method for the labeled antibody (1), the compound (3-NHS) was used instead of the compound (1-NHS).

### [Synthesis Example 4: Synthesis of compound (4) and labeled antibody (4)]

A compound (4) and a labeled antibody (4) were synthesized based on the following scheme.

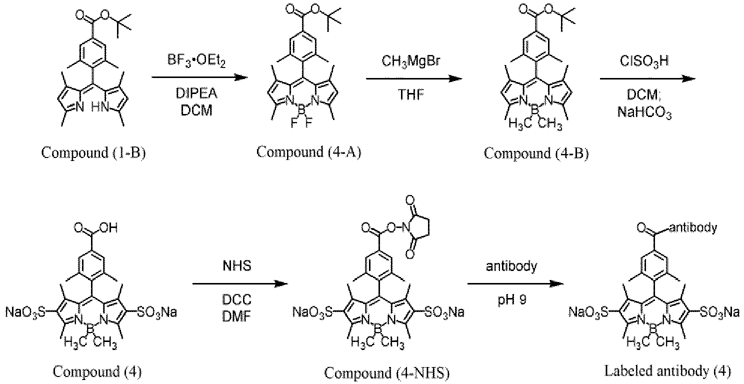

### <Synthesis of compound (4-A)>

0.10 g of the compound (1-B) and 4.3 mL of methylene chloride were added to a 50 mL eggplant flask. Subsequently, under a nitrogen atmosphere, 0.26 mL of N,N-diisopropylethylamine (Fujifilm FUJIFILM Wako Pure Chemical Corporation) and 0.30 mL of boron trifluoride diethyl ether complex (Fujifilm FUJIFILM Wako Pure Chemical Corporation) were added, and the mixture was stirred at room temperature for 10 minutes. After adding a saturated aqueous sodium chloride solution, water was added, the mixture was extracted with methylene chloride, and purification was carried out by silica gel column chromatography (ethyl acetate: normal hexane = 20:80). The solvent was distilled off under reduced pressure to obtain 0.17 g (yield: 89%, reddish brown solid) of a compound (4-A).
[M + H⁺]⁺: 453

### <Synthesis of compound (4-B)>

0.025 g of the compound (4-A) and 1.1 mL of tetrahydrofuran (FUJIFILM Wako Pure Chemical Corporation) were added to a 50 mL eggplant flask, and under a nitrogen atmosphere, 0.55 mL of methylmagnesium bromide (a 12% tetrahydrofuran solution, about 1 mol/L) (Tokyo Chemical Industry Co., Ltd.) was added thereto under ice-cooling, followed by stirring for 1 hour. After adding a saturated aqueous ammonium chloride solution, water was added, the mixture was extracted with methylene chloride, and purification was carried out by silica gel column chromatography (ethyl acetate: normal hexane = 5:95). The solvent was distilled off under reduced pressure to obtain 0.027 g (yield: 111%, reddish solid) of a compound (4-B).
[M + H⁺]⁺: 445

### <Synthesis of compound (4)>

0.013 g (reddish solid) of a compound (4) was obtained in the same manner except that in the synthesis method for the compound (1), the compound (4-B) was used instead of the compound (1-C).
[M - 2Na⁺ + H⁺]⁻: 547

### <Synthesis of compound (4-NHS)>

0.004 g (reddish solid) of a compound (4-NHS) was obtained in the same manner except that in the synthesis method for the compound (1-NHS), the compound (4) was used instead of the compound (1).
[M - 2Na⁺ + H⁺]⁻: 644

### <Synthesis of comparative labeled antibody (4)>

A labeled antibody (4) was obtained in the same manner except that in the synthesis method for the labeled antibody (1), the compound (4-NHS) was used instead of the compound (1-NHS).

### [Synthesis Example 5: Synthesis of compound (5) and labeled antibody (5)]

A compound (5) and a labeled antibody (5) were synthesized based on the following scheme.

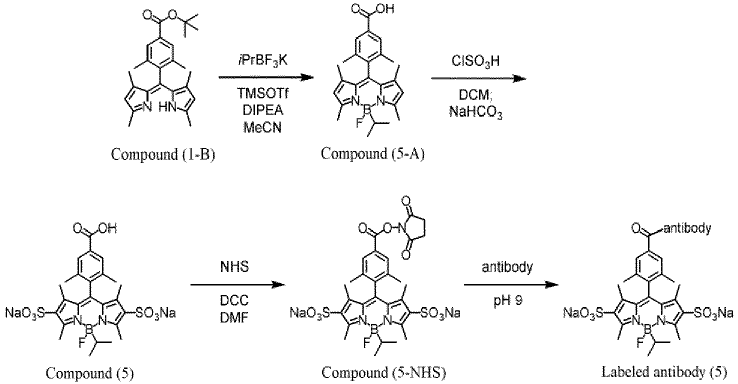

### <Synthesis of compound (5-A)>

0.008 g (reddish brown solid) of a compound (5-A) was obtained in the same manner except that in the synthesis method for the compound (1-C), potassium isopropyltrifluoroborate (manufactured by Sigma-Aldrich Co., LLC) was used instead of potassium trifluoro(trifluoromethyl)borate.
[M + H⁺]⁺: 421

### <Synthesis of compound (5)>

0.0009 g (reddish solid) of a compound (5) was obtained in the same manner except that in the synthesis method for the compound (1), the compound (5-A) was used instead of the compound (1-C).
[M - 2Na⁺ + H⁺]⁻: 579

### <Synthesis of compound (5-NHS)>

0.0005 g (reddish solid) of a compound (5-NHS) was obtained in the same manner except that in the synthesis method for the compound (1-NHS), the compound (5) was used instead of the compound (1).
[M - 2Na⁺ + H⁺]⁻: 676

### <Synthesis of labeled antibody (5)>

A labeled antibody (5) was obtained in the same manner except that in the synthesis method for the labeled antibody (1), the compound (5-NHS) was used instead of the compound (1-NHS).

### [Synthesis Example 6: Synthesis of compound (6) and labeled antibody (6)]

A compound (6) and a labeled antibody (6) were synthesized based on the following scheme.

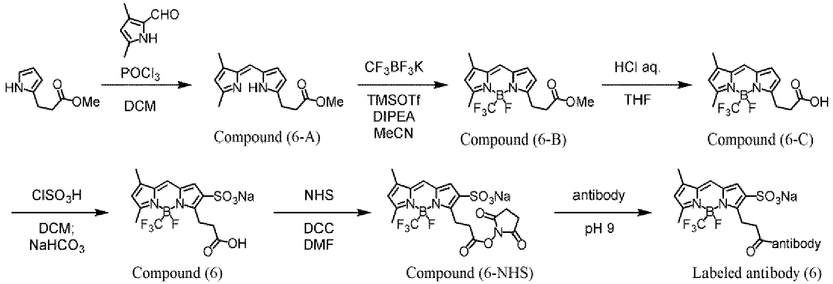

### <Synthesis of compound (6-A)>

0.15 g of 3,5-dimethyl-1H-pyrrole-2-carbaldehyde (FUJIFILM Wako Pure Chemical Corporation), 0.13 g of methyl 3-(2-pyrrolyl)propanoate (Toronto Research Chemicals), and 10 mL of methylene chloride were added to a 50 mL eggplant flask, and after adding 0.09 mL of phosphorus oxychloride (FUJIFILM Wako Pure Chemical Corporation) under ice-cooling, the temperature was raised to room temperature, and the mixture was stirred for 4 hours. The reaction solution was added to water at room temperature, the mixture was extracted with methylene chloride, and purification was carried out by silica gel column chromatography (ethyl acetate only). The solvent was distilled off under reduced pressure to obtain 0.19 g (yield: 76%, reddish brown solid) of a compound (6-A).
[M + H⁺]⁺: 259

### <Synthesis of compound (6-B)>

0.05 g of the compound (6-A) and 5 mL of methylene chloride were added to a 50 mL eggplant flask, and after adding 0.10 mL of N,N-diisopropylethylamine (FUJIFILM Wako Pure Chemical Corporation) under a nitrogen atmosphere, the resultant mixture was stirred at room temperature for 10 minutes. Subsequently, a solution obtained by mixing 0.14 g of potassium trifluoro(trifluoromethyl)borate (FUJIFILM Wako Pure Chemical Corporation), 0.34 g of trimethylsilyl trifluoromethanesulfonate (FUJIFILM Wako Pure Chemical Corporation), and 1.3 mL of acetonitrile was added under ice-cooling, the temperature was raised to room temperature, and the mixture was stirred for 3 hours. The reaction mixture was directly charged on silica gel and purified by silica gel column chromatography (ethyl acetate: normal hexane = from 0:100 to 100:0). The solvent was distilled off under reduced pressure to obtain 0.017 g (yield: 25%, reddish solid) of a compound (6-B).
[M + H⁺]⁺: 357

### <Synthesis of compound (6-C)>

0.002 g of the compound (6-B), 0.2 mL of tetrahydrofuran, 0.1 mL of 30% hydrochloric acid (FUJIFILM Wako Pure Chemical Corporation), and 0.1 mL of distilled water were added to a 50 mL eggplant flask, and the resultant mixture was stirred at room temperature for 12 hours. Subsequently, extraction was carried out with ethyl acetate, washing was carried out with saturated saline, drying was carried out with sodium sulfate, and the solvent was distilled off under reduced pressure. The obtained residue was directly charged on silica gel and purified by silica gel column chromatography (ethyl acetate: normal hexane = 0:100 to 100:0). The solvent was distilled off under reduced pressure to obtain 0.001 g (yield: 69%, reddish brown solid) of a compound (6-C).
[M + H⁺]⁺: 343

### <Synthesis of compound (6)>

0.001 g of the compound (6-C) and 0.3 mL of methylene chloride were added to a 50 mL eggplant flask, 0.0002 mL of chlorosulfonic acid (FUJIFILM Wako Pure Chemical Corporation) was added thereto under ice-cooling, and the resultant mixture was stirred for 5 minutes. Subsequently, 1 mL of saturated aqueous sodium hydrogen carbonate solution (FUJIFILM Wako Pure Chemical Corporation) was added, and after distilling off methylene chloride under reduced pressure, the mixed solution was purified by reverse phase silica gel column chromatography (manufactured by Biotage, LLC, a C18 column, distilled water only), and the solvent was removed by evaporation and freeze-drying to obtain 0.0005 g (yield: 40%, red solid) of a compound (6).
[M - Na⁺]⁻: 421

### <Synthesis of compound (6-NHS)>

0.0003 g (reddish solid) of a compound (6-NHS) was obtained in the same manner except that in the synthesis method for the compound (1-NHS), the compound (6) was used instead of the compound (1).

### <Synthesis of labeled antibody (6)>

A labeled antibody (6) was obtained in the same manner except that in the synthesis method for the labeled antibody (1), the compound (6-NHS) was used instead of the compound (1-NHS).

### [Synthesis Example 7: Synthesis of compound (7) and labeled antibody (7)]

A compound (7) and a labeled antibody (7) were synthesized based on the following scheme.

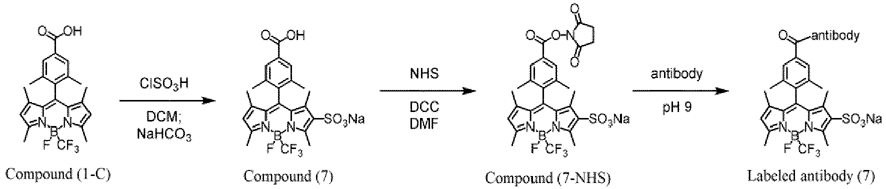

### <Synthesis of compound (7)>

0.001 g (reddish solid) of a compound (7) was obtained in the same manner except that in the synthesis method for the compound (1), the amount of chlorosulfonic acid was changed from 0.09 mL to 0.01 mL.
[M - Na⁺]⁻: 525

### <Synthesis of compound (7-NHS)>

0.0005 g (reddish solid) of a compound (7-NHS) was obtained in the same manner except that in the synthesis method for the compound (1-NHS), the compound (7) was used instead of the compound (1).
[M - Na⁺]⁻: 622

### <Synthesis of labeled antibody (7)>

A labeled antibody (7) was obtained in the same manner except that in the synthesis method for the labeled antibody (1), the compound (7-NHS) was used instead of the compound (1-NHS).

### [Synthesis Example 8: Synthesis of comparative compound (3) and comparative labeled antibody (3)]

A comparative compound (3) and a comparatively labeled antibody (3) were synthesized based on the following scheme.

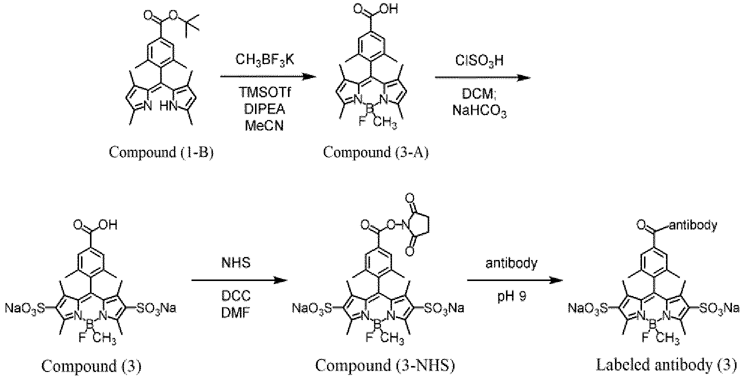

### <Synthesis of comparative compound (3-A)>

0.30 g (reddish brown solid) of a comparative compound (3-A) was obtained in the same manner except that in the synthesis method for the compound (1-C), a boron trifluoride diethyl ether complex (FUJIFILM Wako Pure Chemical Corporation) was used instead of potassium trifluoro(trifluoromethyl)borate and trimethylsilyl trifluoromethanesulfonate.
[M + H⁺]⁺: 397

### <Synthesis of comparative compound (3)>

0.10 g (reddish solid) of a comparative compound (3) was obtained in the same manner except that in the synthesis method for the compound (1), the comparative compound (3-A) was used instead of the compound (1-C).
[M - 2Na⁺ + H⁺]⁻: 555

### <Synthesis of comparative compound (3-NHS)>

0.04 g (reddish solid) of a comparative compound (3-NHS) was obtained in the same manner except that in the synthesis method for the compound (1-NHS), the comparative compound (3-NHS) was used instead of the compound (1).
[M - 2Na⁺ + H⁺]⁻: 652

### <Synthesis of comparative labeled antibody (3)>

A comparative labeled antibody (3) was obtained in the same manner except that in the synthesis method for the labeled antibody (1), the comparative compound (3-NHS) was used instead of the compound (1-NHS).

### [Synthesis Example 9: Synthesis of comparative compound (4) and comparative labeled antibody (4)]

A comparative compound (4) and a comparative labeled antibody (4) were synthesized based on the following scheme.

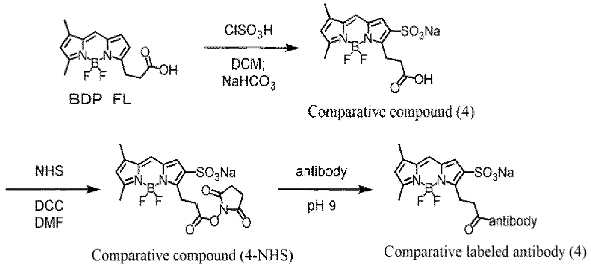

### <Synthesis of comparative compound (4)>

8 mg (reddish solid) of a comparative compound (4) was obtained in the same manner except that in the synthesis method for the compound (6), BDP FL (Tokyo Chemical Industry Co., Ltd.) was used instead of the compound (6-C).
[M - Na⁺]⁻: 371

### <Synthesis of comparative compound (4-NHS)>

0.005 g (reddish solid) of a comparative compound (4-NHS) was obtained in the same manner except that in the synthesis method for the compound (1-NHS), the comparative compound (4-NHS) was used instead of the compound (1).
[M - Na⁺]⁻: 468

### <Synthesis of comparative labeled antibody (4)>

A comparative labeled antibody (4) was obtained in the same manner except that in the synthesis method for the labeled antibody (1), the comparative compound (4-NHS) was used instead of the compound (1-NHS).

Although it is not particularly described, the sulfo group and the carboxy group may adopt a salt structure (for example, a potassium salt, a sodium salt, or a DIPEA salt).

### <Example 1> Evaluation of water solubility and fluorescence quantum yield of compound

The following characteristics were evaluated for each of the compounds, and the obtained results are shown in Table 1. In the column of the moiety bondable to the biological substance in the table, "o" indicates that the moiety bondable to the biological substance is provided, and "×" indicates that the moiety bondable to the biological substance is not proved.

### [1] Evaluation of water solubility

5 µL of a DMSO solution having a concentration of 20 mM of the above compound (hereinafter, referred to as a sample) and 495 µL of PBS having a pH of 7.4 were added and mixed in a 1.5 mL Eppendorf tube and stirred with a multi-shaker MS300 (product name, AS ONE Corporation) at 2,000 rpm for 30 minutes. The obtained mixed solution was allowed to be left for 60 minutes with light shielding and then subjected to centrifugal precipitation (12,000 rpm, 5 minutes). For the filtrate filtered using a filter having a pore diameter of 0.20 µm, the sample concentration was measured with Nexera UHPLC [product name, manufactured by Shimadzu Corporation, column: Shim-pack XR-ODSII] (the sample concentration is 200 µM in a case where the sample is completely dissolved) and evaluated based on the following evaluation standards.

In the present test, it is determined that a compound has passed the evaluation of the water solubility in a case where the compound has a rank "B" or higher.

### - Evaluation of water solubility -

A: 100 µM or more
B: 10 µM or more and less than 100 µM
C: 1 µM or more and less than 10 µM
D: Less than 1 µM, or water solubility is low and thus concentration cannot be measured.

### [2] Evaluation of fluorescence quantum yield

A PBS solution (pH 7.4) of the above compound was evaluated according to the method described in the following reference document.

"A Guide to Recording Fluorescence Quantum Yields" (a HORIBA Scientific document, available from https://www.horiba.com/fileadmin/uploads/Scientific/Documents/Fluorescence/quantumyields trad.pdf)

In the present test, it is preferable that the fluorescence quantum yield satisfies the evaluation rank "B" or higher from the viewpoint of practicality as the fluorescent compound.

### - Evaluation standards for fluorescence quantum yield -

A: 0.9 or more
B: 0.7 or more and less than 0.9
C: 0.5 or more and less than 0.7
D: 0.3 or more and less than 0.5
E: less than 0.3
F: not evaluable due to low water solubility

**[Table 1]**

| | Fluorescent protein | Water solubility | Fluorescence quantum yield | Moiety bondable to biological substance |
|---|---|---|---|---|
| Example 1-1 | Compound (1) | A | A | ∘ |
| Example 1-2 | Compound (2) | A | A | ∘ |
| Example 1-3 | Compound (3) | A | B | ∘ |
| Example 1-4 | Compound (4) | A | B | ∘ |
| Example 1-5 | Compound (5) | A | B | ∘ |
| Example 1-6 | Compound (6) | A | B | ∘ |
| Example 1-7 | Compound (7) | A | A | ∘ |
| Comparative Example 1-1 | BODIPY FL | C | B | ∘ |
| Comparative Example 1-2 | BODIPY 493/503 | C | A | × |
| Comparative Example 1-3 | BODIPY492/515 | A | A | × |
| Comparative Example 1-1 | Comparative compound (1) | C | B | × |
| Comparative Example 1-2 | Comparative compound (2) | A | D | ∘ |
| Comparative Example 1-3 | Comparative compound (3) | A | A | ∘ |
| Comparative Example 1-4 | Comparative compound (4) | A | B | ∘ |

From the results in Table 1 above, the following can be seen.

The fluorescent compound used in Comparative Example 1-1 has low water solubility and is inferior in hydrophilicity. Further, among Reference Examples 1-1 to 1-3 using commercially available dipyrromethene boron complex compounds, Reference Examples 1-1 and 1-2 have low water solubility and are inferior in hydrophilicity, and Reference Examples 1-3, which is not intended to be used as a labeled biological substance by being bonded to a biological substance, is inferior in general-purpose properties in a case of being used as a labeled biological substance.

On the other hand, the fluorescent compounds (1) to (7) according to the embodiment of the present invention, which are represented by Formula (1), and the comparative compounds (2) to (4) are all at the acceptable level in terms of water solubility and have a moiety bondable to a biological substance, and thus it is expected to have general-purpose properties in a case of being used as a labeled biological substance.

For example, the fluorescent compounds (1) and (2), in which Q² in Formula (1) is a halogenoalkyl group and both R³ and R⁴ have at least one group of the group Pi of hydrophilic groups, have a good water-solubility and a good fluorescence quantum yield.

Similarly, the fluorescent compound (7), in which Q² in Formula (1) is a halogenoalkyl group and one of R³ or R⁴ has at least one group of the group Pi of hydrophilic groups, also has a good water-solubility and a good fluorescence quantum yield.

Among the other fluorescent compounds (3) to (6), the fluorescent compound (3), in which Q² in Formula (1) is an alkyl group and both R³ and R⁴ have at least one group of the group Pi of hydrophilic groups, is preferable.

Hereinafter, in Example 2, each of the labeled antibodies of these fluorescent compounds (1) to (5) and (7) and the comparative compounds (2) and (3) were used to prepare an immunohistochemically stained specimen, and the light resistance thereof was evaluated.

### <Example 2> Evaluation of light resistance (fading resistance) of labeled antibody in immunohistochemically stained specimen

Using each of the labeled antibodies described above, immunohistochemical staining was carried out as follows. The following characteristics were evaluated for the prepared specimens, and the obtained results are shown in Table 2.

### [Preparation of immunohistochemically stained specimen]

Brain sections (5 µm) of Wistar rats, subjected to perfusion fixation, were washed with a Triton X-100 solution, blocked, and then reacted with an anti-Ibal antibody (manufactured by FUJIFILM Wako Pure Chemical Corporation) overnight. Subsequently, after washing, the brain section was reacted with the solution of each labeled antibody for about 2 hours. After washing, the brain section was sealed with 70% glycerol-PBS to prepare an immunohistochemically stained specimen.

### [Evaluation of fading resistance in stained specimen]

Each stained cell sample was placed under a confocal laser microscope [TCS SP5, manufactured by Leica Microsystems], and a time-lapse measurement was carried out with a He-Ne laser (output: 100%) as a light source at a wavelength of 488 nm and a detection wavelength of 500 to 550 nm. The time-lapse profiles of the fluorescence intensities at the four stained and four unstained areas were acquired, and the average signal intensity of the unstained areas was subtracted from the average signal intensity of the stained areas to take the resultant difference as the net fluorescence intensity. The fluorescence intensity before light exposure was set to 100% and the light exposure time until the absorbance decreased by 50% (the absorbance reaches 50%) was measured. The results were evaluated based on the following evaluation standards.

In the present test, it is determined that a compound has passed the evaluation of the fading resistance in a case where the compound has a rank "B" or higher.

### - Evaluation standards for fading resistance -

A: 3 minutes or more
B: 2 minutes or more and less than 3 minutes
C: 1 minute or more and less than 2 minutes
D: less than 1 minute

**[Table 2]**

| | Name of labeled antibody used in staining | Fading resistance |
|---|---|---|
| Example 2-1 | Labeled antibody (1) | A |
| Example 2-2 | Labeled antibody (2) | A |
| Example 2-3 | Labeled antibody (3) | A |
| Example 2-4 | Labeled antibody (4) | A |
| Example 2-5 | Labeled antibody (5) | A |
| Example 2-6 | Labeled antibody (7) | A |
| Comparative Example 2-1 | Comparative labeled antibody (2) | D |
| Comparative Example 2-2 | Comparative labeled antibody (3) | C |

From the results of Table 2, it can be seen that, as compared with the stained specimen prepared using the comparative labeled antibody (2) or (3), the stained specimen prepared using the antibody labeled with the fluorescent compound according to the embodiment of the present invention, which is represented by Formula (1), exhibit excellent fading resistance.

As described above, the fluorescent compound according to the embodiment of the present invention exhibits high water solubility and can be used as a labeled biological substance due to having an excellent binding property to the biological substance. In addition, it exhibits a high fluorescence quantum yield required from the viewpoint of practicality as a fluorescent compound. Furthermore, the obtained labeled antibody can improve the light resistance in the biological tissue, which has been a drawback of the compound in the related which has a dipyrromethene boron complex structure. As a result, the fluorescent compound according to the embodiment of the present invention can greatly improve the general-purpose properties as a fluorescent dye that is used in the imaging of the biological substance or the detection of the biological substance.

### <Reference Example 1> Evaluation of light resistance of compound

The following characteristics were evaluated for each of the above-described compounds and each of the labeled antibodies, and the obtained results are shown in Table 3.

### [1] Evaluation of light resistance in aqueous solution

The above compound or labeled antibody (hereinafter, referred to as a sample) was dissolved in PBS having a pH of 7.4 so that the absorbance at the absorption wavelength peak was 0.095 to 0.105. In a state of being exposed to light using a merry-go-round type light irradiator, the obtained solution was subjected to the temporal measurement of the absorbance of the absorption wavelength peak of the sample with a spectroscope (manufactured by Hewlett-Packard Company, product name: Agilent 8453).

For the light exposure using the merry-go-round type light irradiator, a xenon lamp UXL-500D-O (product name) manufactured by Ushio, Inc. was used, and an HA-50 filter and a Y44 filter manufactured by HOYA were used as sharp cut filters (both are product names), where the light exposure intensity was 22 mW/cm² (in terms of 500 nm conversion).

The absorbance at the absorption peak wavelength before light exposure was set to 100% and the light exposure time until the absorbance at this absorption peak wavelength decreases by 50% (the absorbance at the absorption peak wavelength reaches 50%) was measured. The results were evaluated based on the following evaluation standards. A high evaluation rank is highly preferable since stability is kept for a long time.

### - Evaluation standards for light resistance -

A: 80 hours or more
B: 60 hours or more and less than 80 hours
C: 40 hours or more and less than 60 hours
D: 20 hours or more and less than 40 hours
E: 10 hours or more and less than 20 hours
F: less than 10 hours
G: not evaluable due to low water solubility

### [2] Evaluation of water solubility and fluorescence quantum yield

The water solubility and the fluorescence quantum of the labeled antibody were evaluated according to the same methods as those described in Example 1 except that in the evaluation of the water solubility and the fluorescence quantum yield in Example 1, the sample for measurement was changed from the compound to the labeled antibody.

In a case where the evaluation rank of the water solubility is high, the hydrophilicity is high, which is very preferable, and in a case where the evaluation rank of the fluorescence quantum yield is high, the fluorescence quantum yield is excellent, which is very preferable.

**[Table 3-1]**

| | | Fluorescent protein | | | Light resistance | |
|---|---|---|---|---|---|---|
| Example 3-1 | | Compound (1) | | | A | |
| Example 3-2 | | Compound (2) | | | A | |
| Example 3-3 | | Compound (3) | | | D | |
| Comparative Example 3-1 | | BODIPY FL | | | F | |
| Comparative Example 3-2 | | BODIPY493/503 | | | F | |
| Comparative Example 3-3 | | BODIPY492/515 | | | E | |
| Comparative Example 3-1 | | Comparative compound (1) | | | C | |
| Comparative Example 3-2 | | Comparative compound (2) | | | B | |

**[Table 3-2]**

| | Labeled antibody | | Water solubility | Light resistance | | Fluorescence quantum yield |
|---|---|---|---|---|---|---|
| Example 4-1 | Labeled antibody (1) | | A | B | | B |
| Example 4-2 | Labeled antibody (2) | | A | B | | B |
| Example 4-3 | Labeled antibody (3) | | A | E | | B |
| Comparative Example 4-1 | Comparative labeled antibody (2) | | A | E | | D |

From the results in Table 3 above, the following can be seen.

Among the compounds according to the embodiment of the present invention, which are represented by Formula (1), the compounds (1) and (2) used in Examples 3-1 and 3-2, in which at least one of Q¹ or Q² is a halogenoalkyl group, has excellent light resistance in the aqueous solution as compared with the fluorescent compounds used in Reference Examples 3-1 to 3-3 and Comparative Examples 3-1 and 3-2.

Further, the labeled antibodies of Examples 4-1 and 4-2, which are the labeled antibodies of the compounds used in Examples 3-1 and 3-2, has high water solubility, excellent light resistance in the aqueous solution, and an excellent fluorescence quantum yield, as compared with the comparative labeled antibody (2) used in Comparative Example 4-1.

As described above, in a case where the results of Tables 1 and 2 are combined, it can be seen that among the compounds according to the embodiment of the present invention, which are represented by Formula (1), the compounds in which at least one of Q¹ or Q² is a halogenoalkyl group, and the antibodies labeled with these compounds have excellent light resistance in the aqueous solution in addition to the hydrophilicity and the excellent light resistance in the biological tissue.

The present invention has been described together with the embodiments of the present invention. However, the inventors of the present invention do not intend to limit the present invention in any part of the details of the description unless otherwise specified, and it is conceived that the present invention should be broadly construed without departing from the spirit and scope of the invention shown in the attached "WHAT IS CLAIMED IS".

This application claims priority based on JP2019-210701 filed in Japan on November 21, 2019, and JP2020-110909 filed in Japan on June 26, 2020, which are incorporated herein by reference as a part of the description of the present specification.

## Claims

1. A compound represented by Formula (1), 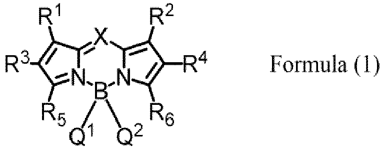 in the formula, X represents CR⁷ or N,
R¹ to R⁷, Q¹, and Q² represent a hydrogen atom, a halogen atom, a cyano group, or a group represented by Formula (A), and adjacent substituents among R¹ to R⁷, Q¹, and Q² may form a ring structure,
provided that at least one of R³, R⁴, R⁷, Q¹, or Q² contains at least one group in the following group Pi of hydrophilic groups, and at least one of Q¹ or Q² represents an alkyl group,
^{∗}-L³-R¹¹¹ Formula (A)
in the formula, L³ is a single bond or a linking group obtained by combining one or more kinds of groups among an alkylene group, an alkenylene group, an alkynylene group, an arylene group, a heteroarylene group, and groups respectively represented by Formula (1-1) to Formula (1-9),
R¹¹¹ represents a hydrogen atom, an alkyl group, an alkenyl group, an alkynyl group, an aryl group, a heteroaryl group, or a monovalent aliphatic heterocyclic group,
provided that, in a case where the hydrogen atom in R¹¹¹ is a dissociative hydrogen atom, a salt may be formed, and each group in L³ and R¹¹¹ may further have a substituent, and
^{∗} represents a bonding portion, 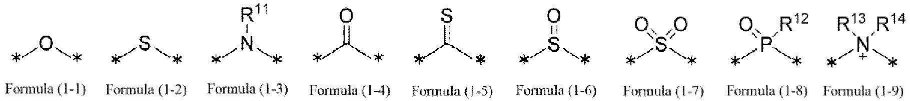
in the formula, R¹¹ to R¹⁴ represent a hydrogen atom or a substituent, and
^{∗} represents a bonding portion,
<the group Pi of hydrophilic groups>
a carboxy group or a salt thereof, a sulfo group or a salt thereof, a phosphono group or a salt thereof, an onio group, and a polyamino acid residue.

2. The compound according to claim 1,
wherein X is CR⁷, and R⁷ is a hydrogen atom or the group represented by Formula (A).

3. The compound according to claim 1 or 2,
wherein at least one of R³, R⁴, or R⁷ has at least one group of the group Pi of hydrophilic groups.

4. The compound according to any one of claims 1 to 3,
wherein at least one of R³ or R⁴ is the group represented by Formula (A), and the group represented by Formula (A) has at least one group in the following group Pi-1 of hydrophilic groups,
<the group Pi-1 of hydrophilic groups>
a carboxy group or a salt thereof, a sulfo group or a salt thereof, and a phosphono group or a salt thereof.

5. The compound according to claim 4,
wherein L³ in the group represented by Formula (A) is a linking group obtained by combining two or more kinds of groups among an alkenylene group, an alkynylene group, an arylene group, a heteroarylene group, and groups respectively represented by Formula (1-1), Formula (1-3), and Formula (1-4), Formula (1-7), and Formula (1-8), and has at least one group in the group Pi-1 of hydrophilic groups.

6. The compound according to any one of claims 1 to 5,
wherein at least one of R³ or R⁴ is a carboxy group or a salt thereof, a sulfo group or a salt thereof, or a phosphono group or a salt thereof.

7. The compound according to any one of claims 1 to 6,
wherein at least one of R³ or R⁴ is a sulfo group or a salt thereof.

8. The compound according to any one of claims 1 to 7,
wherein Q¹ is a halogen atom, an alkyl group, an alkynyl group, an aryl group, a hydroxy group, or an alkoxy group.

9. The compound according to any one of claims 1 to 8,
wherein Q² is an alkyl group having 1 to 4 carbon atoms.

10. The compound according to any one of claims 1 to 9,
wherein Q² is a halogenoalkyl group having 1 to 4 carbon atoms.

11. The compound according to any one of claims 1 to 10,
wherein R⁷ is a hydrogen atom, an aryl group, or a heteroaryl group.

12. The compound according to any one of claims 1 to 11,
wherein R⁷ is a hydrogen atom or a group represented by Formula (C), 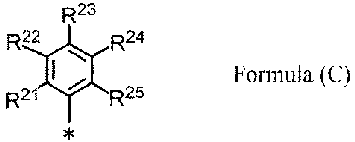
in the formula, R²¹ represents a substituent, and R²² to R²⁵ represents a hydrogen atom or a substituent, and
^{∗} represents a bonding portion.

13. The compound according to claim 1,
wherein the compound is represented by any of the following compounds (1) to (7),

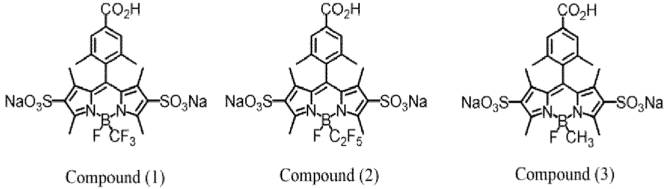

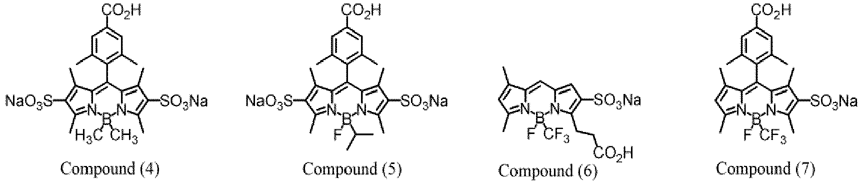

14. The compound according to claim 9,
wherein Q¹ is a halogen atom, and R⁷ is an aryl group.

15. The compound according to claim 14,
wherein both R³ and R⁴ are a sulfo group or a salt thereof.

16. The compound according to claim 15,
wherein Q² is a halogenoalkyl group having 1 to 4 carbon atoms.

17. The compound according to any one of claims 1 to 16,
wherein at least one of R¹ to R⁷, Q¹, or Q² has a moiety bondable to a biological substance.

18. A fluorescently labeled biological substance, which is obtained by bonding between the compound according to claim 17 and a biological substance.

19. The fluorescently labeled biological substance according to claim 18,
wherein the biological substance is any one of a protein, a peptide, an amino acid, a nucleic acid, a sugar chain, or a lipid.

20. The fluorescently labeled biological substance according to claim 18 or 19,
wherein the bonding between the compound and the biological substance is bonding formed by any one of the followings i) to v),
i) non-covalent or covalent bond between peptides,
ii) Van der Waals interaction between a long-chain alkyl group in a compound and a lipid bilayer or lipid in a biological substance,
iii) an amide bond formed by reacting an N-hydroxysuccinimide ester in a compound with an amino group in a biological substance,
iv) a thioether bond formed by reacting a maleimide group in a compound with a sulfanyl group in a biological substance, and
v) a bond associated with a formation of a triazole ring, which is formed by Click reaction between an azido group in a compound and an acetylene group in a biological substance, or between an acetylene group in a compound and an azido group in a biological substance.
